# EUROPEAN PATENT SPECIFICATION

(11) **EP 1 761 248 B1**
(45) Date of publication and mention of the grant of the patent: **23.05.2012**
(21) Application number: 05725950.9
(22) Date of filing: 21.03.2005
(51) Int. Cl.: A61K 9/127, A61K 47/48, A61K 38/16

(54) **CARBOHYDRATE-DERIVATIZED LIPOSOMES FOR TARGETING CELLULAR CARBOHYDRATE RECOGNITION DOMAINS OF CTL/CTLD LECTINS, AND INTRACELLULAR DELIVERY OF THERAPEUTICALLY ACTIVE COMPOUNDS**
KOHLEHYDRAT-DERIVATISIERTE LIPOSOME GEGEN ZELLULÄRE KOHLEHYDRAT-ERKENNUNGSDOMÄNEN VON CTL/CTLD-LEKTINEN UND INTRAZELLULÄRE FREISETZUNG VON THERAPEUTISCHEN WIRKVERBINDUNGEN
LIPOSOMES DERIVES D'HYDRATES DE CARBONE DESTINES A CIBLER DES DOMAINES DE RECONNAISSANCE D'HYDRATES DE CARBONE CELLULAIRES DE LECTINES CTL/CTLD, ET ADMINISTRATION INTRACELLULAIRE DE COMPOSES THERAPEUTIQUEMENT ACTIFS

(30) Priority: 19.03.2004 US 554790 P
(43) Date of publication of application: 14.03.2007
(73) Proprietor: Rodos BioTarget GmbH, 30625 Hannover (DE)
(72) Inventor: GIESELER, Robert K., 45259 Essen-Helsingen (DE); MARQUITAN, Guido, Los Angeles, California 90038 (US); SCOLARO, Michael J., Los Angeles, California 90077 (US); SCHWARZ, Andreas, D-50937 Koln (Cologne) Germany (DE)
(74) Representative: Schüssler, Andrea
(86) International application number: PCT/US2005/009228
(87) International publication number: WO 2005/092288

(56) References cited:
- WO-A-00/63251
- WO-A-98/07408
- WO-A-02/096390
- WO-A-2005/027979
- US-A- 5 686 103
- CHARAN R D ET AL: "Isolation and characterization of Myrianthus holstii lectin, a potent HIV-1 inhibitory protein from the plant Myrianthus holstii" 2000, JOURNAL OF NATURAL PRODUCTS, XX, XX, PAGE(S) 1170-1174 , XP002330343 ISSN: 0163-3864 the whole document

## Description

### BACKGROUND OF THE INVENTION

### 1. Field of the Invention

The present invention relates to the medical arts, and in particular, to targeted liposomal delivery of active agents.

### 2. Discussion of the Related Art

### I. Chronic Infectious Diseases: HIV/AIDS and other Infectious Diseases

Infectious agents call an immune system to action. In an evolutionarily advanced system such as that of a human being or a vertebrate animal, this typically implies that antigen-specific immunity is activated, and shaped upon and directed against, the invader. In this sense, professional antigen-presenting cells (APCs) are the first immune cells to recognize and process the infections agent, so to initiate primary antigen-specific responses. As discussed herein, such APCs also carry CRD lectin receptors which bind (and internalize) the invader. On the on hand, such engagement is a powerful means to help an APC shape the protective response required; on the other hand, a pathogen may also exploit this mechanism to its own advantage.

In the specific sense discussed herein, this may mean that he infectious agent utilizes the integrative receptor for entering the APC, shuts down or misdirects the APCs immune-regulating functions, and further resides within intracytoplasmatic compartments, such as endosmes, in unimpaired form. Such an APC, typically located within a lymphoid organ, is continuously physically engaged by myriads of T cells requesting instruction. This means that a T cell, once establishing contact, is at a high risk of becoming infected by pathogens released from the APC. In essence, this is the nature of a pathogen reservoir (i.e., on the example of an APC as the reservoir cell).

Indeed, since APCs display certain processed particles of the agent on their surface, it appears very likely that an APC impaired by a reservoir-forming agent will, with increased probability, be engaged by T cells specific for this agent. As a consequence, one could further expect that such T cells become infected more likely and, as in the case of HIV disease, be killed off more frequently. Indeed, Douek and colleagues have shown that HIV-specific T-helper memory cells contain more HIV proviral DNA than those memory cells not specific for HIV (Douek, D.C. et al. HIV preferentially infects HIV-specific CD4+ T cells. Nature 2002;417:95-8). These results highlight that the greatest danger of a pathogen reservoir, besides establishing a chronic state of disease, may be the complete knock-out of protective immunity against the invader. This can be fatal.

According to current knowledge, professional APCs comprise four major classes of cells, i.e., (i) myeloid dendritic cells (mDCs); (ii) plasmacytoid dendritic cells (pDCs); (iii) follicular dendritic cells (FDCs); and (iv) macrophages (MPs). In the mammalian organism, these cells are located both in nonlymphoid organs and tissues (certain subsets of immature mDCs; certain subsets of pDCs; and certain subsets of MPs), as well as lymphoid organs and tissues (certain subsets of mature mDCs [also termed interdigitating DCs, or IDCs]; certain subsets of pDCs; certain subsets of MPs; and FDCs) (Imai Y, Yamakawa M, Kasajima T. The lymphocyte-dendritic cell system. Histol Histopathol 1998;13:469-510; and Cella M, Jarrossay D, Facchetti F, Alebardi O, Nakajima H, Lanzavecchia A, Colonna M. Plasmacytoid monocytes migrate to inflamed lymph nodes and produce large amounts of type I interferon. Nat Med 1999;5:919-23).

Among the professional APCs, mDCs are the most potent stimulators of antigen-specific immunity, and they also have the unique ability to induce *primary* cellular and humoral immune responses (e.g., in the intestinal mucosa, as reviewed in Telemo E, Korotkova M, Hanson LA. Antigen presentation and processing in the intestinal mucosa and lymphocyte homing. Ann Allergy Asthma Immunol 2003;90(Suppl 3):28-33, or in the microenvironment of the eye Novak N, Siepmann K, Zierhut M, Bieber T. The good, the bad and the ugly - APCs of the eye. Trends Immunol 2003;24:570-4).

As to MPs, antigen presentation is one of several functions of these cells. MPs may trigger *secondary* antigen-specific responses earlier initiated by mDCs (such as, for example, in the brain, as reviewed in Thomas WE. Brain macrophages: on the role of pericytes and perivascular cells. Brain Res Brain Res Rev 1999;31:42-57).

The principal known function of pDCs is to produce potent *antiviral* type-I interferons (Cella M, Jarrossay D, Facchetti F, Alebardi O, Nakajima H, Lanzavecchia A, Colonna M. Plasmacytoid monocytes migrate to inflamed lymph nodes and produce large amounts of type I interferon. Nat Med 1999;5:919-23).

Finally, at least under healthy conditions, the presence of FDCs is tightly restricted to the follicles within lymphoid organs and tissues where they are instructing B cells for the production of antigen-specific immunoglobulins. Importantly, FDCs provide potent long-term memory for antigens, or entire intact pathogens such as viruses, by retaining these structures within their abundant cytoplasmic projections (reviewed in van Nierop K, de Groot C. Human follicular dendritic cells: function, origin and development. Semin Immunol 2002;14:251-7).

Of note, mDCs and MPs mediate T-cell (or cellular) immunity and memory (Steinman RM. The dendritic cell system and its role in immunogeneicity. Annu Rev Immunol 1991;9:271-96; and Banchereau J, Paczesny S, Blanco P et. al. Dendritic cells: controllers of the immune system and a new promise for immunotherapy. Ann N Y Acad Sci 2003;987:180-7), while FDCs realize B-cell (or humoral) immunity and memory (Burton G F, Conrad DH, Szakal AK, Tew JG. Follicular dendritic cells (FDC) and B cell co-stimulation. J Immunol 1993;150:31; and Qin D, Wu J, Carroll MC, Burton GF, Szakal AK, Tew JG. Evidence for an important interaction between a complement-derived CD21 ligand on follicular dendritic cells and CD21 on B cells in the initiation of IgG responses. J. Immunol 1988;161:4549).

In context of the invention described herein it is to be stressed that professional APCs can provide a long-term reservoir for infectious agents, such as the human immunodeficiency virus 1 (HIV) the hepatitis C virus (HCV), and intracellularly persisting bacteria, such as *Mycobacterium tuberculosis* (Cambi A, Figdor CG. Dual function of C-type lectin-like receptors in the immune system. Curr Opin Cell Biol 2003;15:539-46; and Kaufmann SHE, Schaible UE. A Dangerous liaison between two major killers: Mycobacterium tuberculosis and HIV target dendritic cells through DC-SIGN. J Exp Med 2003;197;1-5). In addition, after infection, similar latent pathogen reservoirs can also establish in non-APCs, such as memory, resting, and/or senescent T lymphocytes (Voehringer D, Blaser C, Brawand P, Raulet DH, Hanke T, Pircher H. Viral infections induce abundant numbers of senescent CD8 T cells. J Immunol 2001;167:4838-43; Douek, D.C. et al. HIV preferentially infects HIV-specific CD4+ T cells. Nature 2002;417:95-8).

For definition, various terms, i.e., *reservoir, sanctuary, latency,* and *dormancy,* are currently used in the literature for describing the pathologic phenomenon that intact infectious agents can be stored for indefinite times in discrete anatomic sites and cells (e.g., Schrager LK, D'Souza MP. Cellular and anatomical reservoirs of HIV-1 in patients receiving potent antiretroviral combination therapy. JAMA 1998;280:67-71; and Savla U. Reservoir: a dirty word in HIV. J Clin Invest 2004;113:146). As to the invention described herein, due to the fact that the terms sanctuary, latency, and dormancy are also used in different contexts, the definition by Nickle *et al.,* in that "virologic *reservoirs* are cell types and tissues in which there is a relative restriction of replication" is used herein (Nickle DC, Jensen MA, Shriner D, Brodie SJ, Frenkel LM, Mittler JE, Mullins JI. Evolutionary indicators of human immunodeficiency virus type 1 reservoirs and compartments. J Virol 2003;77:5540-6). The term, reservoir, is further employed when referring to the specific intracellular compartments where structurally intact infectious agents are stored.

In order to conceive and devise causative therapies for HIV disease and other infectious diseases where infectious sanctuaries are established, it must be the ultimate goal to eliminate the pathogenic agent from its cellular reservoirs. As again discussed on the example of HIV-1, it is now broadly agreed upon that the formation, and persistence, of cellular HIV reservoirs is the most important factor to prevent state-of-the-art highly active antiretroviral treatment (HAART) from clearing HIV from the system (reviewed in Stebbing J, Gazzard B, Douek DC. Where does HIV live? N Engl J Med 2004;350:1872-80). This is due to the fact that, in these reservoir sites, an infectious agent, such as HIV-1, does not actively replicate (Nickle DC, Jensen MA, Shriner D, Brodie SJ, Frenkel LM, Mittler JE, Mullins JI. Evolutionary indicators of human immunodeficiency virus type 1 reservoirs and compartments. J Virol 2003;77:5540-6).

Conversely, HAART only interferes with actively replicating HIV-1. Typically, besides an HIV protease inhibitor (PI), HAART protocols involve a nucleoside reverse transcriptase inhibitor (NRTI) and/or a non-nucleoside reverse transcriptase inhibitor (NNRTI). Both, NRTIs and NNRTIs, specifically block the mechanism of active viral genomic transcription (e.g., Ena J, Amador C, Benito C, Fenoll V, Pasquau F. Risk and determinants of developing severe liver toxicity during therapy with nevirapine-and efavirenz-containing regimens in HIV-infected patients. Int J STD AIDS 2003;14:776-81). Thus, by design, reservoir-confined, non-replicating virions remain unaffected by such treatment which, therefore, may never remove HIV from the system.

Exactly the same conclusion applies to other infectious agents establishing intracellular reservoirs, and for which treatment relies on interference with active replication only. For example, this is the case for the hepatitis C virus (HCV) where state-of-the-art treatment, besides applying interferon-α as an immunostimulant, employs ribavirin (McHutchison JG, Gordon SC, Schiff ER et al. Interferon alfa-2b alone or in combination with ribavirin as initial treatment for chronic hepatitis C. Hepatitis Interventional Therapy Group. N Engl J Med 1998;339:1485-92; and Davis GL, Esteban-Mur R, Rustgi V et al. Interferon alfa-2b alone or in combination with ribavirin for the treatment of relapse of chronic hepatitis C. International Hepatitis Interventional Therapy Group. N Engl J Med 1998;339:1493-9).

In addition, application of HAART in HIV disease entails various side-effects. For example, current treatment acts metabolically detrimental (Lee GA, Seneviratne T, Noor MA, Lo JC, Schwarz J-M, Aweeka FT, Mulligan K, Schambelan M, Grunfeld C. The metabolic effects of lopinavir/ritonavir in HIV-negative men. AIDS 2004;18:641-9), perhaps most prominently exemplified by the development of lipodystrophy, i.e. the HIV-associated adipose redistribution syndrome (Pond CM. Paracrine relationships between adipose and lymphoid tissues: implications for the mechanism of HIV-associated adipose redistribution syndrome. Trends Immunol 2003;24:13-8). Moreover, it recently turned out that, in HIV-infected patients on HAART, T-cell cytokine networks are tonically pre-activated due to drug-dependent endogenous hyper-activation of the cAMP/PKA type I pathway (Johansson CC, Bryn T, Yndestad T, Eiken HG, Bjerkeli V, Frøland SS, Aukrust P, Taskén K. Cytokine networks are pre-activated in T cells from HIV-infected patients on HAART and are under the control of cAMP. AIDS 2004;18:171-9). Therefore, as the activation of a T cell harboring proviral DNA co-activates HIV transcription and propagation, current treatment itself may exacerbate the course of disease.

Productive replication of HIV requires reverse transcription of its RNA genome, (partial) integration into the host cell's double stranded DNA, and transcription from the integrated proviral DNA template, as well as from certain forms of non-integrated viral DNA (Wu Y. HIV-1 gene expression: lessons from provirus and non-integrated DNA. Retrovirology 2004;1:13). Importantly, RNA viruses such as HIV and HCV eventually evade treatment with reverse transcriptase inhibitors by developing resistant strains as a result of frequent mutation due to their lack of a proof-reading mechanism (e.g., Major ME, Rehermann B, Feinstone S. Hepatitis C Viruses. In: Knipe DM, Howley PM, Griffin DE, Lamb RA, Martin MA, Roizman B, Straus SE (eds.). Fields Virology. 4th ed., Vol. I. Lippincott, Williams & Wilkins, Baltimore; 2001: pp. 1127-62). Thus, suitable ways to inactivate structurally intact infectious retrovirions prior to reverse transcription promise to significantly reduce, or completely prevent, the emergence of drug-resistant strains. This may be achieved by ways for inactivating such virions while stored in viral reservoir cells (McDonald D, Wu L, Bohks SM, KewalRamani VN, Unutmaz D, Hope TJ. Recruitment of HIV and its receptors to dendritic cell-T cell junctions. Science 2003;300:1295-7. Epub 2003 May 01; Turville SG, Santos JJ, Frank I, Cameron PU, Wilkinson J, Miranda-Saksena M, Dable J, Stössel H, Romani N, Piatak M Jr, Lifson JD, Pope M, Cunningham AL. Immunodeficiency virus uptake, turnover, and 2-phase transfer in human dendritic cells. Blood 2004;103:2170-9. Epub 2003 Nov 20; and Arrighi JF, Pion M, Garcia E, Escola JM, van Kooyk Y, Geijtenbeek TB, Piguet V. DC-SIGN-mediated infectious synapse formation enhances X4 HIV-1 transmission from dendritic cells to T cells. J Exp Med 2004;200:1279-88).

### A. Anatomic and Cellular Reservoirs

As both exemplarily and specifically discussed for HIV, this virus is preserved in infectious form in cellular and anatomical reservoirs. *Cellular reservoirs* mainly consist of (i) follicular dendritic cells; (ii) myeloid dendritic cells; (iii) macrophages; and (iv) T-memory cells (reviewed in Schrager LK, D'Souza MP. Cellular and anatomical reservoirs of HIV-1 in patients receiving potent antiretroviral combination therapy. JAMA 1998;280:67-71; Burton GF, Keele BF, Estes JD, Thacker TC, Gartner S. Follicular dendritic cell contributions to HIV pathogenesis. Semin Immunol 2002;14:275-84; and Gieseler RK, Marquitan G, Scolaro MJ, Cohen MD. Lessons from history: dysfunctional APCs, inherent dangers of STI and an important goal, as yet unmet. Trends Immunol 2003;24:11). These cells preferentially reside in discrete *anatomic reservoirs.*

First findings pointing to the existence of anatomic and cellular HIV reservoirs were reported in the 1980s (Müller H, Falk S, Stutte HJ. Accessory cells as primary target of human immunodeficiency virus HIV infection. J Clin Pathol 1986;39:1161; Le Tourneau A, Audouin J, Diebold J, Marche C, Tricottet V, Reynes M. LAV-like viral particles in lymph node germinal centers in patients with the persistent lymphadenopathy syndrome and the acquired immunodeficiency syndrome-related complex: an ultrastructural study of 30 cases. Hum Pathol 1987;17:1047-53; and Biberfeld P, Ost A, Porwit A, Sandstedt B, Pallesen G, Bottiger B, Morfelt-Mansson L, Biberfeld G. Histopathology and immunohistology of HTLV-III/LAV related lymphadenopathy and AIDS. Acta Pathol Microbiol Immunol Scand 1987;95:47-65; and Gritti FM, Raise E, Gualandi G, Bonazzi L, Martuzzi M, Schiattone ML, Di Pede B, Gallo R, Rivano MT, Taruscio D, et al. A clinical-immunological evaluation of AIDS cases and related syndromes. J Exp Pathol 1987;3:723-36). Since then, it has become known that several organs and tissues, and discrete cell types located therein, serve HIV as reservoir sites.

### 1. The Anatomic HIV Reservoir

*The Gastrointestinal Tract:* In most patients, HIV-1 infection occurs *via* the gastrointestinal tract. (Smith PD, Meng G, Salazar-Gonzalez JF, Shaw GM. Macrophage HIV-1 infection and the gastrointestinal tract reservoir. J Leukoc Biol 2003;74:642-9. Epub 2003 Aug 11). However, total figures may change in the foreseeable future, as already today the vaginal route is the key infection pathway on the African and Asian continents (Hu Q, Frank I, Williams V, Santos JJ, Watts P, Griffin GE, Moore JP, Pope M, Shattock RJ. Blockade of attachment and fusion receptors inhibits HIV-1 infection of human cervical tissue. J Exp Med 2004;199:1065-75. Epub 2004 Apr 12). (For more information, see below.) In the gastrointestinal tract, the majority of transmitted HIV, which is CCR5-tropic, can be transferred by intestinal CCR5⁺ epithelial cells (Smith PD, Meng G, Salazar-Gonzalez JF, Shaw GM. Macrophage HIV-1 infection and the gastrointestinal tract reservoir. J Leukoc Biol 2003;74:642-9. Epub 2003 Aug 11). When comparing blood, gastrointestinal tract, and lymph nodes, the gastrointestinal tract reveals the most substantial CD4⁺ T cell depletion, preferentially CCR5⁺CD4⁺ T cells, at all stages of HIV disease (Brenchley JM, Schacker TW, Ruff LE, Price DA, Taylor JH, Beilman GJ, Nguyen PL, Khoruts A, Larson M, Haase AT, Douek DC. CD4+ T cell depletion during all stages of HIV disease occurs predominantly in the gastrointestinal tract. J Exp Med 2004;200:749-59. Epub 2004 Sep 13). Specifically within the intestinal mucosa, CCR5⁺ and CXCR4⁺ lymphocytes are the initial target cells and support HIV-1 replication. Indeed, of all anatomic sites, profound CD4⁺ T-cell depletion first becomes apparent shortly after infection in the intestinal lamina propria. However, in the further course of disease, myeloid cells become increasingly important as HIV-1 reservoir cells as successive generations of blood monocytes are recruited to the mucosa where differentiating into lamina propria macrophages. Indeed, these regional macrophages belong to the largest anatomical pool of mononuclear cells. Thus, although only 0.06% of intestinal macrophages harbor virions, they are a major HIV reservoir (Smith PD, Meng G, Salazar-Gonzalez JF, Shaw GM. Macrophage HIV-1 infection and the gastrointestinal tract reservoir. J Leukoc Biol 2003;74:642-9. Epub 2003 Aug 11). Macrophages, therefore, are the principal type of HIV reservoir cell in the gastrointestinal mucosa.

*Lymphoid Organs and Tissues:* Both directly associated with the gastrointestinal tract, as well as in various peripheral sites, lymphoid organs and tissues (e.g., lymph nodes) are another main anatomic HIV reservoir (Racz P, Tenner-Racz K, van Vloten F, Schmidt H, Dietrich M, Gluckman JC, Letvin NL, Janossy G. Lymphatic tissue changes in AIDS and other retrovirus infections: tools and insights. Lymphology 1990;23:85-91; Wood GS. The immunohistology of lymph nodes in HIV infection: a review. Prog AIDS Pathol 1990;2:25-32; Marcoty C, Heinen E, Antoine N, Tsunoda R, Simar LJ. Rapid and selective isolation of follicular dendritic cells by low speed centrifugations on discontinuous BSA gradients. Adv Exp Med Biol 1993;329:.425-9; Rosenberg YJ, Kosco MH, Lewis MG, Leon EC, Greenhouse JJ, Bieg KE, Eddy GA, Zack PM. Changes in follicular dendritic cell and CD8+ cell function in macaque lymph nodes following infection with SIV251. Adv Exp Med Biol 1993;329:.417-23; A, Burton GF, Fuchs BA, Szakal AK, Tew JG. Destruction of follicular dendritic cells in murine acquired immunodeficiency syndrome. Adv Exp Med Biol 1993;329:411-6; Pantaleo G, Graziosi C, Demarest JF, Cohen OJ, Vaccarezza M, Gantt K, Muro-Cacho C, Fauci AS. Role of lymphoid organs in the pathogenesis of human immunodeficiency virus (HIV) infection. Immunol Rev 1994;140:105-30; Cavert W, Notermans DW, Staskus K, Wietgrefe SW, Zupancic M, Gebhard K, Henry K, Zhang ZQ, Mills R, McDade H, Schuwirth CM, Goudsmit J, Danner SA, Haase AT. Kinetics of response in lymphoid tissues to antiretroviral therapy of HIV-1 infection. Science 1997;276:960-4; Soontornniyomkij V, Wang G, Kapadia SB, Achim CL, Wiley CA. Confocal microscopy assessment of lymphoid tissues with follicular hyperplasia from patients infected with human immunodeficiency virus type 1. Arch Pathol Lab Med 1998;122:534-8; and Haase AT. Population biology of HIV-1 infection: viral and CD4+ T cell demographics and dynamics in lymphatic tissues. Annu Rev Immunol 1999;17:625-56). In quantitative terms, follicular dendritic cells are the major HIV reservoir population in the lymphoid organs and tissues.

*The Central Nervous System:* By infecting the central nervous system, HIV causes various neurobehavioral and neuropathological symptoms. In its severe form, they are commonly referred to as HIV-associated dementia (HAD). In persons benefiting from state-of-the-art treatment, this syndrome is less severe and termed minor cognitive motor disorder (MCMD). Today, HIV reservoir cells in the brain are thought to mediate the neurogenerative processes leading to HAD or MCDM (Gonzáles-Scarano F, Martin-Garcia J. The neuropathogenesis of AIDS. Nature Rev 2005;5:69-81). Due to the accumulation of demonstrable HIV virions, early findings suggested the choroid plexus as yet another HIV reservoir site (Hanly A, Petito CK. HLA-DR-positive dendritic cells of the normal human choroid plexus: a potential reservoir of HIV in the central nervous system. Hum Pathol 1998;29:88-93; and Petito CK, Chen H, Mastri AR, Torres-Munoz J, Roberts B, Wood C. HIV infection of choroid plexus in AIDS and asymptomatic HIV-infected patients suggests that the choroid plexus may be a reservoir of productive infection. J Neurovirol 1999;5:670-7). However, scattered throughout the brain, microglia and astrocytes are HIV-infected as well. When trying to pinpoint potential entry factors, Liu *et al.* found that microglia express CD4⁺, CCR5⁺, and CCR3⁺, all of which are known to participate in T-cell infection. Astrocytes, on the other hand, do not express CD4 (the key HIV receptor in T cells), but only the co-factors CCR5 and PCR3. Nevertheless, it then turned out that both cell types, with the mannose receptor, express a C-type lectin, which was shown to be essential for CD4-independent HIV-1 infectivity in astrocytes and microglia. Importantly, upon binding the virus, subsequent intracellular infection depends upon endocytic trafficking of HIV, as the process can be blocked by endosomo- and lysosomotropic inhibitors (Liu Y, Liu H, Kim BO, Gattone VH, Li J, Nath A, Blum J, He JJ. CD4-independent infection of astrocytes by human immunodeficiency virus type 1: requirement for the human mannose receptor. J Virol 2004;78:4120-33. Erratum in: J Virol 2004;78:7288-9). These findings are in line with the fact that the mannose receptor C-type lectin mediates pinocytosis by both astrocytes and microglia, and phagocytosis by microglia (Regnier-Vigouroux A. The mannose receptor in the brain. Int Rev Cytol 2003;226:321-42). Finally, in expanding on a previous study revealing a population of HIV⁺ stromal cells in the choroid plexus, Hanly *et al.* then, for the first time, documented a dendritic-cell population in the normal human choroid plexus. The authors also showed that, in this location, exactly these cells harbor HIV and, thus, are the cellular HIV reservoir of the choroid plexus (Hanly A, Petito CK. HLA-DR-positive dendritic cells of the normal human choroid plexus: a potential reservoir of HIV in the central nervous system. Hum Pathol 1998;29:88-93). Thus, the HIV reservoir cells in the human brain are of myelomonocytic origin and express C-type lectin receptors.

*Placenta, Cord Blood, Lactating Breast, and Cervix:* Importantly, the female organism features further anatomical HIV reservoirs that enable mother-to-child transmission of the virus during pregnancy and, potentially, after birth. As to the transmission to the embryo/fetus, it has been known for a while that HIV infection effects cellular phenotypic changes in the placenta (Goldstein J, Braverman M, Salafia C, Buckley P. The phenotype of human placental macrophages and its variation with gestational age. Am J Pathol 133;1988:648-59). Even before these findings, a mannose receptor had been identified in the human placenta (Lennartz MR, Cole FS, Shepherd VL, Wileman TE, Stahl PD. Isolation and characterization of a mannose-specfic endocytosis receptor from human placenta. J Biol Chem 1987;262:9942-4) which, when characterized, revealed carbohydrate recognition domains (Taylor ME, Conary JT, Lennartz MR, Stahl PD, Drickamer K. Primary structure of the mannose receptor contain multiple motifs resembling carbohydrate-recognition domains. J Biol Chem 1990;265:12156-62). Eventually, this mannose receptor was correlated with efficient HIV binding (Curtis BM, Schamowske S, Watson AJ. Sequence and expression of a membrane-associated C-type lectin that exhibits CD4-independent binding of human immunodeficiency virus envelope glycoprotein gp120. Proc Natl Acad Sci USA 1992;89:8356-60). As to its actual cellular expression, Soilleux and colleagues then identified the receptor on placental dendritic cells (Soilleux EJ, Barten R, Trowsdale J. DC-SIGN; a related gene, DC-SIGNR; and CD23 form a cluster on 19p13. J Immunol 2000;165:2937-42). Moreover, it became apparent that placental tissue also expresses other C-type lectins, such as hSRCL types I and II (Nakamura K, Funakoshi H, Miyamoto K, Tokunaga F, Nakamura T. Molecular cloning and functional characterization of a human scavenger receptor with C-type lectin (SRCL), a novel member of a scavenger receptor family. Biochem Biophys Res Commun 2001;280:1028-35), as well as CL-P1, which is restricted to the placental vascular endothelial cells (Ohtani K, Suzuki Y, Eda S, Kawai T, Kase T, Keshi H, Sakai Y, Fukuoh A, Sakamoto T, Itabe H, Suzutani T, Ogasawara M, Yoshida I, Wakamiya N. The membrane-type collectin CL-P1 is a scavenger receptor on vascular endothelial cells. J Biol Chem 2001;276:44222-8. Epub 2001 Sep 19). Finally, DC-SIGN was also found expressed in the placenta and first demonstrated on specialized placental macrophages (Soilleux EJ, Morris LS, Leslie G, Chehimi J, Luo Q, Levroney E, Trowsdale J, Montaner LJ, Doms RW, Weissman D, Coleman N, Lee B. Constitutive and induced expression of DC-SIGN on dendritic cell and macrophage subpopulations in situ and in vitro. J Leukoc Biol 2002;71:445-57). Yet importantly, Kämmerer *et al.* then identified a uterine immature DC-SIGN⁺ dendritic-cell population restricted to the pregnancy-associated decidua. The case that these cells revealed high proliferation and were physically tightly associated with a natural-killer subset (Kämmerer U, Eggert AO, Kapp M, McLellan AD, Geijtenbeek TB, Dietl J, van Kooyk Y, Kämpgen E. Unique appearance of proliferating antigen-presenting cells expressing DC-SIGN (CD209) in the decidua of early human pregnancy. Am J Pathol 2003;162:887-96) indicates that they are of major importance in the mother-to-child transfer of HIV. Finally, further cells of major concern for the pregnancy-associated transfer of HIV. are the cord-blood monocytes which can give rise to HIV-infected dendritic cells (Folcik RM, Merrill JD, Li Y, Guo CJ, Douglas SD, Starr SE, Ho WZ. HIV-1 infection of placental cord blood monocyte-derived dendritic cells. J Hematother Stem Cell Res 2001;10:609-20), even more so when viewed on the background that their precursor monocytes are already susceptible to HIV infection (Weinberg JB, Matthews TJ, Cullen BR, Malim MH. Productive human immunodeficiency virus type 1 (HIV-1) infection of nonproliferating human monocytes. J Exp Med 1991;174,1477-82; and Zhu T. HIV-1 genotypes in peripheral blood monocytes. J Leukoc Biol 2000;68:338-44).

In contrast, not much is currently known about a mother-to-child transmission of HIV after birth. Still, Ichikawa and colleagues found that healthy peripheral blood monocytes, when exposed to breast milk *in vitro,* started producing GM-CSF, which they usually are incapable of. Moreover, when adding exogenous IL-4 only, these cells differentiated into CD1⁺ myeloid dendritic cells. Most intriguingly, macrophages freshly isolated from breast milk were also found to produce GM-CSF in an autocrine fashion and, in the presence exogenous IL-4 alone, to differentiate into dendritic cells that efficiently stimulated T-cell responses (Ichikawa M, Sugita M, Takahashi M, Satomi M, Takeshita T, Araki T, Takahashi H. Breast milk macrophages spontaneously produce granulocyte-macrophage colony-stimulating factor and differentiate into dendritic cells in the presence of exogenous interleukin-4 alone. Immunology 2003;108:189-95). As described in detail below, monocytes, macrophages and myeloid dendritic cells all are components of the HIV reservoir. Thus, although not known at this time, in HIV-infected lactating mothers, a portion of both breast milk monocytes/macrophages and the myeloid dendritic cells easily derived therefrom can be expected to harbor HIV. If verified, this would definitely increase the risk of vertical mother-to-infant transmission of the virus *via* breast milk. In this case, and due to the fact that monocytes, macrophages and myeloid dendritic cells express C-type lectins, one would further expect breast milk to bear a mobile HIV reservoir population, with the potential to settle as a viral chimeric reservoir in the infant. Such a scenario is supported by results obtained on human cervical mucosa. When HIV-1 entry inhibitors were evaluated for their ability to prevent HIV infection of cells within, and dissemination from, the female cervix (i.e., the most important route of HIV transmission in Africa and Asia), it was shown that blockade of CD4 alone or both CCR5 and CXCR4 inhibited *localized* mucosal infection. However, HIV uptake and *systemic* dissemination by migratory cells was only inhibited when CD4 and C-type lectin receptors (including DC-SIGN) were blocked simultaneously. Such migratory cells consisted of two major subsets (CD3⁺HLA-DR⁻ and CD3⁻HLA-DR⁺), with the HLA-DR⁺ subset accounting for as much as 90% of HIV dissemination and mostly expressing DC-SIGN (Hu Q, Frank I, Williams V, Santos JJ, Watts P, Griffin GE, Moore JP, Pope M, Shattock RJ. Blockade of attachment and fusion receptors inhibits HIV-1 infection of human cervical tissue. J Exp Med 2004;199:1065-75. Epub 2004 Apr 12). Myelomonocytic descendants, therefore, appear to be the major HIV reservoir in pregnancy-associated placental decidua, cervical mucosal tissue and, very likely, the lactating breast. In pregnancy and nursing, such cells may transfer HIV directly to the child's T cells and may, in part, also act as mobile cellular HIV reservoirs with a potential to settle within the newborn's organism.

### 2. The Cellular HIV Reservoir

*Follicular Dendritic Cells (FDCs):* As to the actual cellular HIV reservoirs found in the sites mentioned above, FDCs are the most abundant type of cell known to retain HIV for prolonged periods of time. Due to this fact, latency of HIV (then still referred to as HTLV-III/LAV) was first identified in 1986 in FDCs by Klara Tenner-Racz and colleagues. Indeed, this finding also led to the first suggestion of an obviously important intracellular viral reservoir in HIV disease (Tenner-Racz K, Racz P, Bofill M, Schulz-Meyer A, Dietrich M, Kern P, Weber J, Pinching AJ, Veronese-Dimarzo F, Popovic M, et al. HTLV-III/LAV viral antigens in lymph nodes of homosexual men with persistent generalized lymphadenopathy and AIDS. Am J Pathol 1986;123:9-15). In 1991 and 1992, Stein, Spiegel and colleagues then clearly demonstrated that FDCs are the major reservoir population, showed that these cells retain HIV within membrane-entrapped immunocomplexes, and also provided a rationale on the pathology of HIV disease which, in most of its aspects, is still in line with current understanding (Stein H, Spiegel H, Herbst H, Niedobitek G, Foss HD. Lymphoid tissues and AIDS: role of lymphocytes and follicular dendritic cells (FDC). Verh Dtsch Ges Pathol 1991;75:4-19: and Spiegel H, Herbst H, Niedobitek G, Foss HD, Stein H. Follicular dendritic cells are a major reservoir for human immunodeficiency virus type 1 in lymphoid tissues facilitating infection of CD4+ T-helper cells. Am J Pathol 1992;140:15-22). In the following years, by including both HIV and an animal model infected with the feline immunodeficiency virus (FIV), much more insight was gained on the pathogenetic relevance of this type of reservoir cell (Embretson J, Zupancic M, Ribas JL, Burke A, Racz P, Tenner-Racz K, Haase AT. Massive covert infection of helper T lymphocytes and macrophages by HIV during the incubation period of AIDS. Nature 1993;362:359-62; Tenner-Racz K, von Stemm AM, Guhlk B, Schmitz J, Racz P. Are follicular dendritic cells, macrophages and interdigitating cells of the lymphoid tissue productively infected by HIV? Res Virol 1994;145:177-82; Hurtrel B, Chakrabarti L, Hurtrel M, Bach JM, Ganiere JP, Montagnier L. Early events in lymph nodes during infection with SIV and FIV. Res Virol 1994;145:221-7; Schuurman HJ, Joling P, van Wichen DF, Rademakers LH, Broekhuizen R, de Weger RA, van den Tweel JG, Goudsmit J. Follicular dendritic cells and infection by human immunodeficiency virus type 1 - a crucial target cell and virus reservoir. Curr Top Microbiol Immunol 1995;201:161-88; Cohen OJ, Pantaleo G, Schwartzentruber DJ, Graziosi C, Vaccarezza M, Fauci AS. Pathogenic insights from studies of lymphoid tissue from HIV-infected individuals. J Acquir Immune Defic Syndr Hum Retrovirol 1995;10 (Suppl 1):S6-S14; Sprenger R, Toellner KM, Schmetz C, Luke W, Stahl-Hennig C, Ernst M, Hunsmann G, Schmitz H, Flad HD, Gerdes J et al. Follicular dendritic cells productively infected with immunodeficiency viruses transmit infection to T cells. Med Microbiol Immunol 1995;184:129-34; Burton GF, Masuda A, Heath SL, Smith BA, Tew JG, Szakal AK. Follicular dendritic cells (FDC) in retroviral infection: host/pathogen perspectives. Immunol Rev 1997;156:185-97; and Smith BA, Gartner S, Liu Y, Perelson AS, Stilianakis NI, Keele BF, Kerkering TM, Ferreira-Gonzalez A, Szakal AK, Tew JG, Burton GF. Persistence of infectious HIV on follicular dendritic cells. J Immunol 2001;166:690-6). Importantly, Tachetti and colleagues revealed that FDCs, which specifically reside in the lymph nodes' germinal centers, not only retain HIV within their outer membrane projections, but also harbor infectious virions within intracytoplasmatic compartments (Tacchetti C, Favre A, Moresco L, Meszaros P, Luzzi P, Truini M, Rizzo F, Grossi CE, Ciccone E (1997). HIV is trapped and masked in the cytoplasm of lymph node follicular dendritic cells. Am J Pathol 150:533-42). In 1999, Hlavacek *et al.* presented a first *in-silico* model for the transfer of HIV from this reservoir population (Hlavacek WS, Wofsy C, Perelson AS (1999). Dissociation of HIV-1 from follicular dendritic cells during HAART: mathematical analysis. Proc Natl Acad Sci USA 96:14681-6).

As is the case with other antigens and entire infectious agents, FDCs trap and functionally preserve trapped HIV for many months. In lymph nodes, about 90% of HIV-1 RNA is localized in the FDC network from as early as several days after infection until this network collapses in the late stage of disease. *In vivo,* at any point in time, as few as 100 HIV-harboring FDCs are sufficient to transmit HIV to other cells (Smith BA, Gartner S, Liu Y, Perelson AS, Stilianakis NI, Keele BF, Kerkering TM, Ferreira-Gonzalez A, Szakal AK, Tew JG, Burton GF. Persistence of infectious HIV on follicular dendritic cells. J Immunol 2001;166:690-6). While it was earlier believed that FDC surface-bound HIV/antibody immunocomplexes are infectious, it later turned out that HIV is actually transmitted from FDCs to Th cells in a non-complexed form attached to CD54 (ICAM-1) and/or CD11a (LFA-1) (Fujiwara M, Tsunoda R, Shigeta S, Yokota T, Baba M. Human follicular dendritic cells remain uninfected and capture human immunodeficiency virus type 1 through CD54-CD11a interaction. J Virol 1999;73:3603-7). Importantly, even upon highly active antiretroviral treatment (HAART), slowly replicating virus may replenish the FDC reservoir (Smith BA, Gartner S, Liu Y, Perelson AS, Stilianakis NI, Keele BF, Kerkering TM, Ferreira-Gonzalez A, Szakal AK, Tew JG, Burton GF. Persistence of infectious HIV on follicular dendritic cells. J Immunol 2001;166:690-6). However, the finding, in macaques, that FDCs within lymph-node germinal centers express the C-type lectin DC-SIGN (Schwartz AJ, Alvarez X, Lackner AA. Distribution and immunophenotype of DC-SIGN-expressing cells is SIV-infected and uninfected macaques. AIDS Res Hum Retroviruses 2002;18:1021-9) was recently confirmed and extended for humans in that human FDCs express both DC-SIGN and one of its phylogenetic relatives, L-SIGN (Taruishi M, Terashima K, Dewan Z, Yamamoto N, Ikeda S, Kobayashi D, Eishi Y, Yamazaki M, Furusaka T, Sugimoto M, Ishii M, Kitamura K, Yamamoto N. Role of follicular dendritic cells in the early HIV-1 infection: in vitro model without specific antibody. Microbiol Immunol 2004;48:693-702). Therefore, FDCs are an important HIV reservoir population, as HIV is retained within such cells for long times before being reintroduced to the surface, that have C-type lectin receptors. The inventions described herein target FDCs' intracellular viral reservoir sites via the C-type lectin receptors.

*Myeloid Dendritic Cells (mDCs).* Due to their role as exclusive stimulators of *primary* T-cell mediated antigen-specific immunity (i.e., the hallmark of elaborate immunity), mDCs have been dubbed "nature's adjuvant" (Steinman RM. The dendritic cell system and its role in immunogeneicity. Annu Rev Immunol 1991;9:271-96; and Banchereau J, Paczesny S, Blanco P et al. Dendritic cells: controllers of the immune system and a new promise for immunotherapy. Ann N Y Acad Sci 2003;987:180-7). As such, and with T cells as HIV's most obvious target, it is compelling that these cells may be important players in the pathogenesis of HIV disease. However, the term, mDC, denotes a cell class that comprises many systemic, local, and regional cell types expressing both overlapping and distinctive characteristics. The main subpopulations of mDCs can be classified as
(i) MDCs of solid peripheral nonlymphoid organs;
(ii) MDCs of solid lymphoid organs and tissues;
(iii) Circulating blood mDCs;
(iv) Veiled cells of the afferent lymph; and
(v) MDCs of the cerebrospinal fluid

(Peters JH, Gieseler R, Thiele B, Steinbach F. Dendritic cells: from ontogenetic orphans to myelomonocytic descendants. Immunol Today 1996;17:273-8; and Pashenkov M, Huang YM, Kostulas V, Haglund M, Soderstrom M, Link H. Two subsets of dendritic cells are present in human cerebrospinal fluid. Brain 2001;124:480-92). However, each of these five principal groups comprises various subsets >Austyn, JM. Antigen-presenting cells. Experimental and clinical studies on dendritic cells. Am J Resp Crit Care Med 2000;162:S146-50). A rough estimate is that several dozens of mDC phenotypes may exist physiologically. Moreover, it is known that mDCs, at a stage in their life, leave given tissues, extensively migrate throughout the body, and home to new anatomic sites (F Steinbach, R Gieseler, A Soruri, B Krause & JH Peters: Myeloid DCs deduced from monocytes: in-vitro and in-vivo data support a monocytic origin of DCs. Adv Exp Med Biol 1997;417:27-32). Still, migration patterns observed in healthy persons profoundly change in disease (Austyn, JM. Antigen-presenting cells. Experimental and clinical studies on dendritic cells. Am J Resp Crit Care Med 162;S146-50). For these reasons, mDCs are quite an elusive subject for pinpointing a specific role in HIV disease. Moreover, due to their plethora of subsets, it is highly questionable whether results obtained on one, or a few, mDC types can allow to draw conclusions for the cell class *in toto*.

Nevertheless, at a time when mDCs still were referred to as "accessory cells", Müller *et al.* first demonstrated that HIV actually targets and infects mDCs (Müller H, Falk S, Stutte HJ. Accessory cells as primary target of human immunodeficiency virus HIV infection. J Clin Pathol 1986;39:1161). This finding was followed by various *in-vitro* and *in-vivo* studies, encompassing different types of such cells, that highlighted a variety of mDC-associated pathogenetic aspects (Knight SC, Macatonia SE. Dendritic cells and viruses. Immunol Lett 1988;19:177-81; Rappersberger K, Gartner S, Schenk P, Sting1 G, Groh V, Tschachler E, Mann DL, Wolff K, Konrad K, Popovic M. Langerhans' cells are an actual site of HIV-1 replication. Intervirology 1988;29:185-94; Langhoff E, Terwilliger EF, Bos HJ, Kalland KH, Poznansky MC, Bacon OM, Haseltine WA. Replication of human immunodeficiency virus type 1 in primary dendritic cell cultures. Proc Natl Acad Sci USA 1991;88:7998-8002; Kalter DC, Gendelman HE, Meltzer MS. Monocytes, dendritic cells, and Langerhans cells in human immunodeficiency virus infection. Dermatol Clin 1991;9:415-28; Dusserre N, Dezutter-Dambuyant C, Mallet F, Delorme P, Philit F, Ebersold A, Desgranges C, Thivolet J, Schmitt D. In vitro HIV-1 entry and replication in Langerhans cells may clarify the HIV-1 genome defection by PCR in epidermis of seropositive patients. J Invest Dermatol 1992;99:99S-102S; Chehimi J, Prakash K, Shanmugam V, Collman R, Jackson SJ, Bandyopadhyay S, Starr SE. CD4-independent infection of human peripheral blood dendritic cells with isolates of human immunodeficiency virus type 1. J Gen Virol 1993;74:1277-85; Hosmalin A. Dendritic cells of spleen and blood and HIV-1 infection. Pathol Biol (Paris) 1995;43:889-96; Dezutter-Dambuyant C, Charbonnier AS, Schmitt D. Epithelial dendritic cells and HIV-1 infection in vivo and in vitro. Pathol Biol (Paris) 1995;43:882-8; Zambruno G, Giannetti A, Bertazzoni U, Girolomoni G. Langerhans cells and HIV infection. Immunol Today 1995;16:520-4; Grouard G, Clark EA. Role of dendritic and follicular dendritic cells in HIV infection and pathogenesis. Curr Opin Immunol 1997;9:563-7; and Charton-Bain MC, Terris B, Dauge MC, Marche C, Walker F, Bouchaud O, Xerri L, Potet F. Reduced number of Langerhans cells in oesophageal mucosa from AIDS patients. Histopathology 1999;34:399-404).

In the course of these early years, it had become apparent that both HIV RNA and proviral DNA can be detected in mDCs (Giannetti A, Zambruno G, Cimarelli A, Marconi A, Negroni M, Girolomoni G, Bertazzoni U. Direct detection of HIV-1 RNA in epidermal Langerhans cells of HIV-infected patients. J Acquir Immune Defic Syndr 1993;6:329-33; and Patterson S, Roberts MS, English NR, Macatonia SE, Gompels MN, Pinching AJ, Knight SC. Detection of HIV DNA in peripheral blood dendritic cells of HIV-infected individuals. Res Virol 1994;145:171-6). However, the key discoveries leading to the current understanding on the role of mDCs as HIV reservoir cells trace back to a study in which Rossi *et al.* could specify that such cells express a C-type lectin (Rossi G, Heveker N, Thiele B, Gelderblom H, Steinbach F. Development of a Langerhans cell phenotype from peripheral blood monocytes. Immunol Lett 1992;31:189-97). Eight years later, Teunis Geijtenbeek, Yvette van Kooyk and colleagues first characterized this molecule structurally, termed it dendritic cell-specific intercellular adhesion molecule (ICAM)-3-grabbing nonintegrin (DC-SIGN; CD209) (Geijtenbeek TB, Torensma R, van Vliet SJ, van Duijnhoven GC, Adema GJ, van Kooyk Y, Figdor CG. Identification of DC-SIGN, a novel dendritic cell-specific ICAM-3 receptor that supports primary immune responses. Cell 2000;100:575-85), and demonstrated a role for DC-SIGN in the mDC-T-cell transfer of HIV (Geijtenbeek TB, Kwon DS, Torensma R, van Vliet SJ, van Duijnhoven GC, Middel J, Comelissen IL, Nottet HS, KewalRamani VN, Littman DR, Figdor CG, van Kooyk Y. DC-SIGN, a dendritic cell-specific HIV-1-binding protein that enhances trans-infection of T cells. Cell 2000;100:587-97).

Being aware of the essential immunologic role of mDCs, this discovery let many infectious-disease researchers focus on C-type lectins more closely. It has meanwhile turned out that DC-SIGN is most highly expressed by immature mDCs (Soilleux EJ, Morris LS, Leslie G, Chehimi J, Luo Q, Levroney E, Trowsdale J, Montaner LJ, Doms RW, Weissman D, Coleman N, Lee B. Constitutive and induced expression of DC-SIGN on dendritic cell and macrophage subpopulations in situ and in vitro. J Leukoc Biol 2002;71:445-57), i.e., the dendritic cells that populate the peripheral non-lymphoid tissues before emigrating to lymphoid sites where maturing for T-cell instruction. In case of an infection, all peripheral organs, therefore, feature an mDC stage excellently furnished with a mechanism for fixating, internalizing, and digesting the agent. However, reservoir formation occurs when such an agent (e.g., HIV) can decapacitate intracellular degradation and thus remains intact in a highly infectious form. C-type lectins in general play a crucial role in this process, and thereby allow for the establishment of endosomal/intracellular pathogen (e.g., HIV) reservoirs in cells expressing such receptors (Gieseler RK, Marquitan G, Hahn MJ, Perdon LA, Driessen WH, Sullivan SM, Scolaro MJ. DC-SIGN-specific liposomal targeting and selective intracellular compound delivery to human myeloid dendritic cells: implications for HIV disease. Scand J Immunol 2004;59:415-24; and references therein). In essence, the infectious agent turns its host cell into a "Trojan horse"-like reservoir where being hidden from any immunologic attack.

Among mDCs, reservoir formation, quantitatively (yet, by no means, exclusively), occurs in the cutaneous mDCs of the skin (Simonitsch I, Geusau A, Chott A, Jurecka W. Cutaneous dendritic cells are main targets in acute HIV-1-infection. Mod Pathol 2000;13:1232-7; and Turville SG, Cameron PU, Handley A, Lin G, Pöhlmann S, Doms RW, Cunningham AL. Diversity of receptors binding HIV on dendritic cell subsets. Nat Immunol 2002;3:975-83). However, the problem expands as such peripheral mDCs emigrate to the lymphoid organs. Here, "Trojan" HIV-laden mDCs arrive right amongst myriads of T cells. Once matured, mDCs establish tight immunological synapses with such cells, so that the mDC's CTL receptors can most easily transfer HIV to the T cells by processes known as *cis* transfer and *in-trans* (or *trans*) infection (reviewed in Turville S, Wilkinson J, Cameron P, Dable J, Cunningham AL. The role of dendritic cell C-type lectin receptors in HIV pathogenesis. J Leukoc Biol 2003;74:710-8. Epub 2003 Sep 02). Geijtenbeek, van Kooyk and their colleagues recently provided most direct proof that DC-SIGN infectiously transfers HIV from mDCs to T cells (Arrighi JF, Pion M, Wiznerowicz M, Geijtenbeek TB, Garcia E, Abraham S, Leuba F, Dutoit V, Ducrey-Rundquist O, van Kooyk Y, Trono D, Piguet V. Lentivirus-mediated RNA interference of DC-SIGN expression inhibits human immunodeficiency virus transmission from dendritic cells to T cells. J Virol 2004;78:10848-55). However, other CTL receptors, i.e., Langerin (CD207; exclusively expressed by immature epidermal and mucosal Langerhans-type mDCs), the mannose receptor (MR, CD206; most prominently expressed by dermal mDCs), and perhaps DEC-205 (CD205) are at least equally important (Turville SG, Cameron PU, Handley A, Lin G, Pöhlmann S, Doms RW, Cunningham AL. Diversity of receptors binding HIV on dendritic cell subsets. Nat Immunol 2002;3:975-83; and reviewed in van Kooyk Y, Geijtenbeek TB. DC-SIGN: escape mechanism for pathogens. Nat Rev Immunol 2003;3:697-709). In 2001, another mDC-expressed C-type lectin, DLEC, was characterized (Arce I, Roda-Navarro P, Montoya MC, Hernanz-Falcon P, Puig-Kroger A, Fernandez-Ruíz E. Molecular and genomic characterization of human DLEC, a novel member of the C-type lectin receptor gene family preferentially expressed on monocyte-derived dendritic cells. Eur J Immunol 2001;31:2733-40). Its potential role in reservoir formation has not yet been investigated, but appears as likely as that verified for all other CTL receptors thus far examined. Finally, when transmitting HIV to a T cell, viral replication may be initiated as early as during the transfer within the mDC itself (Granelli-Piperno A, Finkel V, Delgado E, Steinman RM. Virus replication begins in dendritic cells during the transmission of HIV-1 from mature dendritic cells to T cells. Curr Biol 1999;14:21-9).

There are several decisive factors for mDCs to strike as the most dangerous and virulent type of HIV reservoir cell. First, these cells have a high turnover rate which, depending on the subset, ranges between a few days to more than one month (Ruedl C, Koebel P, Bachmann M, Hess M, Karjalainen K. Anatomical origin of dendritic cells determines their life span in peripheral lymph nodes. J Immunol 2000;165:4910-6; Kamath AT, Pooley J, O'Keeffe MA, Vremec D, Zhan Y, Lew AM, D'Amico A, Wu L, Tough DF, Shortman K. The development, maturation, and turnover rate of mouse spleen dendritic cell populations. J Immunol 2000;165:6762-70; and Kamath AT, Henri S, Battye F, Tough DF, Shortman K. Developmental kinetics and lifespan of dendritic cells in mouse lymphoid organs. Blood 2002;100:1734-41). This implies that relative frequent, and overlapping, new generations of mDCs can sequentially be infected by HIV, form intracellular reservoirs, and thus transfer much larger amounts of virus to T cells than a type of reservoir cell that replenishes at low rates, lasts for years and, in comparison, incorporates only relatively low amounts of virus. Second, once matured, mDCs specifically take residence in the paracortical areas of the lymphoid organs where physically tightly interacting at extremely high frequency with T lymphocytes for stimulating T-cell responses (Steinman RM. The dendritic cell system and its role in immunogeneicity. Annu Rev Immunol 1991;9:271-96; and Banchereau J, Paczesny S, Blanco P et al. Dendritic cells: controllers of the immune system and a new promise for immunotherapy. Ann N Y Acad Sci 2003;987:180-7). Out of functional necessity, about 99% of all systemic T cells thus reside in exactly the same subhistologic regions. Intriguingly, about 99% of HIV-infected T cells are also found in these sites (Snedecor SJ. Comparison of three kinetic models of HIV-1 infection: implications for optimization of treatment. J Theor Biol 2003;221:519-41).

Internalization of HIV into endosomes is a committed step in both immature and mature mDCs, yet with potentially different outcomes:
(i) In both stages of mDC development, endosomes are the starting point from where the virus is directed to mDC-T-cell synapses as sites of secondary T-cell infection (McDonald D, Wu L, Bohks SM, KewalRamani VN, Unutmaz D, Hope TJ. Recruitment of HIV and its receptors to dendritic cell-T cell junctions. Science 2003;300:1295-7. Epub 2003 May 01; and Turville SG, Santos JJ, Frank I, Cameron PU, Wilkinson J, Miranda-Saksena M, Dable J, Stössel H, Romani N, Piatak M Jr, Lifson JD, Pope M, Cunningham AL. Immunodeficiency virus uptake, turnover, and 2-phase transfer in human dendritic cells. Blood 2004;103:2170-9. Epub 2003 Nov 20).
(ii) However, when infecting a dendritic cell in its immature stage, HIV can also force such a cell to integrate its proviral DNA. Upon maturation, the mDC may then start to produce virions *de novo* (Turville SG, Santos JJ, Frank I, Cameron PU, Wilkinson J, Miranda-Saksena M, Dable J, Stössel H, Romani N, Piatak M Jr, Lifson JD, Pope M, Cunningham AL. Immunodeficiency virus uptake, turnover, and 2-phase transfer in human dendritic cells. Blood 2004;103:2170-9. Epub 2003 Nov 20).
The inventions disclosed herein prevent both processes by therapeutic targeting of the endosomal compartment as HIV's intracellular turnstile. The inventions also target the mDC class in unison as mDCs abundantly display C-type lectin receptors as a common denominator bridging all phenotypic diversity..

*Macrophages.* Macrophages are critical in adaptive and specific immune responses because of their abilities to phagocytose, produce cytokines, and process/present antigens for stimulating *secondary* antigen-specific immune responses by T cells (North RJ. The relative importance of blood monocytes and fixed macrophages to the expression of cell-mediated immunity to infection. J Exp Med 1970;132:521-30; and Bancroft GJ, Schreiber RD, Unanue ER. Natural immunity: a T-cell-independent pathway of macrophage activation, defined in the scid mouse. Immunol Rev 1991;124:5-24). As a major cell population of the human immune system, macrophages are targeted by HIV and exploited as a viral reservoir (Wahl SM, Orenstein JM, Smith PD. Macrophage functions in HIV-1 infection. 1996; New York, Plenum). Importantly, macrophages express the mannose-receptor C-type lectin so abundantly that it was first characterized in these cells and originally termed "macrophage mannose receptor" (Barratt G, Tenu JP, Yapo A, Petit JF. Preparation and characterisation of liposomes containing mannosylated phospholipids capable of targetting drugs to macrophages. Biochim Biophys Acta 1986;862:153-64). Regionally confined macrophages, such as microglia (Takahashi K. Development and differentiation of macrophages and related cells: historical review and current concepts. J Clin Exp Hematopathol 2001;41:1-33) also express this receptor (Liu Y, Liu H, Kim BO, Gattone VH, Li J, Nath A, Blum J, He JJ. CD4-independent infection of astrocytes by human immunodeficiency virus type 1: requirement for the human mannose receptor. J Virol 2004;78:4120-33. Erratum in: J Virol 2004;78:7288-9). Binding to the mannose receptor accounts for 60% of the initial association of HIV with DC-SIGN-negative macrophages, which have been verified to transfer HIV to T cells. Direct HIV transmission to T cells occurs for up to approximately 24 hours due to rapid mannose receptor-mediated internalization of HIV, thus shifting the virus to the intracellular infectious reservoir (Nguyen DG, Hildreth JE. Involvement of macrophage mannose receptor in the binding and transmission of HIV by macrophages. Eur J Immunol 2003;33:483-93). Specifically, the crucial involvement of macrophages in secondary T-cell stimulation makes them a dangerous reservoir population for the transfer of virions to T cells. Interestingly, expression of another C-type lectin, DC-SIGN (which is a prominent HIV transmission factor in myeloid dendritic cells; see above), is also inducible in macrophages in the presence of cytokines. Like in myeloid dendritic cells, DC-SIGN can bind additional virions and transfer them to T cells (Chehimi J, Luo Q, Azzoni L, Shawver L, Ngoubilly N, June R, Jerandi G, Farabaugh M, Montaner LJ. HIV-1 transmission and cytokine-induced expression of DC-SIGN in human monocyte-derived macrophages. J Leukoc Biol 2003;74:757-63. Epub 2003 Aug 21). In macrophages, a reservoir-forming role for DC-SIGN has not yet been investigated, but is most likely, due to the molecule's cellular internalization cycle typical for C-type lectins and documented in other cells. Depending on the regional subset, macrophages have a life-span of months to years (Takahashi K. Development and differentiation of macrophages and related cells: historical review and current concepts. J Clin Exp Hematopathol 2001;41:1-33). Such periods of time may also be envisioned for a portion of the macrophage-restricted HIV reservoir, although it also known that HIV-infected macrophages can undergo apoptosis (Wang X, Lewis DE. CD86 expression correlates with amounts of HIV produced by macrophages in vitro. J Leukoc Biol 2001;69:405-13), and, thus, will die off at much earlier times. The inventions disclosed herein likewise target this cell class since, such cells, both constitutively and facultatively, express C-type lectins.

*Plasmacytoid Dendritic Cells (pDCs).* In humans, pDCs (a.k.a. plasmacytoid monocytes; plasmacytoid T cells) act as natural type-I interferon (IFN-α/- β)-producing cells and, therefore, are important first-line antiviral responders (Bjorck P. Isolation and characterization of plasmacytoid dendritic cells from Flt3 ligand and granulocyte-macrophage colony-stimulating factor-treated mice. Blood 2001;98:3520-6). They represent a minor population in all tissues, even including cerebrospinal fluid, but still are ontogenetically disputed (Banchereau J, Pulendran B, Steinman R, Palucka K. Will the making of plasmacytoid dendritic cells in vitro help unravel their mysteries? J Exp Med 2000;192:F39-44; Pashenkov M, Huang YM, Kostulas V, Haglund M, Soderstrom M, Link H. Two subsets of dendritic cells are present in human cerebrospinal fluid. Brain 2001;124:480-92). First studies on C-type lectins in differentiated pDCs showed that these cells express the blood dendritic cell antigen-2 (BDCA-2), but not DC-SIGN (Dzionek A, Sohma Y, Nagafune J, Cella M, Colonna M, Facchetti F, Gunther G, Johnston I, Lanzavecchia A, Nagasaka T, Okada T, Vermi W, Winkels G, Yamamoto T, Zysk M, Yamaguchi Y, Schmitz J. BDCA-2, a novel plasmacytoid dendritic cell-specific type II C-type lectin, mediates antigen capture and is a portent inhibitor of interferon α/β induction. J Exp Med 2001;194:1823-34; and Patterson S, Rae A, Hockey N, Gilmour J, Gotch F. Plasmacytoid dendritic cells are highly susceptible to human immunodeficiency virus type 1 infection and release infectious virus. J Virol 2001;75:6710-3). However, both in fetuses and adults, a small subset of blood-borne pDC precursors, termed pDC2, is BDCA-2/DC-SIGN double-positive (Soilleux EJ, Morris LS, Leslie G, Chehimi J, Luo Q, Levroney E, Trowsdale J, Montaner LJ, Doms RW, Weissman D, Coleman N, Lee B. Constitutive and induced expression of DC-SIGN on dendritic cell and macrophage subpopulations in situ and in vitro. J Leukoc Biol 2002;71:445-57).

Upon HIV infection, both pDCs and their precursors develop functional deficits (Foussat A, Bouchet-Delbos L, Berrebi D, Durand-Gasselin I, Coulomb-L'Hermine A, Krzysiek R, Galanaud P, Levy Y, Emilie D. Deregulation of the expression of the fractalkine/fractalkine receptor complex in HIV-1-infected patients. Blood 2001;98:1678-86; and Feldman S, Stein D, Amrute S, Denny T, Garcia Z, Kloser P, Sun Y, Megjugorac N, Fitzgerald-Bocarsly P. Decreased interferon-alpha production in HIV-infected patients correlates with numerical and functional deficiencies in circulating type 2 dendritic cell precursors. Clin Immunol 2001;101:201-10). Yet, shortly after HIV infection, the rapid decrease in numbers of both subsets actually precedes any other cellular alteration (Donaghy H, Pozniak A, Gazzard B, Qazi N, Gilmour J, Gotch F, Patterson S. Loss of blood CD11c+ myeloid and CD11c- plasmacytoid dendritic cells in patients with HIV-1 infection correlates with HIV-1 RNA virus load. Blood 2001;98:2574-6; and Chehimi J, Campbell DE, Azzoni L, Bacheller D, Papasavvas E, Jerandi G, Mounzer K, Kostman J, Trinchieri G, Montaner LJ. Persistent decreases in blood plasmacytoid dendritic cell number and function despite effective highly active antiretroviral therapy and increased blood myeloid dendritic cells in HIV-infected individuals. J Immunol 2002;168:4796-801). PDCs, therefore, are sensitive to HIV infection. Although no data are as yet available on a potential role of their CTL receptors, BDCA-2 and DC-SIGN are likely involved in virus binding and uptake. This might transform pDCs into an HIV reservoir cell although, clearly, pDCs are not listed among the known reservoir cells. The inventions disclosed herein may successfully target pDCs via CTL receptors. In addition, such inventions may prove helpful for interfering with a virus which so seriously harms this antiviral type of cell.

*T-Memory and Natural Killer Cells*: A most worrisome HIV reservoir consists of latently infected resting CD4⁺ T-memory cells carrying integrated proviral HIV DNA (Pierson T, McArthur J, Siliciano RF. Reservoirs for HIV-1: mechanisms for viral persistence in the presence of antiviral immune responses and antiretroviral therapy. Annu Rev Immunol 2000;18:665-708). When re-activated, such cells can begin to produce virus again and, thereby, release many archived strain variants, thus increasing the risk for therapy resistance (Siliciano JD, Siliciano RF. A long-term latent reservoir for HIV-1: discovery and clinical implications. J Antimicrob Chemother 2004;54:6-9. Epub 2004 May 26). Pierson *et al.* extrapolated that, at an extremely long half-life of the T-memory reservoir of 44 months, and based on state-of-the-art treatment protocols, its eradication would require over 60 years of treatment. By pointing out that hopes of eradicating HIV with current antiretroviral regimens are unrealistic and would entail substantial long-term toxicities, they concluded that new approaches for eradicating this HIV reservoir are urgently needed (Pierson T, McArthur J, Siliciano RF. Reservoirs for HIV-1: mechanisms for viral persistence in the presence of antiviral immune responses and antiretroviral therapy. Annu Rev Immunol 2000;18:665-708). Another type of HIV reservoir cell identified, but yet only incompletely explored, is a subset of CD56⁺CD3⁻ human natural killer (NK) cells expressing CD4, CCR5, and CXCR4. Once infected *via* these (co-)receptors, this NK-cell subset reveals proviral DNA and remains persistently infected in patients for (at least) 1-2 years after onset of highly active antiretroviral therapy (Valentin A, Rosati M, Patenaude DJ, Hatzakis A, Kostrikis LG, Lazanas M, Wyvill KM, Yarchoan R, Pavlakis GN. Persistent HIV-1 infection of natural killer cells in patients receiving highly active antiretroviral therapy. PNAS USA 2002;99:7015-7020).

Indirect evidence supports the concept that T-memory cells and/or natural killer cells also establish CTL receptor-dependent intracellular reservoirs of infectious intact viruses/pathogens as these cells express such receptors. First, in adults, 25% of peripheral blood CD4⁺ and CD8⁺ T cells (most of which reveal a T-memory phenotype), as well as a NK-cell subset express the NKR-P1 glycoprotein family type II CTL receptor hNKR-P1A (Lanier LL, Chang C, Phillips JH. Human NKR-P1A. A disulfide-linked homodimer of the C-type lectin superfamily expressed by a subset of NK and T lymphocytes. J Immunol 1994;153:2417-28). Second, and in contrast to adults, the cord blood of newborns contains substantial subsets of CD4⁺ and CD8⁺ T cells expressing killer cell lectin-like receptor G1 (KLRG1), an inhibitory C-type lectin. After birth, KLRG1⁺ cells rapidly shift to the anatomic effector/memory T-cell compartments as seen in adults (Marcolino I, Przybylski GK, Koschella M, Schmidt CA, Voehringer D, Schlesier M, Pircher H. Frequent expression of the natural killer cell receptor KLRG1 in human cord blood T cells: correlation with replicative history. Eur J Immunol 2004;34:2672-80). This implies a markedly increased risk for a fetus to be infected by an HIV⁺ mother via these circulating cells. We further hypothesize that these cells, owing to their relocation to the newborn's intralymphoid memory pools, may establish an HIV reservoir pool very early in life. Therefore, in pregnancy, not only CTL receptor-expressing HIV reservoir cells located in the mother's solid placental tissue (see above), but also CTL and CTLD receptors expressed by embryonal circulating cells may act as receptors enabling the formation of cellular HIV reservoirs. Both receptor types are thus implied in the mother-to-child transfer of HIV-1.

However, later in life, KLRG1 expression by T cells is considered a hallmark of dormancy or dysfunction. Most interestingly, throughout life the amount of such senescent T cells increases upon viral infection and also correlates with ageing (Voehringer D, Blaser C, Brawand P, Raulet DH, Hanke T, Pircher H. Viral infections induce abundant numbers of senescent CD8 T cells. J Immunol 2001;167:4838-43; and Ouyang Q, Wagner WM, Voehringer D, Wikby A, Klatt T, Walter S, Müller CA, Pircher H, Pawelec G. Age-associated accumulation of CMV-specific CD8+ T cells expressing the inhibitory killer cell lectin-like receptor G1 (KLRG1). Exp Gerontol 2003;38:911-20). We believe that these facts may actually correlate. It has been suggested that the overall functional T-cell repertoire may shrink as the pool of these cells expands, thus contributing to the increased incidence of infectious diseases in the elderly (Ouyang Q, Wagner WM, Voehringer D, Wikby A, Klatt T, Walter S, Müller CA, Pircher H, Pawelec G. Age-associated accumulation of CMV-specific CD8+ T cells expressing the inhibitory killer cell lectin-like receptor G1 (KLRG1). Exp Gerontol 2003;38:911-20). This may also correlate specifically to HIV, as well as other infectious diseases in which chronic intracellular pathogen reservoirs are established.

*Actively HIV-replicating T Cells*: Besides HIV reservoir cells as continuous source of infectious virions, establishment of a persistent infection also critically depends on activation signals that regulate HIV replication within target T cells. Quiescent T cells are resistant to infection unless T-cell receptor- and/or cytokine-mediated activation signals are provided (Unutmaz D. T cell signaling mechanisms that regulate HIV-1 infection. Immunol Res 2001;23:167-77). Therefore, HIV infection comprises a combination of chronic states of T-cell hyperactivation, HIV persistence, and T-cell depletion (Grossman Z, Meier-Schellersheim M, Sousa AE, Victorino RM, Paul WE. CD4+ T-cell depletion in HIV infection: are we closer to understanding the cause? Nat Med 2002;8:319-23).

Next to the long-known role of CD4+ T-helper cells as targets of HIV (Fauci AS. Multifactorial nature of human immunodeficiency virus disease: implications for therapy. Science 1993;262:1011-8), it is now apparent that a subset CD4⁺CD25^{hi} T cells is also infected. The CD4⁺CD25^{hi} subset of regulatory T cells (Treg cells) is now broadly acknowledged to suppress T-cell activation in both mice and humans (Sakaguchi S, Sakaguchi N, Asano M, Itoh M, Toda M. Immunologic selftolerance maintained by activated T cells expressing IL-2 receptor alphachains (CD25): Breakdown of a single mechanism of self-tolerance causes various autoimmune diseases. J Immunol 1995;155:1151-64; Asano M, Toda M, Sakaguchi N, Sakaguchi S. Autoimmune disease as a consequence of developmental abnormality of a T cell subpopulation. J Exp Med 1996;184:387-96; Suri-Payer E, Amar AZ, Thornton AM, Shevach EM. CD4+CD25+ T cells inhibit both the induction and effector function of autoreactive T cells and represent a unique lineage of immunoregulatoty cells. J Immunol 160;1998: 1212-8; Takahashi T, Kuniyasu Y, Toda M, Sakaguchi N, Itoh M, et al. Immunologic self-tolerance maintained by CD25+CD4+ naturally anergic and suppressive T cells: Induction of autoimmune disease by breaking their anergic/suppressive state. Int Immunol 1998;10:1969-80; Thornton AM, Shevach EM. CD4+CD25+ immunoregulatory T cells suppress polyclonal T cell activation in vitro by inhibiting interleukin 2 production. J Exp Med 1998;188:287-96; Baecher-Allan C, Brown JA, Freeman GJ, Hafler DA. CD4+CD25high regulatory cells in human peripheral blood. J Immunol 2001;167:1245-53; Dieckmann D, Plottner H, Berchtold S, Berger T, Schuler G. Ex vivo isolation and characterization of CD4+CD25+ T cells with regulatory properties from human blood. J Exp Med 2001;193:1303-10; Jonuleit H, Schmitt E, Stassen M, Tuettenberg A, Knop J, et al. Identification and functional characterization of human CD4+CD2+ T cells with regulatory properties isolated from peripheral blood. J Exp Med 2001;193:1285-94; Taams LS, Smith J, Rustin MH, Salmon M, Poulter LW, et al. Human anergic/suppressive CD4+CD25+ T cells: A highly differentiated and apoptosis-prone population. Eur J Immunol 2001;31:1122-31; Levings MK, Sangregorio R, Roncarolo MG. Human CD25+CD4+ T regulatory cells suppress naive and memory T cell proliferation and can be expanded in vitro without loss of function. J Exp Med 2001;193:1295-302; Ng WF, Duggan PJ, Ponchel F, Matarese G, Lombardi G, et al. Human CD4+CD25+ cells: A naturally occurring population of regulatory T cells. Blood 98: 2736-2744; and Jonuleit H, Schmitt E, Kakirman H, Stassen M, Knop J, et al. Infectious tolerance: Human CD25+ regulatory T cells convey suppressor activity to conventional CD4+ T helper cells. J Exp Med 2002;196:255-60. Oswald-Richter *et al.* demonstrated that human Treg cells isolated from healthy donors express the HIV-coreceptor CCR5 and are highly susceptible to HIV infection and replication. They also provided evidence for the fact that the immunosuppressive Treg cells may be disrupted in a portion of HIV-positive individuals with a low percentage of CD4⁺ and higher levels of activated T cells, which may contribute to characteristic T-cell hyperactivation in the progression of HIV disease. Infection of these cells, thus, implies the loss of regulatory immunosuppression (Oswald-Richter K, Grill SM, Shariat N, Leelawong M, Sundrud MS, Haas DW, Unutmaz D. HIV Infection of naturally occurring and genetically reprogrammed human regulatory T-cells. PLoS Biology 2004;2:0955-66).

Moreover, cytotoxic CD8⁺ T cells are infected, too, leading to the loss of antiviral responses (Livingstone WJ, Moore M, Innes D, Bell JE, Simmonds P. Frequent infection of peripheral blood CD8-positive T-lymphocytes with HIV-1. Edinburgh Heterosexual Transmission Study Group. Lancet 1996;348:649-54). The resulting state of chronic immune activation, combined with the direct destruction of CD4⁺ and CD4⁺CD25^{hi} T cells by HIV, eventually leads to AIDS, as characterized by progressive deterioration of immunity (Fauci AS. Multifactorial nature of human immunodeficiency virus disease: implications for therapy. Science 1993;262:1011-8). Thus, while not excluding benefits of a (transient) co-administration of HAART with our therapeutic system, it thus would be advantageous if this system also interfered with the active replication of HIV in T cells.

### B. Myelomonocytic Plasticity: new Implications for Infectious Reservoirs

Potentially far-reaching conclusions as to pathogen reservoirs derive from the fact that various types of reservoir cells are members of a highly plastic system of interchangeable cell types. Implications of this concept for the nature and dynamics of cellular reservoirs of infectious agents have thus far been completely neglected.

First indications for the existence of a plastic system of cells as based on the myeloid differentiation lineage trace back to the finding that peripheral blood monocytes, when entering a tissue, do not *per se* progress to the macrophage stage. Thus contrasting an earlier dogma, blood monocytes are now broadly acknowledged to give rise to both tissues macrophages and mDCs. Moreover, in the presence of appropriate signals, mDCs can even interconvert into macrophages. Owing to numerous regional subsets of both cell classes *in vivo,* this finding led to the concept of plasticity for myelomonocytic descendants (Peters JH, Ruppert J, Gieseler RKH, Najar HM, Xu H. Differentiation of human monocytes into CD14-negative accessory cells: do dendritic cells derive from the monocytic lineage? Pathobiology 1991;59:122-6; and Peters JH, Gieseler R, Thiele B, Steinbach F. Dendritic cells: from ontogenetic orphans to myelomonocytic descendants. Immunol Today 1996;17:273-8). Successively, signals were identified that differentiate monocytes into specialized types of macrophages, i.e., microglia (regional macrophages of the central nervous system) and osteoclasts (regional macrophages of the bone compartment) (Servet-Delprat C, Arnaud S, Jurdic P, Nataf S, Grasset MF, Soulas C, Domenget C, Destaing O, Rivollier A, Perret M, Dumontel C, Hanau D, Gilmore GL, Belin MF, Rabourdin-Combe C, Mouchiroud G. Flt3+ macrophage precursors commit sequentially to osteoclasts, dendritic cells and microglia. BMC Immunol 2002;3:15). It then turned out that a subset of monocytes can act as pluripotent stem cells which, in the presence of the appropriate factors, give rise to lymphocytes, as well as epithelial, endothelial, neuronal, and liver cells (Zhao Y, Glesne D, Huberman E. A human peripheral blood monocyte-derived subset acts as pluripotent stem cells. Proc Natl Acad Sci USA;2003:2426-31; and Kuman M, Okazaki Y, Kodama H et al. Human circulating CD14+ monocytes as a source of progenitors that exhibit mesenchyma cell differentiation. J Leukoc Biol 2003;74:833-45). Currently, peripheral blood monocytes also emerge as the persistently elusive precursor of follicular dendritic cells. However, in contrast to the studies mentioned above identifying a stem-cell subset among monocytes, monocyte-FDC differentiation was in this case achieved at a 1:1 ratio. It was thus suggested to now consider monocytes *in toto* as a class of circulating stem cell (Heinemann DEH, Peters JH. Follicular dendritic cells deduced from human monocytes. BMC Immunol: in press).

Implications of this novel, and increasingly solidifying, paradigm are immense and entail
(i) To broaden our understanding of the biology of myeloid differentiation plasticity;
(ii) To generate approaches for stem cell-dependent therapies defying ethical doubt; and
(iii) To reconsider current concepts of the nature of pathogen reservoirs.

As to HIV, it has more recently become evident that this virus, indeed, infects peripheral blood monocytes (Weinberg JB, Matthews TJ, Cullen BR, Malim MH. Productive human immunodeficiency virus type 1 (HIV-1) infection of nonproliferating human monocytes. J Exp Med 1991;174,1477-82; Zhu T. HIV-1 genotypes in peripheral blood monocytes. J Leukoc Biol 2000;68:338-44). The CD14^{lo}CD16^{hi} monocyte subset is most, but not exclusively, susceptible to infection (Crowe S, Zhu T, Muller WA. The contribution of monocyte infection and trafficking to viral persistence, and maintenance of the viral reservoir in HIV infection. J Leukoc Biol 2003;74:635-41. Epub 2003 Aug 21). Hence, in patients, a portion of monocytes is already infected when emigrating into the tissues.

This means that myeloid cells can, (at least) as early as at the monocytic stage, be recruited as part of the HIV reservoir. When considering monocytes as a type of pluripotent stem cell, a very diverse spectrum of primary descendants of CD14^{lo}CD16^{hi} (and other) monocytes, may thus be HIV-infected. As mentioned, monocytic differentiation into (i) mDCs; (ii) macrophages; (iii) microglia; (iv) osteoclasts; (v) follicular dendritic cells; (vi) liver cells; (vii) lymphocytes; and (viii) endothelial; (ix) epithelial; and (x) neuronal cells has so far been verified. When differentiating from infected monocytes, such progeny may be seriously hampered in fulfilling its ontogenetic potential and function. However, in any event, all monocyte derivatives become susceptible to infection at later stages of their development, depending on their expression of surface markers serving HIV for convenient entry. For example, of the ten verified types of monocyte derivatives listed, at least the first eight ones express CTL and/or CTLD receptors (see references above).

In this field of investigation, for example, it is unknown whether CD14^{lo}CD16^{hi} monocytes only give rise to a very limited set of descendants. Also, while the inducible differentiation program of monocytes obviously is broad, it is further unknown whether the diverse monocyte derivatives generated *in vitro* correspond to sizeable differentiation events *in vivo* or rather represent relatively minor salvage pathways. Thus, taken together, one cannot currently assess the physiologic extent, and scope, of monocyte-dependent broad-spectrum differentiation.

On the other hand, when focusing only on one type of monocyte derivative (mDCs), thirteen different signals that steer mDCs differentiation from (myelo-)monocytic precursors have been identified and/or investigated. These signals comprise a colorful spectrum of classical growth and differentiation factors (multi-CSF [IL-3], M-CSF, GM-CSF); cytokines (IL-4, TNF-α, IFN-γ); vitamins (α-tocopherol, calciferol, 1,25-dihydroxycalciferol, all-trans-retinoic acid); an amino-acid derivative (cis-urocanic acid); and essential fatty acids (linoleic acid, linolenic acid) (Gieseler RKH, Röber R-A, Kuhn R, Weber K, Osborn M, Peters JH: Dendritic cells derived from rat bone marrow precursors under chemically defined conditions in vitro belong to the myeloid lineage. Eur J Cell Biol 1991;54:171-81; Gieseler RKH, Peters JH. Linoleic acid supports the differentiation and enhances the accessory activity of dendritic cells in vitro. 8th International Congress of Immunology; Budapest, Hungary. Abstract Book 1992:501; Peters JH, Xu H, Ruppert J, Ostermeier D, Friedrichs D, Gieseler RKH: Signals required for differentiating dendritic cells from human monocytes in vitro. Adv Exp Med Biol 1993,329:275-80; Xu H, Soruri A, Gieseler RKH, Peters JH. 1,25-Dihydroxyvitamin D3 exerts opposing effects to IL-4 on MHC class II antigen expression, accessory activity, and phagocytosis of human monocytes. Immunobiology 1993;189:69; Peters JH, Gieseler R, Thiele B, Steinbach F. Dendritic cells: from ontogenetic orphans to myelomonocytic descendants. Immunol Today 1996;17:273-8; Steinbach F, Gieseler R, Soruri A, Krause B, Peters JH. Myeloid DCs deduced from monocytes: in-vitro and in-vivo data support a monocytic origin of DCs. Adv Exp Med Biol 1997;417:27-32; Gieseler R, Heise D, Soruri A, Schwartz P, Peters JH. In-vitro differentiation of mature dendritic cells from human blood monocytes. Develop Immunol 1998;6:25-39; Gieseler R, Schlemminger R, Fayyazi A, Peters JH. Cis-UCA Effects on Experimental Small Bowel Transplantation: Evidence for Suppression of the Graft-versus-Host Response. In: Hönigsmann H, Knobler R, Trautinger F, Jori G (eds.): Landmarks in Photobiology. OEMF Publishers spa. Milano 1998:285-8; Heise D, Peters JH, Schedlowski M, Gieseler R. Maturation of monocyte-derived dendritic cells (MoDC) by autocrine TNF-α signaling. Immunobiology 1999;200:561; and Gieseler R, Hollmann K, Scolaro MJ, Peters JH. All-trans-retinoic acid upregulates CD1a on human monocyte-derived dendritic cells (MoDC): implications for autologous melanoma-specific tumor vaccination. Immunobiology 2000;203:378-9). These findings highlight how sensitive monocytes respond to environmental stimuli and illustrate the diversity of signals these cells respond to by differentiating into different mDC subtypes. Thus, even such a very limited "one-differentiation-product scenario" impressively illuminates the potential of monocytes. Accordingly, a plethora of hitherto unidentified signals may give rise to a huge spectrum of monocytic differentiation products - a conclusion which, in essence, again arrives at the monocyte stem-cell concept (Heinemann DEH, Peters JH. Follicular dendritic cells deduced from human monocytes. BMC Immunol: in press).

Specifically, as to the formation of infectious intracellular reservoirs, and viewed on this background, two decisive factors include (i) that monocytes, by design, are able to generate a variety of differentiation products; and (ii) that monocyte differentiation is altered under pathologic conditions (e.g., Austyn, JM. Antigen-presenting cells. Experimental and clinical studies on dendritic cells. Am J Resp Crit Care Med 2000;162: S 146-50; Stucke M, Quadbeck B, Eckstein AK, Tews S, Mann K, Esser J. Gieseler R. MHC class II focusing, significant increase in CD40+ cells, and faint expression of Langerin by peripheral blood leukocytes are distinctive features of thyroid-associated ophthalmopathy. 27th Annual Meeting of the European Thyroid Association. Warsaw, Poland: Aug. 25-29, 2001; and Quadbeck B, Eckstein A, Tews S, Walz M, Hoermann R, Mann K, Gieseler R. Maturation of thyroidal dendritic cells in Graves' disease. Scand J Immunol 2002; 55:612-20). Importantly, this has also been documented for mDCs in HIV disease (briefly reviewed in Gieseler RK, Marquitan G, Scolaro MJ, Cohen MD. Lessons from history: dysfunctional APCs, dangers of STI and an important goal, as yet unmet. Trends Immunol 2003; 24:11). In addition, therapeutic drugs profoundly influence the systemic cytokine milieu governing myelomonocytic cell differentiation (e.g., Galon J, Franchimont D, Hiroi N, Frey G, Boettner A, Ehrhart-Bomstein M, O'Shea JJ, Chrousos GP, Bornstein SR. Gene profiling reveals unknown enhancing and suppressive actions of glucocorticoids on immune cells. FASEB J 2002; 16:61-71). It is therefore compelling that, under pathologic conditions and in the presence of drugs, the monocytes' pluripotent potential is drawn upon in a variety of ways presently completely unknown. These considerations suggest that, under conditions of an infectious disease, diverse types of monocyte-derived cells may give rise to a variety of pathogen reservoir cells. More importantly, such derivatives may be infected as early as at their common point of origin, i.e., the monocytic precursor. The inventions presented herein contemplate the targeted treatment of monocytes. Such treatments may have considerable benefits.

*Bone Marrow:* The recent discovery that bone marrow not only serves as a primary but also a secondary lymphoid organ (Feuerer M, Beckhove P, Garbi N, Mahnke Y, Limmer A, Hommel M, Hämmerling GJ, Kyewski B, Hamann A, Umansky V, Schirrmacher V. Bone marrow as a priming site for T-cell responses to blood-borne antigens. Nature Med 2003;9:1151-7) strongly suggests bone marrow to be an anatomic reservoir site in certain infectious diseases. In addition, bone marrow also houses the myelomonocytic precursors, as well as freshly differentiated monocytes prior to their release into the circulation. As to the dynamics of the systemic anatomic reservoir sites, these considerations favor the hypothesis that bone marrow may be a "primordial reservoir" from which all other reservoir sites are replenished. The inventions disclosed herein also includes bone-marrow-specific applications of the inventive targeting system. In fact, such a route promises to reach the major pool of reservoir-cell precursors in HIV disease and other infectious diseases before such cells progress to differentiate into various peripheral tissue-resident reservoir populations.

### II. Lectins Expressing Carbohydrate Recognition Domains

In 1988, Kurt Drickamer summarized the advances in the field of animal lectins (i.e., sugar-binding proteins) that carry defined *carbohydrate recognition domains (CRDs)* (reviewed in Drickamer K. Two distinct classes of carbohydrate-recognition domains in animal lectins. J Biol Chem 1988;263:9557-60). Today we know that CRD lectins are part of our ancient immunological heritage and can, for example, be found in species as distant as flies and humans. Importantly, by acting as specific receptors for infectious agents, many CRD lectins serve as a first line of defense (Hallman M, Ramet M, Ezekowitz RA. Toll-like receptors as sensors of pathogens. Pediatr Res 2001;50:315-21). Thus, even in relatively "simple" immune systems, CRD lectins offer a certain degree of specificity for protecting a host from infection. In evolutionary terms, the archaic and more robust branch of defense, termed *innate immunity* (which includes CRD lectins), evolved long before the more sophisticated branch of *adaptive immunity* (which, due to the massive expansion of cellular clones featuring antigen-specific structures, provides us with the luxury of highly specific protective mechanisms). Nevertheless, in humans and vertebrate animals, innate immunity continues to set the stage even for the outcome of antigen-specific responses as a result of clonal expansion and, thus, creates the foundation for an individual's overall defense (Holmskov U, Thiel S, Jensenius JC. Collections and ficolins: humoral lectins of the innate immune defense. Annu Rev Immunol 2003;21:547-78. Epub 2001 Dec 19). However, as discussed herein, some of these ancient lectins, due to adaptation by infectious agents, also play a central role in the establishment of so-called pathogen reservoirs. Such cellular reservoirs are essential for the current incurability of some of the most prevalent lethal and/or debilitating chronic *infectious* diseases in humans and higher animals.

Long before being acknowledged by immunologists, biochemists had accumulated profound knowledge on CRD receptors. However, their physiological function remained obscure. When Charles A. Janeway introduced his Infectious-Nonself Model, immunology realized, on a theoretical construct, the potential existence of archaic "broad-spectrum receptors" for pathogens. Janeway proposed that antigen-presenting cells (APCs), the backbone of the adaptive branch of immunity, may initiate protective immunity when triggered by pathogen-associated molecular patterns (PAMPs). Such triggering would require the presence of specific complementary exocellular receptors dubbed pattern recognition receptors (PRRs) (Janeway Jr, CA. Approaching the asymptote? Evolution and revolution in immunology. Cold Spring Harbor Symp Quant Biol 1989;54:1-13). Once theoretically predicted, these structures could indeed be discovered. With an increasing amount of knowledge amassed, the operative acronym, PRR, became obsolete and was replaced by an expression referring to the identified evolutionary ancestor of these structures, i.e., Toll-like receptors (TLRs) (reviewed in Johnson, GB et al. Evolutionary clues to the functions of the Toll-like family as surveillance receptors. Trends Immunol 2003;24:19-24). Functionally, all these molecules are CRD lectins.

One subgroup of the CRD lectins is the family of *C-type lectin (CTL) receptors,* which binds carbohydrates in calcium-dependent manner (Holmskov U, Malhotra R, Sim RB, Jensenius JC. Collectins: collagenous C-type lectins of the innate immune defense system. Immunol Today 1994;15:67-74). One example for CTL receptors recognizing PAMPs are the so-called *collectins* that are present in plasma and on mucosal surfaces. Human collectins thus far identified are the mannan-binding lectin (MBL) and the surfactant proteins A and D (SP-A and SP-D). When recognizing an infectious agent, they either (MBL) initiate the lectin pathway of complement activation or (SP-A, SP-D) trigger opsonization, neutralization, and agglutination to limit infection and also orchestrate the adaptive immune response (reviewed in Holmskov U, Thiel S, Jensenius JC. Collections and ficolins: humoral lectins of the innate immune defense. Annu Rev Immunol. 2003;21:547-78. Epub 2001 Dec 19). Another example for CTL receptors is the *Regenerating Gene (REG or Reg) family* that mainly plays a protective role in the hepatogastroenterological organs and tissues (reviewed in Zhang YW, Ding LS, Lai MD. Reg gene family and human diseases. World J Gastroenterol 2003;9:2635-41); the REG family is discussed herein. Yet another group of CRD lectins express C-type lectin-*like* domains (CTLDs) (for review, see Cambi A, Figdor CG. Dual function of C-type lectin-like receptors in the immune system. Curr Opin Cell Biol 2003;15:539-46).

### III. Targeted Liposomal Compound Delivery Systems

Targeted liposomes are a suitable vehicle for specifically delivering encapsulated compounds to any given cell type expressing the respective targeting structure. When employed therapeutically, such a technology highly focuses the delivered compound to the cell type(s) of choice. However, even non-targeted liposomes may be a therapeutically beneficial tool, due to the fact that the basic liposomal system itself allows uptake of encapsulated substances into a cell that otherwise might not gain access. For example, we have earlier shown inhibition of HIV propagation in infected peripheral blood mononuclear leukocytes upon delivery, with non-targeted liposomes, of *sense* DNA directed towards the HIV 5' *tat* splice acceptor site (Sullivan SM, Gieseler RK, Lenzner S, Ruppert J, Gabrysiak TG, Peters JH, Cox G, Richer L, Martin WJ, Scolaro MJ. Inhibition of human immunodeficiency virus-1 proliferation by liposome-encapsulated sense DNA to the 5' tat splice acceptor site. Antisense Res Dev; 2:187-97 [1992]). As a second example, we have also shown earlier that compounds delivered by targeted liposomes selectively reach the cell type of choice, such as myeloid dendritic cells, and deliver their contents inside such a cell type (Gieseler RK, Marquitan G, Hahn MJ, Perdon LA, Driessen WH, Sullivan SM, Scolaro MJ. DC-SIGN-specific liposomal targeting and selective intracellular compound delivery to human myeloid dendritic cells: implications for HIV disease. Scand J Immunol 2004;59:415-24).

The potential use of lipid (liposome)-drug complexes as biodegradable or biocompatible drug carriers to enhance the potency and reduce the toxicity of therapeutics was recognized since the discovery in the 1960s that the hydration of dry lipid films formed enclosed spherical vesicles or liposomes resembling miniature cellular organelles (e.g., Bangham AD. Liposomes: the Babraham connection. Chem Phys Lipids 64:275-285 [1993]). Lipid-drug complexes have long been seen as a way to potentially improve the Therapeutic Index (TI) of drugs by increasing their localization to specific organs, tissues or cells. The TI is the ratio between the median toxic dose (TD₅₀) and the median effective dose (ED₅₀) of a particular drug. However, the application of lipid-drug complexes to drug-delivery systems was not realized until 30 years later; only then were the first series of liposome-based therapeutics approved for human use by the U.S. Food and Drug Administration (FDA). Thereupon, liposomes have been used as drug carriers in pharmaceutical applications since the mid-1990s (Lian T, Ho RJY, Trends and Developments in Liposome Drug Delivery Systems, J Pharm Sci 2001;90:667-80).

Although the lipid constituents can vary, many formulations use synthetic products of a natural phospholipid (such as, mainly, phosphatidylcholine). Most of the liposome formulations approved for human use contain phosphatidylcholine (with a neutral electrostatic charge), with fatty acyl chains of varying lengths and degrees of saturation, as a major membrane building block. A fraction of cholesterol (∼30 mol%) is often included in the lipid formulation to modulate liposomal membrane rigidity, and to reduce serum-induced instability caused by the binding of serum proteins to the liposome membrane.

Based on the head group composition of the lipid and the pH, liposomes can bear a negative, neutral, or positive charge on their surface. The nature and density of the liposomes' surface charge influences their stability, kinetics, and extent of biodistribution, as well as their interaction with, and uptake of liposomes by, target cells. Specifically, liposomes with a neutral surface charge have a lower tendency to be cleared by cells of the reticuloendothelial system (RES) after systemic administration, yet have the highest tendency to aggregate. Although negatively charged liposomes reduce aggregation and reveal increased stability in suspension, their non-specific cellular uptake is increased in vivo. When containing phosphatidylserine (PS) or phosphatidylglycerol (PG), such liposomes are endocytosed at a faster rate, and to a greater extent, than neutral liposomes (Allen TM et al., Liposomes containing synthetic lipid derivatives of poly(ethylene glycol) show prolonged circulation half-lives in vivo, Biochim Biophys Acta 1066:29-36 [1991]; Lee RJ, et al., Folate-mediated tumor cell targeting of liposome-entrapped doxorubicin in vitro, Biochim. Biophys. Acta 1233:134-144 [1995]). A negative surface charge is recognized by receptors found on a variety of cells, including macrophages (Allen TM et al. [1991]; Lee RJ, et al., Delivery of liposomes into cultured KB cells via folate receptor-mediated endocytosis, J Biol Chem 269:3198-3204 [1994]).

Inclusion of some glycolipids, such as the ganglioside GM₁ or phosphotidylinositol (PI), into liposome membranes inhibits their uptake by macrophages and RES cells and results in longer circulation times. It has been suggested that a small amount of negatively charged lipids stabilize neutral liposomes against an aggregation-dependent uptake mechanism (Drummond DC et al., Optimizing liposomes for delivery of chemotherapeutic agents to solid tumors, Pharmacol Rev 51:691-743 [1999]). Positively charged, cationic liposomes, often used as a DNA condensation reagent for intracellular DNA delivery in gene therapy, have a high tendency to interact with serum proteins; this interaction results in enhanced uptake by the RES and eventual clearance by lung, liver, or spleen. This mechanism of RES clearance partly explains the low in-vivo transfection efficiency. Other factors, including DNA instability, immune-mediated clearance, inflammatory response, and tissue accessibility can also contribute to a low transfection efficiency in animals. In fact, high doses of positively charged liposomes have been shown to produce varying degrees of tissue inflammation (Scheule RK et al., Basis of pulmonary toxicity associated with cationic lipid-mediated gene transfer to the mammalian lung, Hum Gene Ther 8:689-707 [1997]).

The surface of liposome membranes can be modified to reduce aggregation and avoid recognition by the RES using hydrophilic polymers. This strategy is often referred to as surface hydration or steric modification and is often performed by incorporating gangliosides, such as GM₁, or lipids chemically conjugated to hygroscopic or hydrophilic polymers. The usual method employs polyethyleneglycol (PEG) which is conjugated to the terminal amine of phosphatidylethanolamine. Further hydrophilic polymers added to the liposome membrane surface provide an additional surface hydration layer (Torchilin VP, Immunoliposomes and PEGylated immunoliposomes: possible use of targeted delivery of imaging agents, Immunomethods 4:244-258 [1994]). As a result, the liposomes cannot be recognized by macrophages or the RES as foreign, and thus emerge phagocytic clearance. A number of systematic studies have determined the optimum size of PEG polymer and the density of the respective polymeric PEG lipid in the liposome membrane.

Early research has demonstrated that the liposome size affects vesicle distribution and clearance after systemic administration. The rate of liposome uptake by RES increases with the size of the vesicles (Hwang K, Liposome Pharmacokinetics, In: Ostro MJ, ed., Liposomes: From Biophysics to Therapeutics, New York: Marcel Dekker, pp. 109-156 [1987]). Whereas RES uptake in vivo can be saturated at high doses of liposomes or by predosing with large quantities of control liposomes, this strategy may not be practical for human use because of the adverse effects related to sustained impairment of physiological functions of the RES. Generally, an increase in size of liposomes of similar composition results in enhanced uptake by the RES (Senior J, et al., Tissue distribution of liposomes exhibiting long half-lives in the circulation after intravenous injection, Biochim Biophys Acta 839:1-8 [1985]). Most recent investigations have used unilamellar vesicles, 50-100 nm in size, for systemic drug-delivery applications.

For example, the antifungal liposome product AmBisome is formulated to the size specification of 45-80 nm to reduce RES uptake. Serum protein binding is an important factor that affects liposome size and increases the rate of clearance in vivo. Also, complement activation by liposomes, and opsonization, depend on the liposomes' size (Devine DV, et al., Liposome-complement interactions in rat serum: implications for liposome survival studies, Biochim Biophys Acta 1191:43-51 [1994]; Liu D, et al., Recognition and clearance of liposomes containing phosphatidylserine are mediated by serum opsonin, Biochim Biophys Acta 1235:140-146 [1995]). Even with the inclusion of PEG in liposome compositions to reduce serum protein binding, the upper size limit of long-circulation PEG-PE liposomes is ∼200 nm. Due to biological constraints, development of long circulating large (>500 nm) liposomes using steric stabilization methods has not been successful. Hence, considerations of liposome size and its control in manufacturing at an early stage of drug development provide a means to optimize the efficiency of liposomal drug delivery systems.

One of the key properties that make liposomes an invaluable drug delivery system is their ability to modulate pharmacokinetics of liposome-associated and/or encapsulated drugs (Hwang KJ, Padki MM, Chow DD, Essien HE, Lai JY, Beaumier PL. Uptake of small liposomes by non-reticuloendothelial tissues. Biochim Biophys Acta;901(1):88-96 [1987]; Allen TM, Hansen C, Martin F, Redemann C, Yau-Young A. Liposomes containing synthetic lipid derivatives of poly(ethylene glycol) show prolonged circulation half-lives in vivo. Biochim Biophys Acta;1066(1):29-36 [1991]; Allen TM, Austin GA, Chonn A, Lin L, Lee KC. Uptake of liposomes by cultured mouse bone marrow macrophages: influence of liposome composition and size. Biochim Biophys Acta;1061(1):56-64 [1991]; Hwang, K. [1987]; Allen T, et al., Pharmacokinetics of long-circulating liposomes, Adv Drug Del Rev 16:267-284 [1995]. Specifically, relative to the same drugs in aqueous solution, significant changes in absorption, biodistribution, and clearance of liposome-associated drug are apparent, resulting in dramatic effects on both the efficacy and toxicity of the entrapped compound (Gabizon A, Liposome circulation time and tumor targeting: implications for cancer chemotherapy, Adv Drug Del Rev 16:285-294 [1995]; Bethune C, et al., Lipid association increases the potency against primary medulloblastoma cells and systemic exposure of 1-(2-chloroethyl)-3-cyclohexyl-1-nitrosourea (CCNU) in rats, Pharm Res 16:896-903 [1999]).

The exact mechanisms of liposome biodistribution and disposition depend on their lipid composition, size, charge, and degree of surface hydration/steric hindrance. The in-vivo disposition of liposomes also depends on the route of administration. For example, immediately after intravenous administration, liposomes are usually coated with plasma proteins, taken up by the RES and eliminated (Chonn A, et al., Association of blood proteins with large unilamellar liposomes in vivo. Relation to circulation lifetimes, J Biol Chem 267:18759-18765 [1992]; Rao M, et al., Delivery of lipids and liposomal proteins to the cytoplasm and Golgi of antigen-presenting cells, Adv Drug Deliv Rev 41:171-188 [2000]).

Plasma proteins likely to interact with liposomes include albumin, lipoproteins (e.g., high-density lipoprotein [HDL] and low-density lipoprotein [LDL]), as well as cell-associated proteins. Some of these, such as HDL, can indeed destabilize the liposomes by removing phospholipids from their bilayer, thus potentially leading to premature leakage or drug dissociation. Also, to date, therapeutic applications of systemically administered liposomes have been limited by their rapid clearance from the bloodstream and their uptake by the RES (Alving C, et al., Complement-dependent phagocytosis of liposomes: suppression by 'stealth' lipids, J Liposome Res 2:383-395 [1992]). As mentioned, circulation time can be increased by reducing the liposome size and modifying the surface/steric effect with PEG derivatives. Also, liposomes with membranes engineered for sufficient stability escaping clearance by the RES are now available. Therefore, long-circulation liposomes that also significantly reduce toxicological profiles of the respective drugs can be employed to maintain and extend plasma drug levels. Nevertheless, only prolonged circulation indirectly enhances accumulation of liposome-associated drugs in the tissues or cells intended as the eventual targets.

Thus, in spite of obvious pharmacokinetical advantages as compound delivery vehicles in vivo, such results specify drawbacks with current liposomal preparations and routes of administration. As to the current invention, such drawbacks may be overcome with liposomes (i) that are delivered topically, e.g. via the intradermal or subcutaneous routes, for reaching a pre-defined location such as a lymph node, and (ii) that furnish a targeting mechanism that may specifically pinpoint pre-defined cells within a distinct anatomical site.

It is a desideratum to actively enhance targeting of liposomes so as to direct them to the cell populations of interest before substantially cleared by the RES. For example, immunoliposomes have been successfully employed to target the erythrocyte reservoirs of intracellular malarial parasites (Owais, M. et al., Chloroquine encapsulated in malaria-infected erythrocyte-specfic antibody-bearing liposomes effectively controls chloroquine-resistant Plasmodium berghei infections in mice, Antimicrob Agents Chemother 39(1):180-4 [1995]; Singh, AM et al., Use of specific polyclonal antibodies for site specific drug targeting to malaria infected erythrocytes in vivo, Indian J Biochem Biophys 30:411-3 [1993]).

It is also a desideratum to apply lipid-drug delivery systems to the fight the HIV/AIDS pandemic, with currently approximately 42 million people worldwide estimated to be infected with HIV (UNAIDS. Global summary of the HIV/AIDS epidemic: December 2003. Available from: http://www.unaids.org/wad/2003/press/Epiupdate2003 en/Epi03 02 en.htm). Indeed, anti-HIV drugs, such as nucleoside analogs (e.g., dideoxynucleoside derivatives, including 3'-azido-3'-deoxythymidine [AZT], ddC, and ddI), protease inhibitors, or phosphonoacids (e.g., phosphonoformic and phosphonoacetic acids), have previously been lipid-derivatized or incorporated into liposomes (e.g., Hostetler, KY et al., Methods of treating viral infections using antiviral liponucleotides, Ser. No 09/846,398, US 2001/0033862; U.S. Patent No. 5,223,263; Hostetler, KY et al., Lipid derivatives of phosphonoacids for liposomal incorporation and method of use, U.S. Patent No. 5,194,654; Gagné JF et al., Targeted delivery of indinavir to HIV-1 primary reservoirs with immunoliposomes, Biochim Biophys Acta 1558(2):198-210 [Feb. 2002]). Still, in one report, subcutaneous injection of liposome-encapsulated ddI to C57BL/6 mice, resulted in low accumulation of liposomes in lymph nodes, compared to intravenous injection (Harvie, P et al., Lymphoid tissues targeting of liposome-encapsulated 2',3'-dideoxyinosine, AIDS 9:701-7 [1995]).

Anti-HIV drugs have been encapsulated in the aqueous core of immunoliposomes, which include on their external surfaces antigen-specific targeting ligands (e.g., Bergeron, MG. *et al., Targeting of infectious agents bearing host cell proteins.* WO 00/66173 A3; Bergeron, MG. *et al., Liposomes encapsulating antiviral drugs,* U.S. Patent No. 5,773,027; Bergeron, MG. *et al., Liposome formulations for treatment of viral diseases,* WO 96/10399 A1; Gagné JF et al., Targeted delivery of indinavir to HIV-1 primary reservoirs with immunoliposomes, Biochim Biophys Acta 1558(2):198-210 [2002]; Dufresne I et al., Targeting lymph nodes with liposomes bearing anti-HLA-DR Fab' fragments, Biochim Biophys Acta 1421(2):284-94 [1999]; Bestman-Smith J et al., Sterically stabilized liposomes bearing anti-HLA-DR antibodies for targeting the primary cellular reservoirs of HIV-1. Biochim Biophys Acta 1468(1-2):161-74 [2000]; Bestman-Smith J et al., Targeting cell-free HIV and virally-infected cells with anti-HLA-DR immunoliposomes containing amphotericin B, AIDS 10;14(16):2457-65 [2000]).

In addition to many examples of antibody-targeted liposomes in animal models, at least one immunoliposome, DOXIL, employing a single-chain antibody raised against HER2/*neu* (e.g., Park JW, Hong K, Kirpotin DB, Colbern G, Shalaby R, Baselga J, Shao Y, Nielsen UB, Marks JD, Moore D, Papahadjopoulos D, Benz CC, Anti-HER2 Immunoliposomes: enhanced efficacy attributable to targeted delivery, Clin Cancer Res 2002;8:1172-81 [2002]), is currently clinically evaluated by the same group for therapeutically targeting certain types of breast cancer.

Other targeting approaches are based on attaching specific vector molecules to a liposome surface so as to enhance transmembrane delivery and uptake of liposome-encapsulated compounds that otherwise are only insufficiently delivered into a cell, or that are not efficiently delivered to a specifically desirable intracellular organelle (reviewed in Torchilin VP, Lukyanov AN. Peptide and protein drug delivery to and into tumors: challenges and solutions. Drug Discov Today 2003;8:259-66; Sehgal A. Delivering peptides and proteins to tumors, Drug Discov Today 8:619 [2003]; Koning GA, Storm G. Targeted drug delivery systems for the intracellular delivery of macromolecular drugs, Drug Discov Today 2003;8:482-3). Such vector molecules include protein transduction domains (PTDs) derived from various viruses or from *Drosophila antennapedia.* Of special interest for application in HIV disease are HIV Tat and its derivatives which act as PTDs (e.g., Schwarze, S.R., et al., In vivo protein transduction: delivery of a biologically active protein into the mouse, Science 285:1569-72 [1999]).

Attempts at actively targeting lymphoid cell populations with liposomes have met with some degree of success. For example, anti-HLA-DR Fab'-labeled immunoliposomes injected subcutaneously into mice accumulated in lymphoid tissues (Bestman-Smith J et al., Targeting cell-free HIV and virally-infected cells with anti-HLA-DR immunoliposomes containing amphotericin B, AIDS 10;14:2457-65 [2000]). Similarly, Gagné et al. found accumulated drug concentrations in lymph nodes of injected mice after subcutaneous injections of immunoliposome-encapsulated anti-HIV drugs (Gagné JF, Desormeaux A, Perron S, Tremblay MJ, Bergeron MG. Targeted delivery of indinavir to HTV-1 primary reservoirs with immunoliposomes. Biochim Biophys Acta 2002;1558:198-210).

WO 98/07408 A discloses a liposome comprising DOTAP (1,2-bis-(oleoyloxy)-3-(trimethylammonio)-propane) and at least one cholesterol or cholesterol derivative, an active agent (e.g. nucleic acid) and a targeting ligand.

WO 02/096390 A discloses liposomes comprising as a targeting ligand a galactose and an active agent such as a nucleic acid, DNA or a protein or an expression vector encoding for example factor VIII.

WO 00/63251 A discloses compounds that bind to a C-type lectin on the surface of a dendritic cell and suitable compositions are used for the treatment of immunosuppression or inhibition of an HIV infection.

US 5,686,103 A describes liposome preparations with a ligand having fucose as terminal moiety. The liposome might contain a cosmetic or a pharmaceutical.

WO 2005/027979 A discloses a method of delivering a drug, such as an antiviral, an antifungal, an antibacterial or immunomodulatory drug, a cytotoxic drug, for dendritic cell-mediated vaccination, to an immune cell of a mammalian subject. The lipid drug complex comprises a drug and an outer surface comprising at least one targeting ligand (CD209 specific) that specifically binds a marker on the surface of the immune cell which is infected with an infectious agent.

As to the invention described herein, specific cell populations can be targeted for intracellular delivery of therapeutically active compounds by liposomal systems targeted to CRD lectins.

The present invention provides a targeted liposomal delivery system for selectively delivering active agents, such as lectins or drugs, to cells expressing certain families of surface proteins (termed CTL and CTLD lectins) commonly displaying carbohydrate recognition domains (CRDs). These lectins are expressed by the cell populations of interest The present invention therefore addresses, inter alia, the need to incapacitate latently stored infectious agents, such as HIV or HCV, in the different populations comprising the cellular reservoirs of infected persons and those suffering from the associated disease, such as AIDS or hepatitis C, for delivering a liposomally encapsulated therapeutic compound, such as a plant lectin or a drug, for inactivating the respective infectious agent. In addition, the present invention addresses the need to target CTL or CTLD lectin-positive cells implicated in chronic degenerative or malignant non-infectious diseases for delivering a liposomally encapsulated therapeutic compound, such as an apoptosis inhibitor or a chemotherapeutic, for beneficially interfering with the respective pathogenetic process.

### BRIEF SUMMARY OF INVENTION

The present invention according to claim 1 relates to liposomes and methods of preferentially, or "actively," targeting and delivering an active agent, such as a lectin or drug, to a mammalian immune cell in vivo or in vitro. In particular, the present invention describes liposomes targeted by surface-derivatized mono- or polyfucose , thus creating a targeted compound delivery system that encapsulates a lectin, or lectins, such as, but not limited to, a lectin obtained from the plant Canavalia ensiformis (i.e., Con-A) or the plant Myrianthus holstii (i.e. MHL), to a host of pathogen reservoir cells in HIV disease, HCV-related hepatitis, tuberculosis, and various other disease entities coinciding with the formation of chronic intracellular pathogen reservoirs. These cellular sanctuaries, inter alia, include the different immunological, developmental, and anatomical subsets of dendritic cells, macrophages, and follicular dendritic cells. Liposomal cell-specific targeting is to be achieved via evolutionarily conserved receptors expressing carbohydrate recognition domains (CRDs), and specifically, the so-called C-type lectin receptors (CTLRs), including MR (CD206), langerin (CD207), DEC-205 (CD205), and DC-SIGN (CD209). All of which are so-called multilectin receptors. The objective of this invention is to provide a means for eradicating intracellular pathogen reservoirs, thus providing a most valuable novel approach for the therapy of a great variety of viral, bacterial, and fungal disease entities. In addition, the principle of an irreversible interaction of HIV with a suitable lectin may also fatally interfere with actively propagating HIV and/or other viruses and bacteria. As pointed out, variants of this strategy, including encapsulated approved drugs (such as a ehemotherapeutic) may also be applied for the treatment of chronic non-infectious diseases, especially those relating to the liver and the gastrointestinal tract. In some cases, this goal may be achieved by employing a lipsomal system with a surface-derivatized targeting mechanism.

A benefit of the invention is that it permits targeting of all major known HIV reservoir cell types. Due to the nature of a liposomal delivery system, as combined with reasonable application routes to a patient, extremely low costs and toxicities are expected when compared to conventional therapies. Finally, the invention can be applied to the treatment of a host of other diseases.

### BRIEF DESCRIPTION OF THE DRAWINGS

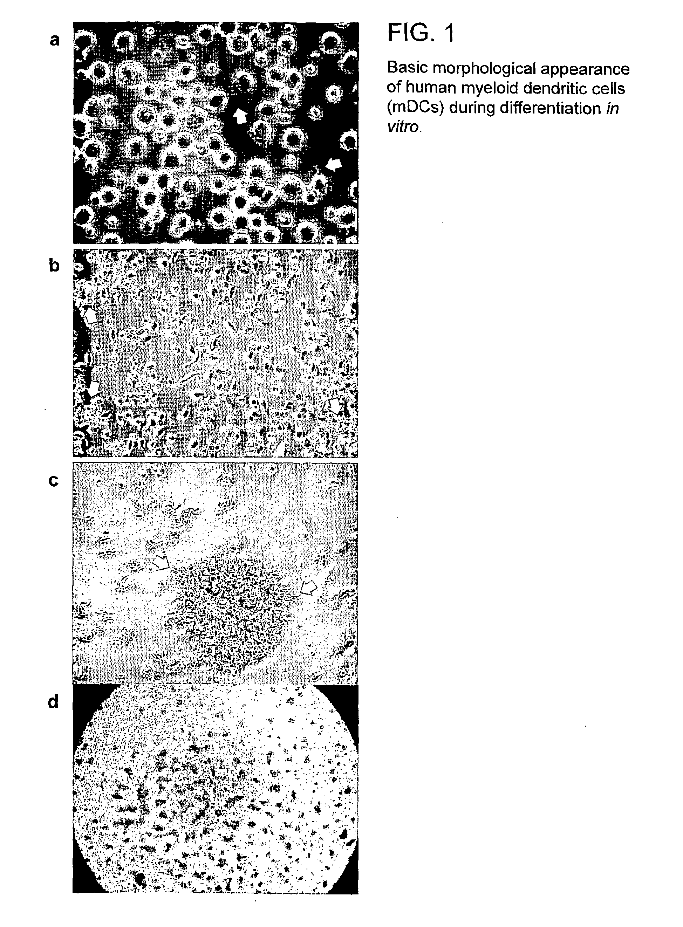

Figure 1 shows the basic morphological appearance of human myeloid dendritic cells (mDCs) during differentiation in vitro. Phase contrast photomicrographs were taken on days 3 (a), 5 (b), 7 (c, d). (a) On day 3, upon induction of differentiation by GM-CSF and IL-4, oval-shaped mDCs start to grow out membrane veils and thin membrane projections (arrows) (original magnification x400). (b) On day 5, most immature mDCs have assumed a stretched morphology and express membrane projections (i.e., dendrites), although oval-shaped cells are still present. Even at this immature stage, some mDCs associate to form small homotypic clusters (arrows) (original magnification x180). (c) However, upon induction of mDC maturation by TNF-a on day 5, fully matured DCs generally associate in the form of large homotypic clusters on day 7. Strong clustering by de novo-expressed adhesion molecules indicates that mDCs have reached their full functional maturity. Note the abundant filiform projections pointing out of the cluster (arrows). When located in a lymphoid organ, such dendrites establish intimate contact with T cells for antigen-specific stimulation in heterotypic mDC-T-cell clusters (original magnification x180).

Although this series of events represents the differentiation course of mDCs in most healthy donors, monocytes obtained from a minor portion of donors respond differently to the same microenvironmental conditions. Specifically, probably due to genetic pre-disposition, cells from some donors express fewer dendrites and/or form smaller, but more numerous, clusters. An example is depicted in (d) at x50 magnification of a complete microtiter well. Also, in very rare cases, mDC differentiation completely fails and macrophages develop instead; this may be due to overriding priming signals acting on the monocytes in the respective donor, for example, in case of an ongoing infection. Importantly, our targeting studies on human myeloid dendritic cells were carried out on mDCs following the regular differentiation path observed in approximately 80-90% of the cases in the presence of GM-CSF/IL-4 and sequential TNF-a.

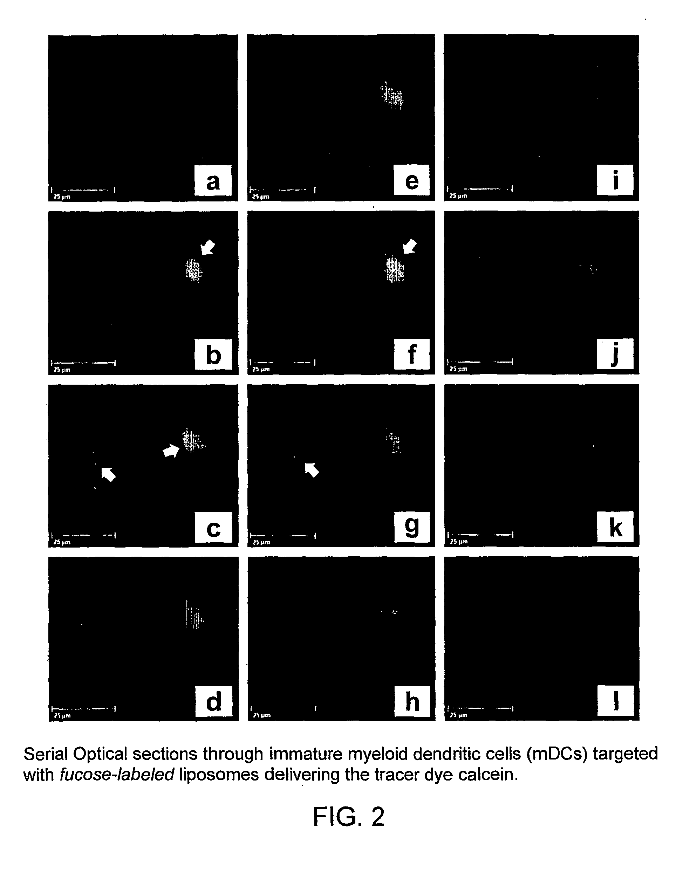

Figure 2 shows serial optical sections through immature myeloid dendritic cells (mDCs) targeted with fucose-labeled liposomes delivering the tracer dye calcein. Immature mDCs generated for 5 days with GM-CSF and IL-4 were detached from the substratum by EDTA and incubated for 3 h under continuous agitation at 37°C with Fuc-C4-Chol-targeted liposomes delivering the green fluorescent tracer dye calcein. Cells were counterstained with blue nuclear DAPI stain and fixated. (a-1) Fluorescence-microscopic overlays of serial sections (~1-mm steps) depict uptake of the system by two mDCs representing the lowest and highest uptake rates observed. In the mDC on the left, calcein was mainly confined to endosomes (e.g., c; arrow), with faint occasional cytoplasmic staining (e.g., overlaid to the nucleus in frame g; arrow). In contrast, the mDC on the right revealed bright staining of both endosomes (punctuate fluorescence, e.g., c, f) and cytoplasm (e.g., b). When comparing larger numbers of cells, all mDCs from all donors tested (n = 3) had internalized the fucose-targeted system. As apparent from the blue stain, liposome payload was never delivered into the nucleus. Man-C4-Chol-targeted positive controls were taken up less efficiently, and Gal-C4-Chol-targeted negative controls were not bound and/or internalized (not shown). Original magnification x400.

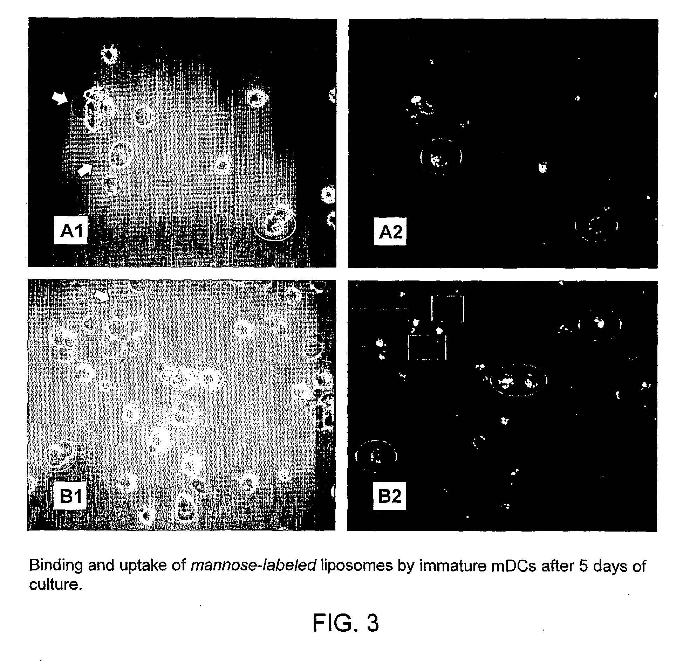

Figure 3 shows binding and uptake of mannose-labeled liposomes by immature mDCs after 5 days of culture. The extent of binding or uptake is depicted in two donors (A, upper row; B, lower row) by comparison of phase contrast (left column) and fluorescence (right column) photomicrographs. In both cases, and in contrast to fucose-labeled liposomes, only less than 50% of the cells revealed the tracer dye, calcein, within liposomes bound to their surface or internalized after 3-hour incubation. While some of tracer-positive mDCs showed intracellular uptake (B1, B2: upper and median circles), others still only revealed surface binding without uptake (A1, A2 and B1, B2: lower circles) after prolonged incubation. In immature mDCs, targeting by Man-C4-Chol-labeled liposomes thus not only reached far fewer mDCs than observed when employing the Fuc-C4-Chol-targeted delivery system (compare also flow cytometry), but the mannose-targeted system was also much less efficiently taken up by receptor-mediated endocytosis. However, mannose targeting was equally efficient in macrophages, probably due to these cells' higher expression of the mannose receptor (CD206) C-type lectin (not shown). Incubation with Gal-C4-Chol-labeled negative-control liposomes never led to surface binding or intracellular uptake (not shown). Arrows in phase contrast micrographs A1 and B1 point at cells that had died off during culture, as apparent by their blebbing surface membranes. Although an occasional dead cell revealed non-specific calcein staining (A1, A2: upper circles), non-specific binding of fucose-, mannose- or galactose-labeled liposomes to dead cells was generally not observed at this point in time (compare boxed cells exactly positioned with equidistant bars in B1 and B2).

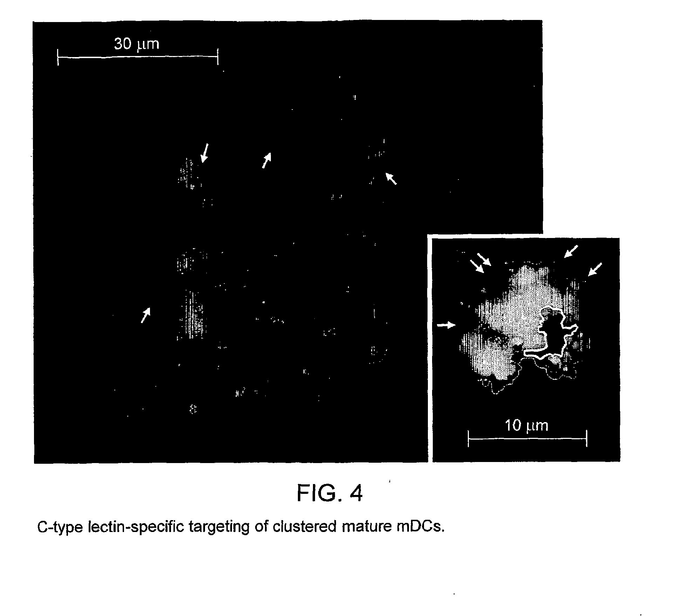

Figure 4 shows C-type lectin-specific targeting of clustered mature mDCs. Homotypic clusters of mature mDCs after 7-day culture in the presence of GM-CSF, IL-4, and TNF-a usually are overall round in shape and can comprise several hundreds of cells (cf. Fig. 1). Clusters partially disintegrate upon processing of cultured cells before incubation with the targeting system. However, due to the tight binding of mature mDCs via adhesion molecules (e.g., ICAM-1, ICAM-3, LFA-1), fragments of such clusters remain physically intact. The large fluorescence photomicrograph shows such a fragment comprising several tightly associated mDCs after 3-hour incubation with Fuc-C4-Chol-labeled liposomes. With a thin blue outline marking the contour of this fragment, each individual cell is enumerated on its lower right-hand side in a clockwise manner spiraling inwards. All 17 mDCs counted display at least faint cytoplasmic staining by liposome-delivered calcein. In most of the cases, stained endosomes stand out by their bright, sometimes outshining, punctuate fluorescence. The tracer dye never stained the cells' nuclei (when visible), as indicated by arrows. Mature mDCs generally revealed a lower uptake after targeting than seen in immature mDCs (see Fig. 2 and flow-cytometric results). The fucose-targeted system thus reached all mature mDCs despite their tight physical association. The same can thus be expected for homotypically clustered mDCs, as well as for mDCs within heterotypic mDC-T-cell clusters in lymphoid organs and tissues in vivo. Original magnification x400. As depicted in the small insert, single mDCs from 7-day cultures more often showed intense uptake of fucose-targeted liposomes and calcein delivery. The typical irregular-shaped nucleus of the mDC outlined in red is completely spared from calcein delivery. Targeted liposomes likely bound to surface (blue outline) C-type lectin receptors are indicated by arrows. Original magnification x1000.

Importantly, a consistent portion of the immature mDCs depicted in Fig. 2 as well as the mature mDCs shown in Fig. 4 expressed the Langerhans-cell marker CD1a (see flow-cytometry). Such cells correspond to mucosal and epidermal mDC subsets first infected upon sexual transmission of HIV. Conversely, another portion of mDCs did not express CD1a (see flow cytometry), thus corresponding to other systemic and lymphoid mDC subsets. Finally, mature mDCs (Fig. 4) expressed the immunoglobulin superfamily marker CD83 consistently expressed by mature DCs located in the lymphoid organs. All these types of mDCs were successfully targeted for intracellular endosomal and cytoplasmic delivery of an encapsulated compound. Thus, these results strongly indicate that all peripheral and lymphoid mDC subsets can be targeted efficiently with a Fuc-4C-Chol-labeled system for intracellular delivery of a therapeutic compound.

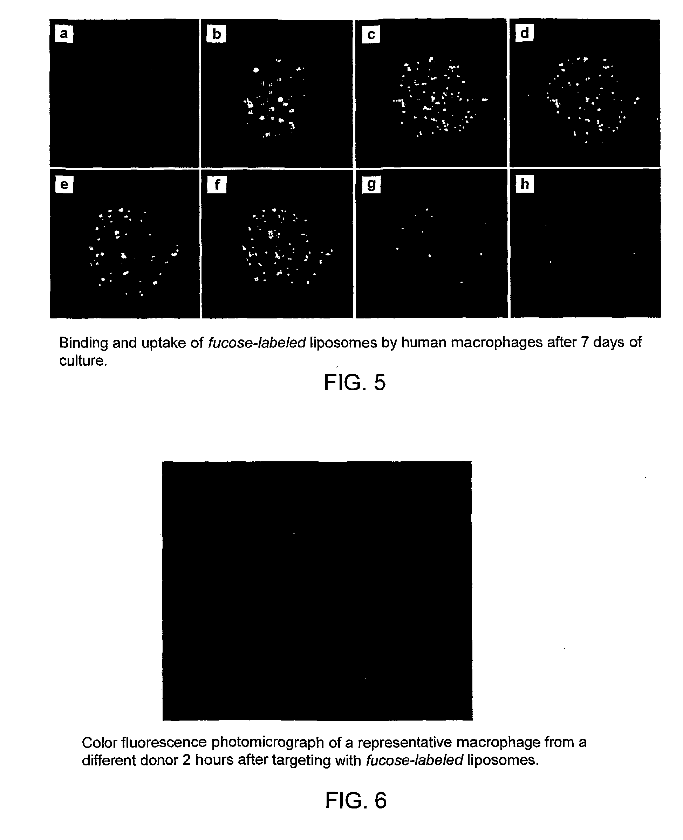

Figure 5 shows binding and uptake of fucose-labeled liposomes by human macrophages after 7 days of culture. Before incubation, macrophages were detached from the substratum by EDTA/trypsin treatment and then kept under continuous agitation to prevent their firm re-attachment, so as to enable their subsequent transfer to slides. (a-h) Serial optical sections through a representative macrophage revealed, already after 2 hours of incubation with Fuc-C4-Chol-targeted liposomes, abundant endosome-confined intracellular staining by the tracer dye, calcein, delivered by the targeting system. In contrast to myeloid dendritic cells, cytoplasmic staining (i.e., liposomal delivery) was much less apparent in macrophages. Man-C4-Chol-labeled liposomes had a comparable targeting efficiency (not shown). Incubation with the Gal-C4-Chol-labeled negative control only led to minor uptake by an occasional macrophage (not shown). In the case depicted in here, the cells were generated in the presence of 10% autologous donor serum. Original magnification x1000.

Figure 6 is a color fluorescence photomicrograph of a representative macrophage from a different donor 2 hours after targeting with fucose-labeled liposomes. In this case, macrophages were differentiated for 7 days in the presence of 10% xenogenic fetal bovine serum (FBS). Under such conditions, binding and uptake results were identical to those obtained with macrophages generated with autologous serum, including the results upon targeting with the positive (Man-C4-Chol) and negative (Gal-C4-Chol) control systems. FBS-dependent differentiation can thus be employed in vitro for macrophage targeting studies. Median section. Original magnification x1000.

Importantly, these results showed that the fucose-targeted liposomal delivery system was also efficiently internalized by macrophages representing a system of cells that, as in HIV disease, forms the major infectious cellular reservoir of the gastrointestinal tract and, perhaps, of the brain.

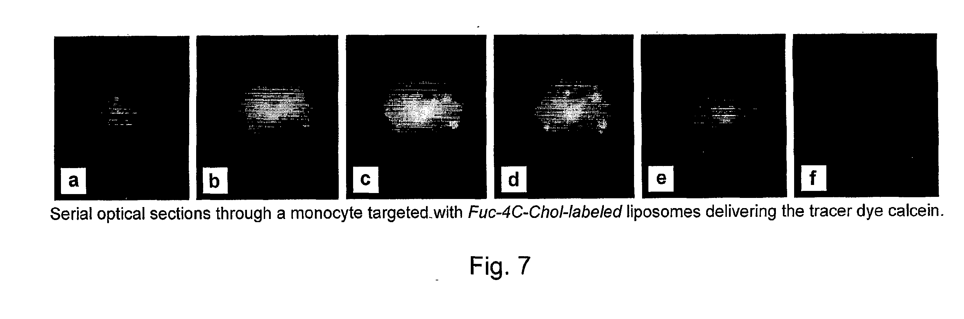

Figure 7 shows serial optical sections through a monocyte targeted with Fuc-4C-Chol-labeled liposomes delivering the tracer dye calcein. Freshly isolated peripheral-blood monocytes were incubated for 3 h under continuous agitation at 37°C with Fuc-C4-Chol-targeted liposomes delivering the green fluorescent tracer dye calcein. Cells were counterstained with the blue nuclear DAPI stain and fixated. (a-f) Fluorescence-microscopic green/blue overlays of serial sections (~1.5-mm steps) depict uptake of the system by a representative monocyte (note the typical nuclear shape). In monocytes, the intracellular distribution of calcein as the targeted system's payload was identical to that seen in mDCs. The fluorescent compound was concentrated in the cells' endosomes (as most apparent in frames c, d, and e; punctuate fluorescence), as well as, more diffusely, in the monocytes' cytoplasm (i.e., all of the serial micrographs), but never within their nuclei. Moreover, as found for mDCs, too, all monocytes from all donors tested (n = 3) had internalized the fucose-targeted system. Again, Man-C4-Chol-targeted positive controls were taken up less efficiently, and Gal-C4-Chol-targeted negative controls were not bound and/or internalized at all (not shown). Original magnification x400.

Importantly, these results show that, besides reaching myeloid dendritic cells and macrophages, fucose-mediated targeted delivery of a therapeutic compound can be achieved for monocytes, too. This conclusion may have a profound impact when considering that monocytes, as has been explained above, potentially are the earliest myeloid lineage-derived cell type to be recruited as an infectious reservoir for HIV and other infectious agents. In fact, the case that monocytes were so efficiently targeted by a C-type lectin-specific system highlights the importance of this pathway for the uptake of infectious agents and the subsequent formation of chronically infectious intracellular reservoirs in a plethora of physiological and pathological monocytic descendants.

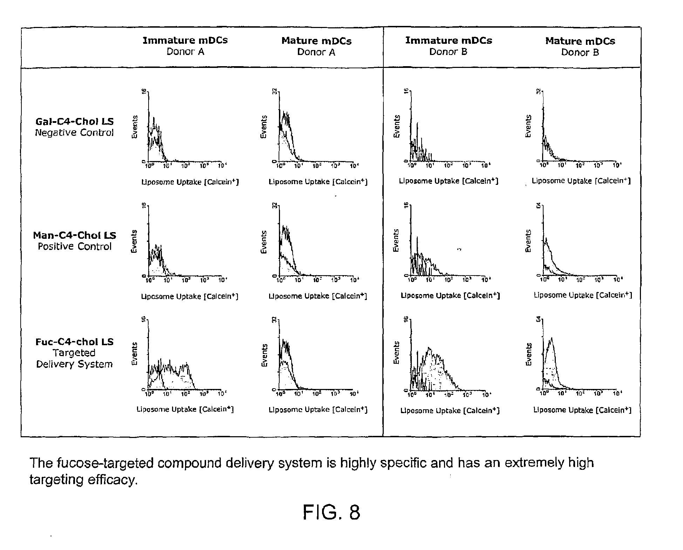

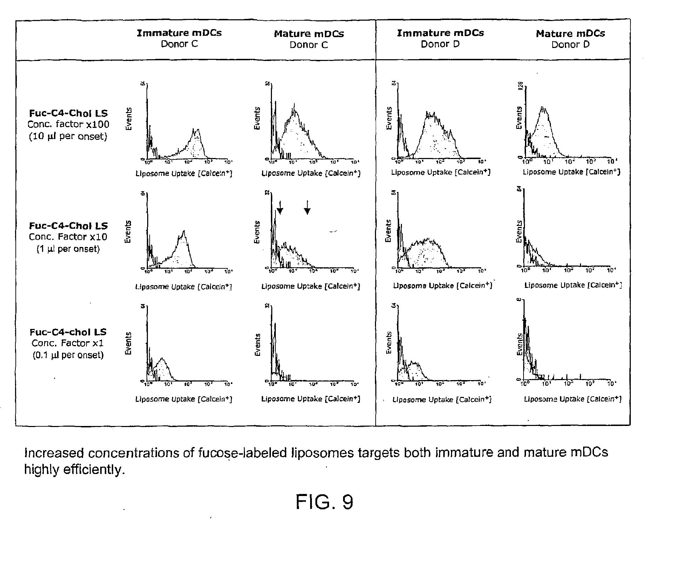

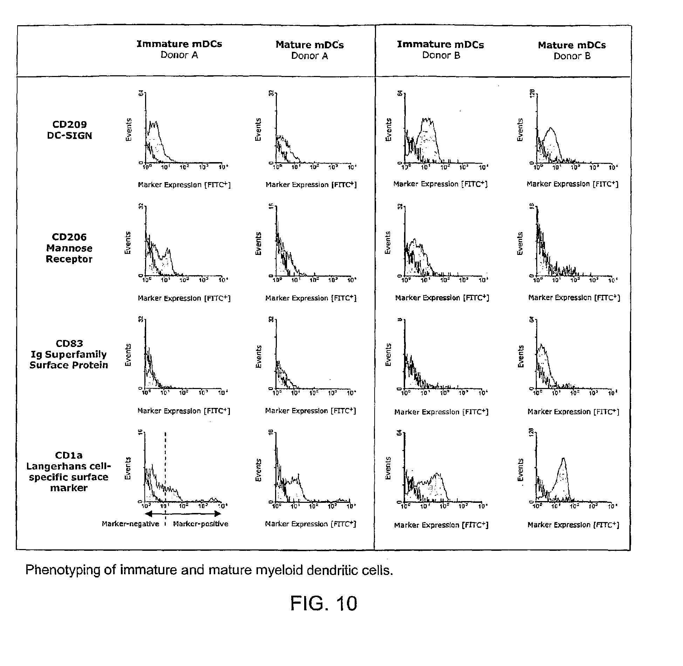

Figure 8 shows that the fucose-targeted compound delivery system is highly specific and has an extremely high targeting efficacy. When employing both immature and mature myeloid dendritic cells as important reservoir populations for HIV and other infectious agents, fucose targeting was most efficient in immature mDCs. Binding or uptake of calcein-delivering carbohydrate-labeled liposomes is depicted as filled histograms overlaid with empty histograms of background staining with non-sugar-labeled liposomes that bind to cells nonspecifically. The binding efficacy of Gal-C4-Chol-labeled negative control liposomes never differed from nonspecific control liposomes, thus verifying the correct choice of galactose labeling as a negative control. In contrast, mannose and fucose-labeled liposomes showed different degrees of specific cellular surface targeting and/or uptake. When compared with the Man-C4-Chol positive control, the Fuc-C4-Chol-targeted system revealed far superior binding efficacy in immature mDCs. In both donors, on a logarithmic scale (abscissa), the targeting efficacy of fucose-labeled liposomes exceeded that of the positive control by one order of magnitude. Specific targeting of mature mDCs was donor-dependent, in that some individuals, such as donor A, produce mature mDCs that express only low levels of C-type lectins. Yet, most donors, e.g. donor B, reveal at least median membrane densities of such molecules (see also Fig. 10), so that their net sum expression allows for efficient targeting with a Fuc-4-Chol-labeled liposomal delivery system. Nevertheless, even low binding to mature mDCs in such individuals can be significantly increased by higher concentrations of this system (see Fig. 9).

Figure 9 shows that increased concentrations of fucose-labeled liposomes targets both immature and mature mDCs highly efficiently. Employing the same positive and negative controls (see legend to Fig. 8), immature and mature mDCs were incubated with different concentrations of the Fuc-C4-Chol-targeted system. This experiment was carried out with two donors (C and D) in which a low concentration of the targeting system (lower row) efficiently reached immature, but not mature mDCs. However, when increasing the system's concentration by factors of x10 or x100, respectively (medium and upper rows), immature DCs were targeted highly efficiently. The medium concentration was applied in the experiments depicted in Fig. 2 and Fig. 4 Arrows in the medium row (donor C) indicate approximated positions of the two cells shown in Fig 2 that represent the cellular spectrum of binding-and-uptake efficacy of the Fuc-4C-Chol targeting system under this condition. Taken together, both cells expressing high and low surface membrane densities of C-type lectin receptors can be addressed successfully with our targeted compound delivery system.

Figure 10 depicts phenotyping of immature and mature myeloid dendritic cells. Marker-positive cells are depicted as filled histograms and overlaid with empty histograms indicating background staining with negative irrelevant control antibody. Gray areas left of the negative-control cutoff reflect the portion of cells not expressing a given marker; gray areas right of the cutoff express the marker (as exemplarily shown in the graph showing CD1a expression in immature mDCs from donor A). Abscissas indicate logarithmic fluorescence intensities of cell labeling with FITC-conjugated secondary antibodies after adding primary monoclonal antibodies recognizing the respective marker. DC-SIGN and the mannose receptor as typical representatives of C-type lectins expressed by mDCs are both expressed more pronounced in immature than in mature mDCs. Individual variances are apparent. In vivo, immature DCs reside in the peripheral nonlymphoid organs and tissues. Here, strong expression of such surface molecules ensures the cells' capability to bind and ingest many pathogens. Once migrated to the lymphoid organs and tissues, matured mDCs downregulate C-type lectin expression, but usually retain medium membrane densities of these targeting markers. Notably, as far as currently known, mDCs generally express at least four different surface C-type lectins (DC-SIGN, DEC-205, MR and DLEC), so that the net sum expression of such molecules always allows for efficient targeting with a fucose-labeled liposomal delivery system. Similarly, macrophages can be targeted in all their developmental stages, as they reveal consistently high expression of the mannose receptor (not shown). These cells can also be induced to express other C-type lectins such as DC-SIGN. Expression of CD83 indicates the mature status of mDCs. In vivo, expression of CD1a is indicative of Langerhans-cell mDC subsets (thus also expressing Langerin as a fifth C-type lectin) located in the mucosae and epidermis. Note that both immature and mature mDCs, at all times, comprised a spectrum of CD1a-negative to strongly CD1a-positive cells, thus covering a corresponding spectrum of non-Langerhans to Langerhans cell-like mDCs. Fuc-C4-Chol-targeted liposomes successfully delivered calcein intracellularly to all these subtypes (see Figs. 2, 4), thus indicating their high potential as a system for delivering therapeutic compound(s) to endosomal and intracytoplasmatic sites.

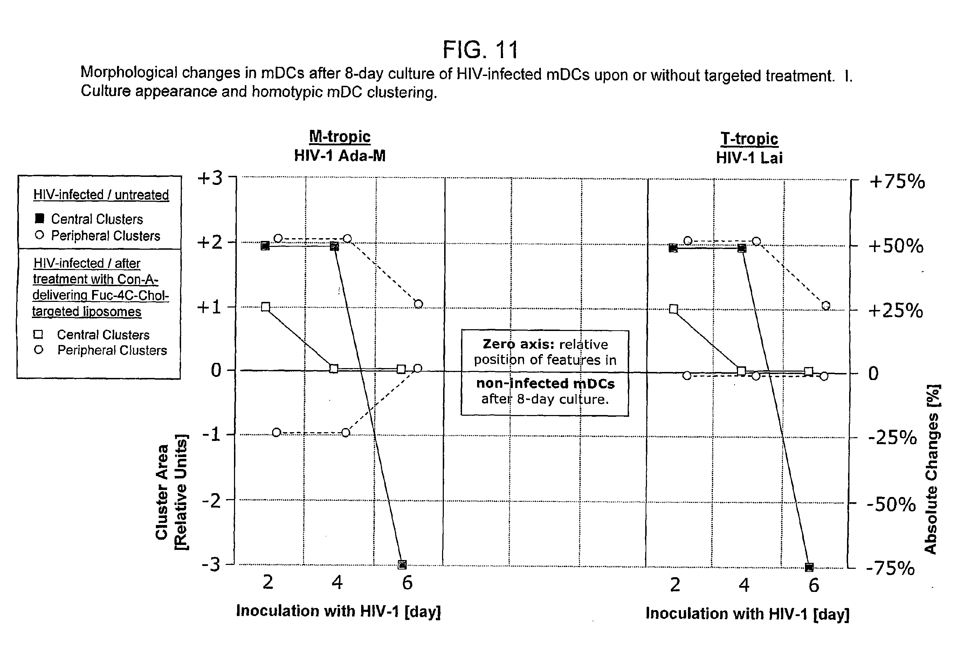

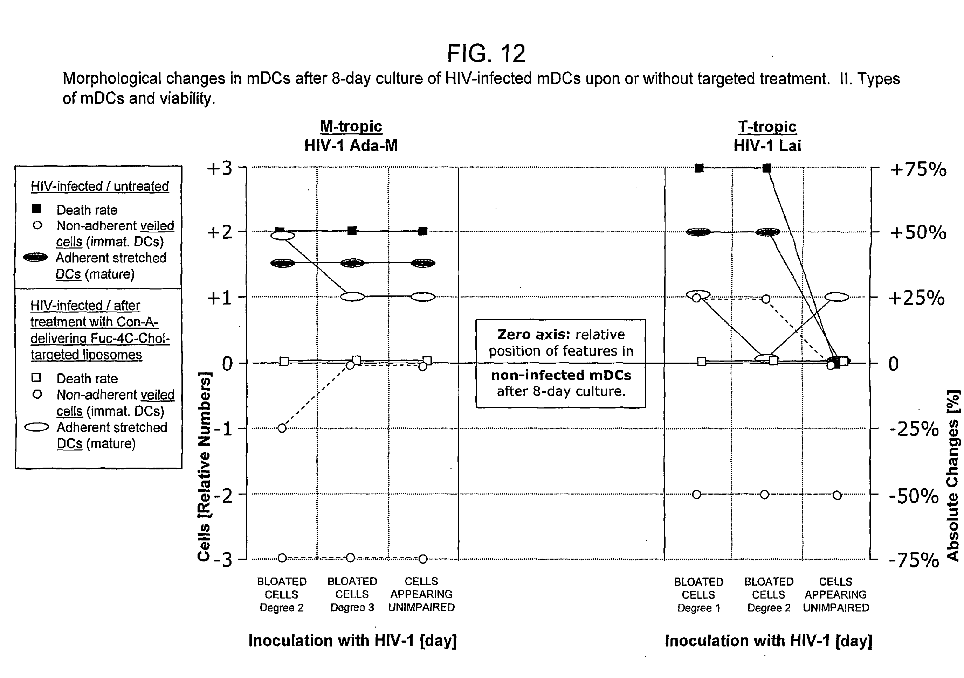

Figure 11 shows the morphological changes in mDCs after 8-day culture of HIV-infected mDCs upon or without targeted treatment. I. Culture appearance and homotypic mDC clustering. Cells were differentiated in the presence of GM-CF/IL-4 (day 0) and sequential TNF-a (day 5). On days 2, 4, or 6, the mDCs were infected with the M-tropic HIV-1 strain, Ada-M, or the T-tropic HIV-strain, Lai, respectively. Tissue culture infective doses for 50% of the cells were I. HIV-1 Ada-M: 67 x TCID50 (i.e., 1 ml virus stock solution + 199 ml culture medium); and II. HIV-1 LAI: 6.7 x TCID50 (i.e., 0.1 ml virus stock solution + 199.9 ml culture medium. Results were obtained by scanning all areas of four separate culture wells for each situation. Homotypic mDC clustering as a criterion indicating the functional integrity of these cells was evaluated on day 8; results are given as semi-quantitative and absolute (rounded) values. One day after infection with the respective strain, mDCs were treated with concanavalin-A (Con-A)-delivering Fuc-4C-Chol-targeted liposomes. This time delay allowed the cells to form intracellular HIV reservoirs. As apparent, in both types of HIV-1 infection, and under all conditions tested, the clustering behavior was normalized. As homotypic and heterotypic mDC clustering is upregulated by the HIV upon infection (Sol-Foulon N, Moris A, Nobile C, Boccaccio C, Engering A, Abastado JP, Heard JM, van Kooyk Y, Schwartz O. HIV-1 Nef-induced upregulation of DC-SIGN in dendritic cells promotes lymphocyte clustering and viral spread. Immunity 2002;16:145-55), these results indirectly demonstrate the successful elimination of HIV (see also Fig. 12).

Figure 12 shows the morphological changes in mDCs after 8-day culture of HIV-infected mDCs upon or without targeted treatment. II. Types of mDCs and viability. All conditions for generating mDCs, infection with HIV-1, targeted treatment are as given in the legend to Fig. 11. Results were obtained by scanning all areas of four separate culture wells for each situation. The increased death rate of mDCs upon infection with HIV-1 was normalized upon treatment with Con-A-delivering fucose-targeted liposomes. Note that the washing procedure after liposomal treatment for 3 hours removed the dead cells accumulated after infection of the mDCs. Cultures, thus, sometimes comprise significantly reduced cell numbers when compared to uninfected cultures at the same given point in time, which, via lower concentrations of autocrine self-conditioning signals, may take effect on the relative ratio of mDC morphologies. Nevertheless, the relative shift between veiled-cell and dendritiform mDC types upon HIV infection was largely normalized after treatment. These results again indirectly demonstrate the successful elimination of HIV.

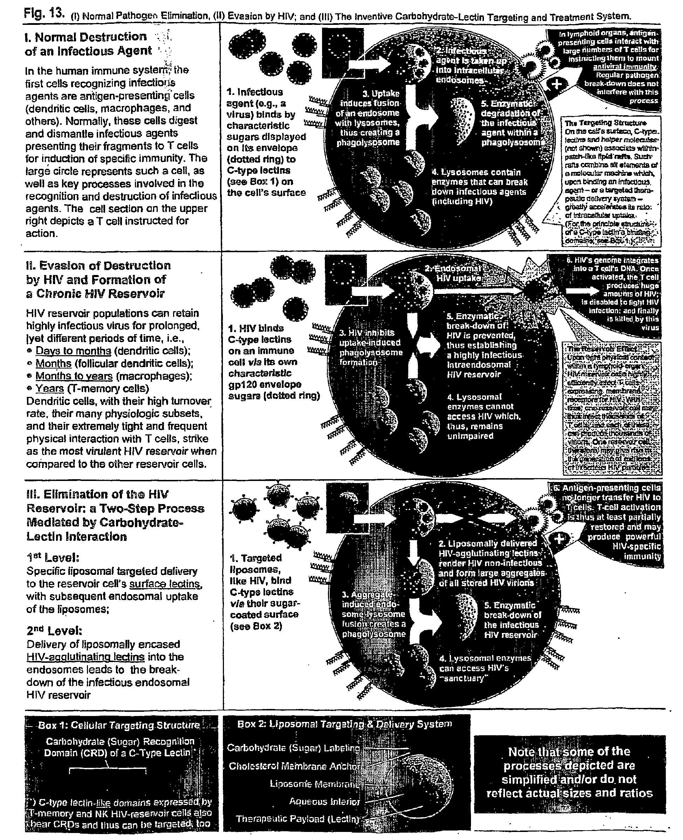

Figure. 13 is titled (I) Normal Pathogen Elimination, (II) Evasion by HIV; and (III) The Inventive Carbohydrate-Lectin Targeting and Treatment System.

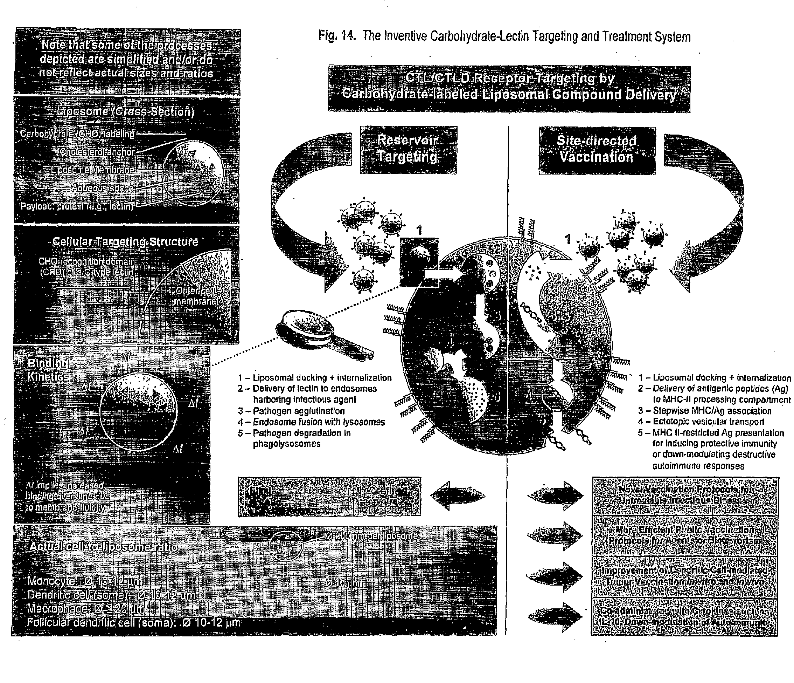

Figure. 14 is titled The Inventive Carbohydrate-Lectin Targeting and Treatment System.

### DETAILED DESCRIPTION OF THE INVENTION

It has become increasingly apparent that some pathogens/infectious agents, including HIV-1, HCV, and *Mycobacterium tuberculosis,* can establish infectious endosomal/intracellular reservoirs by exploiting the cell's evolutionarily ancient C-type lectin-dependent pathogen clearance (Gieseler RK, Marquitan G, Hahn MJ, Perdon LA, Driessen WH, Sullivan SM, Scolaro MJ. DG-SIGN-specific liposomal targeting and selective intracellular compound delivery to human myeloid dendritic cells: implications for HIV disease. Scand J Immunol 2004;59:415-24; and references therein). Moreover, when taking residence in an antigen-presenting cell, these infectious agents also subvert the antigen-presenting cell's T cell-instructive functions and thus escape immunosurveillance. Specifically, misuse of DC-SIGN expressed by a mDC can circumvent the APC's processing of the pathogen's antigens and/or alter its Toll-like receptor-mediated signaling. Potentially protective T-cell responses are thus either abrogated or misdirected. As discussed herein, other CTL receptors such as the mannose receptor, Langerin, and DEC-205 are exploited as well, which likewise results in the skewing of their regular signaling pathways (reviewed in van Kooyk Y, Geijtenbeek TB. DC-SIGN: escape mechanism for pathogens. Nat Rev Immunol 2003;3:697-709). Therefore, the inventions disclosed herein employ a pan-CTL-targeting system to therapeutically enter intracellular compartments where HIV (or other pathogens/infectious agents) are hidden.

CTL receptors recognize carbohydrates that are predominantly expressed on the surface of pathogens. These characteristic sugars bridge species borders and thus allow CTL receptors to interfere with a broad-spectrum of infectious agents/pathogens (reviewed in Taylor ME, Drickamer K. Structural requirements for high affinity binding of complex ligands by the macrophage mannose receptor. J Biol Chem 1993;268:399-404; and reviewed in Botos I, Wlodawer A. Proteins that bind high-mannose sugars of the HIV envelope. Prog Biophys Mol Biol: in press)*.* The inventions disclosed herein take advantage of the C-type lectin receptor's sugar selectivity in the inventive targeting system. CTL receptors preferentially and selectively bind to the largely pathogen-restricted monosaccharides mannose, fucose and *N-*acetylglucosamine, as well as multivalent oligosaccharides of similar nature (Geyer H, Holschbach C, Hunsmann G, Schneider J. Carbohydrates of human immunodeficiency virus. Structures of oligosaccharides linked to the envelope glycoprotein 120. J Biol Chem 1988;263:11760-7; reviewed in Taylor ME, Drickamer K. Structural requirements for high affinity binding of complex ligands by the macrophage mannose receptor. J Biol Chem 1993;268:399-404; and reviewed in Botos I, Wlodawer A. Proteins that bind high-mannose sugars of the HIV envelope. Prog Biophys Mol Biol: in press). With respect to the instant invention, a "pathogen-like" carbohydrate-dependent targeting mechanism has been designed to target reservoir and other cells and deliver active agents/compounds intracelluarly to such cells. Reservoir cells include cells that provide a reservoir for infectious agents, such as viruses and bacteria, and/or latent pathogens.

Cambi and Figdor have pointed out that CTL receptors in the immune system have a dual function, principally, allowing for pathogen recognition and cell adhesion. This implies that (1) pathogen-binding to receptors such as DC-SIGN, mannose receptor and DEC-205 results in endocytosis and fusion of late endosomes/lysosomes, while (2) certain types of receptors, e.g., DC-SIGN, mannose receptor, DCAL1 and selectins mediate contact between, for example, mDCs and T cells as a prerequisite for successive T-cell activation and co-stimulation), as well as leukocytes and endothelium (whereby mediating leukocyte homing). It thus becomes apparent that CTL receptors such as DC-SIGN and mannose receptor mediate both endocytosis and contact/adhesion (Cambi A, Figdor CG. Dual function of C-type lectin-like receptors in the immune system. Curr Opin Cell Biol 2003;15:539-46). Of note, such receptors are expressed by most HIV reservoir cells. The dual physiological function of some CTL molecules may be the decisive factor for their likewise dual pathological role in (i) forming HIV reservoirs (by pathogen binding and uptake), and subsequently (ii) transferring the virus to bystander cells (by contact/adhesion). Most HIV reservoir cells display sugar-binding CTL receptors on their surface. The inventions disclosed herein materialize in a therapeutic strategy for such diseases that build upon, and take advantage of, nature's primordial and, thus, fundamental CRD-lectin concept. Moreover, due to the fact that a cell can internalize a ligand once engaged by a CRD receptor, this strategy may likewise address even *non-infectious* chronic diseases.

However, as to T-memory and natural-killer-cell reservoirs (Weis WI, Taylor ME, Drickamer K. The C-type lectin superfamily in the immune system. Immunol Rev 1998;163:19-34), receptors are expressed that display structurally similar C-type lectin-like domains (CTLDs). Therefore, although CTL and CTLD receptors are evolutionarily unrelated (Khalturin K, Becker M, Rinkevich B, Bosch TC. Urochordates and the origin of natural killer cells: identification of a CD94/NKR-P1-related receptor in blood cells of Botryllus. Proc Natl Acad Sci USA 2003;100:622-7. Epub 2003 Jan 07; and Zelensky AN, Gready JE. C-type lectin-like domains in Fugu rubripes. BMC Genomics 2004;5:51), they both can be engaged not only by HIV, but also by a system targeting their structural similarities. The inventive targeting system is designed to address the entire composition of HIV reservoir cells.

The inventive targeting system may not only reach reservoir cells, but also the T cells infected *via* reservoir cells within lymphoid organs and tissues. The inventive liposomal targeting system revealed approximately two hours of membrane redundancy before being taken up by the targeted cells. Specifically, (i) this delayed uptake; (ii) the association of CTL receptors and transiently bound liposomes in the immunological synapses formed between reservoir and T cells; (iii) the crucial role of these receptors for HIV transfer to T cells (Arrighi JF, Pion M, Garcia E, Escola JM, van Kooyk Y, Geijtenbeek TB, Piguet V. DC-SIGN-mediated infectious synapse formation enhances X4 HIV-1 transmission from dendritic cells to T cells. J Exp Med 2004;200:1279-88); and (iv) the liposomes' HIV-like size (Gieseler RK, Marquitan G, Hahn MJ, Perdon LA, Driessen WH, Sullivan SM, Scolaro MJ. DC-SIGN-specific liposomal targeting and selective intracellular compound delivery to human myeloid dendritic cells: implications for HIV disease. Scand J Immunol 2004;59:415-24) may let the inventive targeted delivery system utilize the pathway for T-cell entry exploited by the virus itself. Thereby, the inventive targeting system may also be transferred to T cells as well. To certain degrees, lectins may be delivered to T cells to agglutinate newly infecting HIV, and/or to interfere with the actively replicating virus (Gieseler RK, Marquitan G, Hahn MJ, Perdon LA, Driessen WH, Sullivan SM, Scolaro MJ. DC-SIGN-specific liposomal targeting and selective intracellular compound delivery to human myeloid dendritic cells: implications for HIV disease. Scand J Immunol 2004;59:415-24).

Membrane cholesterol is a key factor in the cellular uptake of HIV (Liao Z, Cimakasky LM, Hampton R, Nguyen DH, Hildreth JE. Lipid rafts and HIV pathogenesis: host membrane cholesterol is required for infection by HIV type 1. AIDS Res Hum Retroviruses 2001;17:1009-19). In one embodiment of the invention, by featuring fucosyl-cholesterol derivatives, the inventive liposomal targeting system thus provides an HIV-tropic component that may (similar to fusion inhibitors) allow for a pre-fusion association of virions and liposomes within the infectious synapse's lipid raft. In addition, when shuttled into a HIV-replicating T cell, the targeted lectin delivery system encounters an environment in which major steps of gp120 glycosylation and viral assembly occur within endosomal compartments (Greene WC, Peterlin BM. Charting HIV's remarkable voyage through the cell: Basic science as a passport to future therapy. Nat Med 2002;8:673-80); due to the demonstrated endosomal tropism of the inventive targeted liposomes, it is thus expected that lectin interference with the highly conserved gp120 glycosyl residues (see below) will also take effect in T lymphocytes. Third, the HIV Gag polyprotein, which is essential for viral budding, preferentially associates with cholesterol-rich membrane microdomains (Ono A, Freed EO. Plasma membrane rafts play a critical role in HIV-1 assembly and release. Proc Natl Acad Sci USA 2001;98:13925-30). Gag's physicochemical preference may thus lead budding virions directly into a "lectin trap" via the cholesterol component of the inventive liposomal targeting system.

The inventive delivery system, thus, potentially to interacts with all key processes of active viral propagation, i.e., uptake, assembly, and budding. The inventive systems and products, besides interfering with cellular HIV reservoirs, may also disrupt the active propagation of HIV in T cells.

In one embodiment of the invention, specific cell populations are targeted for the delivery of therapeutically active agents/compounds by liposomal systems targeted to CRD lectins on the surface of the cells of the specific cell populations. Liposomal systems (*i.e.* lipid-active agent complexes) which encapsulate an active agent(s), such as a plant lectin(s), and have a targeting ligand(s) that are carbohydrate-derivatized, such as Fuc-4C-Chol, on the outer surface of the liposomal systems, are administered to the cells. Another embodiment of the invention are the targeted liposomal systems (*i.e*. the lipid-active agent complexes which contain the active agent and have a carbohydrate-derivatized targeting ligand on the surface of such complexes). In a preferred embodiment of the invention, the targeting ligand is Fuc-4C-Chol.

In 1981, Robbins *et al.* first identified in mammalian macrophages a transport system that binds and internalizes glycoproteins with exposed mannose residues. They predicted that "synthetic substrates may be useful in targeting pharmacologic agents to macrophages, and analogous compounds may target such agents to other types of cell" (Robbins JC, Lam MH, Tripp CS, Bugianesi RL, Ponpipom MM, Shen TY. Synthetic glycopeptide substrates for receptor-mediated endocytosis by macrophages. Proc Natl Acad Sci USA 1981;78:7294-8).

Early experiments employing liposomes with a mannosylated surface revealed a specific interaction with the macrophage mannose-fucose receptor (now termed mannose receptor, CD206, or MRC1; see below), which led to the suggestion that such a vehicle would be useful for the delivery of immunomodulators to reticuloendothelial cells (Barratt G, Tenu JP, Yapo A, Petit JF. Preparation and characterisation of liposomes containing mannosylated phospholipids capable of targetting drugs to macrophages. Biochim Biophys Acta 1986;862:153-64). When encapsulating an immunomodulator in such liposomes, targeted alveolar macrophages induced a cytotoxic anti-tumor response both in vivo and in vitro (Barratt GM, Nolibe D, Yapo A, Petit JF, Tenu JP. Use of mannosylated liposomes for in vivo targeting of a macrophage activator and control of artificial pulmonary metastases. Ann Inst Pasteur Immunol 1987;138:437-50). These results demonstrated the potential usefulness of a carbohydrate-targeted liposomal delivery system.

More recently, Copland et al. targeted the mannose receptor C-type lectin (CD206; MRC1) of immature monocyte-derived dendritic cells with mannosylated liposomes. As monitored by encapsulated FITC-ovalbumin, such liposomes were preferentially bound and taken up by these cells at 37°C when compared to non-mannosylated neutral or negatively charged liposomes. Moreover, mannosylated liposomes encapsulating tetanus toxoid (TT) elicited a dendritic cell-dependent TT-specific T-cell stimulation/proliferation more effectively than neutral TT-containing liposomes or non-encapsulated free TT, leading to the conclusion that mannosylated liposomes are a versatile delivery vehicle for enhancing immune responses to encapsulated peptide or protein vaccines (Copland MJ et al. Liposomal delivery of antigen to human dendritic cells. Vaccine 2003;21:883-90).

Both studies on myeloid dendritic cells summarized above (Copland MJ et al. Liposomal delivery of antigen to human dendritic cells. Vaccine 2003;21:883-90; and Gieseler RK, Marquitan G, Hahn MJ, Perdon LA, Driessen WH, Sullivan SM, Scolaro MJ. DC-SIGN-specific liposomal targeting and selective intracellular compound delivery to human myeloid dendritic cells: implications for HIV disease. Scand J Immunol 2004;59:415-24) thus independently demonstrated that cells expressing C-type lectins (which are specifically referred to herein) can be highly efficiently targeted for intracellular uptake, site-specific delivery, and functional modulation by both antibody- or carbohydrate-labeled liposomes.

As to the *in-vivo* biodistribution upon systemic administration of C-type lectin receptor-specific liposomes, Kawakami and colleagues investigated mannosylated, fucosylated, and galactosylated liposomes. After intravenous injection in mice, all three types of glycosylated liposomes were rapidly eliminated from the circulating blood and preferentially recovered in the liver, and it was concluded that they were taken up by hepatic parenchymal and non-parenchymal cells, at different rates, *via* asialoglycoprotein receptors (Kawakami S, Wong J, Sato A, Hattori Y, Yamashita F, Hashida M. Biodistribution characteristics of mannosylated, fucosylated, and galactosylated liposomes in mice. Biochim Biophys Acta 2000;1524:258-65).

However, in contrast to a systemic application mode, embodiments of the invention employ local administration of CTL receptor-specific liposomes, such as by sub- or intracutaneous injection, so as to be drained to the local lymph nodes for targeting regional infectious pathogen reservoirs. Even when aiming at cellular targets in hepatogastroenterological contexts of chronic non-infectious diseases, embodiments of the invention increase selectivity of treatment by taking advantage of established organ-directed methods, e.g., direct infusion via the hepatic artery (Kemeny MM, Alava G, Oliver JM. The effects on liver metastases of circadian patterned continuous hepatic artery infusion of FUDR. HPB Surg 1994;7:219-24). Therefore, when employing a glycosylated liposomal targeting system, the result expected is (i) that its local administration prevents intrahepatic loss of the system; and (ii) that its organ-directed administration would minimize the loss of the system in secondary systemic sites of absorption. Besides focusing the effect on the desired region(s) or organ(s), both approaches have apparent pharmacotoxicological advantages.

In preferred embodiments of the invention, fucose-labeled liposomes are employed for specific delivery of liposome-encapsulated therapeutically active compounds to cell populations expressing certain CRD lectins. Specific applications envisioned include therapeutic targeting of
(i) CRD⁺ (i.e., CTL or CTLD receptor⁺) chronically infected reservoir cell populations in infectious diseases, such as in infections with human immunodeficiency virus type 1 (HIV), hepatitis virus C (HCV), and *Mycobacterium tuberculosis;*
(ii) CRD⁺ (i.e., REG family member⁺) neoplastic cells such as in cancers of the colon and rectum; and
(iii) CRD⁺ parenchymal and/or non-parenchymal cells of the liver (asialoglycoprotein receptor⁺ hepatocytes; mannose/fucose receptor⁺ non-parenchymal liver cells; and L-SIGN⁺ liver sinus endothelial cells), e.g., for addressing hyperapoptotic hepatocytes in non-alcoholic steatohepatitis.

Still, besides C-type lectins, receptors implied in establishing infectious intracellular reservoirs also include non-CTL lectins, such as hNKR-P1A and KLRG1. However, as these molecules display C-type lectin-*like* domains (CTLDs), both CTL and CTLD lectins commonly feature pathogen-binding carbohydrate recognition domains (reviewed in Cambi A, Figdor CG. Dual function of C-type lectin-like receptors in the immune system. Curr Opin Cell Biol 2003;15:539-46). Due to this common denominator, the inventive carbohydrate-dependent targeting mechanism also binds CTLD structures on non-CTL receptors. As a result, the inventive pan-CTL/CTLD-targeting system has the ability to therapeutically address all types of HIV reservoir cells.

The inventive methods and products (*i.e.* targeted lipid-active agent compleses, including targeted liposomes), in essence, bind to, and enter, cells by the CTL and CTLD receptor-mediated pathways exploited by HIV. Moreover, the inventive products are preferentially similar in size (⌀ approx. 150 nm) to the virus itself (⌀ approx. 120 nm). Nevertheless, the fact that DC-SIGN internalizes (infectious) particles over a broad range of sizes (Engering A, Geijtenbeek TBH, van Vliet SJ et al. The dendritic cell-specific adhesion receptor DC-SIGN internalizes antigen for presentation to T cells. J Immunol 2002;168:2118-26; Kwon DS, Gregorio G, Bitton N, Hendrickson WA, Littman DR. DC-SIGN-mediated internalization of HIV is required for trans-enhancement of T cell infection. Immunity 2002;16:135-44; Cambi A, Gijzen K, de Vries JM et al. The C-type lectin DC-SIGN (CD209) is an antigen-uptake receptor for Candida albicans on dendritic cells. Eur J Immunol 2003;33:532-8; and Colmenares M, Puig-Kroger A, Pello OM, Corbi AL, Rivas L. Dendritic cell (DC)-specific intercellular adhesion molecule 3 (ICAM-3)-grabbing nonintegrin (DC-SIGN, CD209), a C-type surface lectin in human DCs, is a receptor for Leishmania amastigotes. J Biol Chem 2002;277:36766-9), enables the inventive targeted delivery systems and products to broadly interfere with various infectious agents establishing chronic intracellular reservoirs.

A decisive factor for successful cellular targeting is the membrane density of the target structures. At least in one stage of their life cycles, all pathogen reservoir cells to be targeted express CTL and/or CTLD receptors at medium to high membrane densities. On the representative example of DC-SIGN/CD209, Baribaud *et al.* have measured a surface expression of at least 1x10⁵ molecules per immature mDC (Baribaud F, Pöhlmann S, Leslie G, Mortari F, Doms RW. Quantitative expression and virus transmission analysis of DC-SIGN on monocyte-derived dendritic cells. J Virol 2002;76:9135-42). In addition, these targeting molecules tightly arrange in the cell's membrane within lipid rafts and/or within the (infectious) synapses established with T cells (Arrighi JF, Pion M, Garcia E, Escola JM, van Kooyk Y, Geijtenbeek TB, Piguet V. DC-SIGN-mediated infectious synapse formation enhances X4 HIV-1 transmission from dendritic cells to T cells. J Exp Med 2004;200:1279-88; and Gieseler RK, Marquitan G, Hahn MJ, Perdon LA, Driessen WH, Sullivan SM, Scolaro MJ. DC-SIGN-specific liposomal targeting and selective intracellular compound delivery to human myeloid dendritic cells: implications for HIV disease. Scand J Immunol 2004;59:415-24).

Taken together, such conditions provide a comfortable molecular target, as confirmed herein, by the very high targeting efficacies. It is thus reasonable to assume that the novel unifying technology for targeting intracellular infectious-disease reservoirs presented herein will enable the inventive technology to commonly reach all known HIV reservoir cell types for potent therapeutic intervention. Both CTL and CTLD receptors may be harnessed for shuttling the inventive liposomes labeled with certain mono- or polysaccharides (e.g., fucose or polymerized fucose) into intracellular compartments exploited by other infectious agents, including various species of viruses, bacteria, fungi and parasites (Cambi A, Figdor CG. Dual function of C-type lectin-like receptors in the immune system. Curr Opin Cell Biol 2003;15:539-46). The inventive methods and products allow for interference with, and eradication of, HIV-1, HCV, *Mycobacterium tuberculosis,* and beyond. To this end, a suitable therapeutic compound must be delivered. In an embodiment of the invention, a compound with the ability to agglutinate structurally intact infectious agents *via* their characteristic surface sugars is employed with the methods and products of the invention.

### Pathogen-restricted Structures for Therapeutic Targeting of Intracellular Reservoirs

Due to frequent mutations in retroviruses such as HIV and HCV (e.g., Major ME, Rehermann B, Feinstone S. Hepatitis C Viruses. In: Knipe DM, Howley PM, Griffin DE, Lamb RA, Martin MA, Roizman B, Straus SE (eds.). Fields Virology. 4th ed., Vol. I. Lippincott, Williams & Wilkins, Baltimore; 2001: pp. 1127-62), protein transcripts of such viruses are only poorly reliable targets for the delivery of compounds *via* a targeted liposomal system. In addition, even if viral envelope proteins (or bacterial cell-wall or membrane proteins) would qualify as suitable targets, these proteins are generally masked by heavy glycosylation. However, the structures and compositions of these viral envelope glycosyl branches (or, in the case of bacteria, glycocalices) feature very constant domains (e.g., Geyer H, Holschbach C, Hunsmann G, Schneider J. Carbohydrates of human immunodeficiency virus. Structures of oligosaccharides linked to the envelope glycoprotein 120. J Biol Chem 1988;25;263:11760-7).

As to viruses, and on the example of HIV, the underlying reason is that the amino acids of the HIV gp120 molecule are glycosylated in the host cell's rough endoplasmatic reticulum and further processed in the cell's Golgi apparatus (Kornfeld R, Kornfeld S. Assembly of asparagine-linked oligosaccharides. Annu Rev Biochem 1985;54:631-64). These processes, thus, work independently from any of HIV's own "sloppy" enzymes. As described below, a prominent component of gp120 (as of other viral and bacterial surface sugars) is mannose. In contrast, mammalian cell-surface or serum glycoprotein branches only rarely carry terminal mannose (Weis et al., Immunol Rev 1998;163:19-34). In one embodiment of the invention, the inventive liposomal delivery system contains or encapsulates a multivalent plant lectin(s) that strongly interacts with such envelope or glycocalical carbohydrates (which, most notably, feature mannose and fucose residues). When released into an infected cell the lectin(s) agglutinate intracellular reservoirs of infectious agents.

Specifically, the HIV envelope glycoproteins gp120 and gp41 form a trimeric complex that mediates HIV's entry into target cells (Allan JS, Coligan JE, Barin F, McLane MF, Sodroski JG, Rosen CA, Haseltine WA, Lee TH, Essex M. Major glycoprotein antigens that induce antibodies in AIDS patients are encoded by HTLV-III. Science 1985;228:1091-4). A large fraction of the accessible surface of gp120 in the trimer is composed of variable, heavily glycosylated core and loop structures that surround the receptor-binding regions (Wyatt R, Kwong PD, Desjardins E, Sweet RW, Robinson J, Hendrickson WA, Sodroski JG. The antigenic structure of the HIV gp120 envelope glycoprotein. Nature 1998;393:705-11). Approximately 50% of gp120's molecular weight is provided by sugars, all of them *N*-linked to anchor amino acids of gp120's peptide backbone (Botos I, Wlodawer A. Proteins that bind high-mannose sugars of the HIV envelope. Prog Biophys Mol Biol: in press). In fact, HIV gp120 features all three known categories of *N*-linked carbohydrate types with a common pentasaccharide core, i.e. *high-mannose* (33%), *hybrid* (4%), and *complex* (63%) (Kornfeld R, Kornfeld S. Assembly of asparagine-linked oligosaccharides. Annu Rev Biochem 1985;54:631-64).

In *high-mannose* oligosaccharides, two to six mannose residues are attached to the pentasaccharide core (Sanders RW, Venturi M, Schiffiier L, Kalyanaraman R, Katinger H, Lloyd KO, Kwong PD, Moore JP. The mannose-dependent epitope for neutralizing antibody 2G12 on human immunodeficiency virus type 1 glycoprotein gp120. J Virol 2002;76:7293-305). *Hybrid* oligosaccharides contain elements of both high-mannose and complex carbohydrate structures (Kornfeld R, Kornfeld S. Assembly of asparagine-linked oligosaccharides. Annu Rev Biochem 1985;54:631-64). *Complex* oligosaccharides can contain L-fucose (90%), mannose, galactose, *N*-acytylglucosamine, *N*-acytyl-galactosamine, and sialic acids (Rao VSR, Qasba PK, Balaji PV, Chandrasekaran R. Conformation of Carbohydrates. Harwood Academic Publishers, The Netherlands; 1998; Mizuochi T, Spellman MW, Larkin M, Solomon J, Basa LJ, Feizi T. Carbohydrate structures of the human immunodeficiency virus (HIV) recombinant envelope glycoprotein gp120 produced in Chinese-hamster ovary cells. Biochem J 1988;254:599-603; and Mizuochi T, Spellman MW, Larkin M, Solomon J, Basa LJ, Feizi T. Structural characterization by chromatographic profiling of the oligosaccharides of human immunodeficiency virus (HIV) recombinant envelope glycoprotein gp120 produced in Chinese hamster ovary cells. Biomed Chromatogr 1988;2:260-70).

Available crystal structures of oligosaccharides reveal only a limited number of conformations for glycosidic linkages (Petrescu AJ, Petrescu SM, Dwek RA, Wormald MR. A statistical analysis of N- and O-glycan linkage conformations from crystallographic data. Glycobiology 199:9:343-52). Molecular dynamics simulations and NMR results suggest that branched high-mannose carbohydrates, despite their flexible glycosidic linkages, have well-defined overall conformations (Woods RJ, Pathiaseril A, Wormald MR, Edge CJ, Dwek RA. The high degree of internal flexibility observed for an oligomannose oligosaccharide does not alter the overall topology of the molecule. Eur J Biochem 1998;258:372-86). It appears that this restricted oligosaccharide orientation is functionally important in the interaction with sugar-binding lectins. For example, second lectin-carbohydrate interactions may be enhanced by the reduced conformational space available, and the reduced entropic penalty created, by first lectin-carbohydrate interactions. Similar mechanisms are likely involved in multivalent lectin-carbohydrate interactions (Ng KK, Kolatkar AR, Park-Snyder S, Feinberg H, Clark DA, Drickamer K, Weis WI. Orientation of bound ligands in mannose-binding proteins. Implications for multivalent ligand recognition. J Biol Chem 2002;277:16088-95).

With their high contents of mannose and fucose, and their strong lectin interaction due to restricted conformational liberty for mannose residues, infectious agents such as HIV are well-suited for binding to exocellular C-type lectins and CTLD receptors. Indeed, as cells usually do not attach these sugars to their own secretory glycoproteins, we hypothesize that cells, in case that they are virally infected, specifically attach such saccharides, so as to enable other cells to successively recognize, bind, and ingest the released infectious agent by C-type lectins for their consecutive clearance. Yet, for infectious agents forming intracellular reservoirs, their highly conserved carbohydrate coats ensure their CTL/CTLD-mediated pathway of reservoir formation. At the same time, these characteristics bring about their Achilles' heel: molecules such as HIV gp120 broadly display carbohydrate features perfectly suited for therapeutic lectin interference.

### Therapeutically Active Compounds/Agents

As to the invention described herein, therapeutically active compounds can be specifically introduced into cell populations of interest by targeted liposomal delivery.

### Plant Lectins as Agents for Therapeutic Interference with Intracellular Pathogens

Lectins have been used in a variety of new medical applications such as, for example, in the development of novel approaches for the therapy of cancers (reviewed in de Mejia EG, Bradford T, Hasler C. The anticarcinogenic potential of soybean lectin and lunasin. Nutr Rev 2003;61:239-46. Erratum in: Nutr Rev 2003;61:293). Similarly, a broad range of lectins known to bind high-mannose carbohydrates on HIV's envelope protein gp120 has been recognized to provide a potential new way for controlling HIV infection (reviewed in Botos I, Wlodawer A. Proteins that bind high-mannose sugars of the HIV envelope. Prog Biophys Mol Biol: in press*).* The same rationale applies to many other infectious agents (reviewed in Cambi A, Figdor CG. Dual function of C-type lectin-like receptors in the immune system. Curr Opin Cell Biol 2003;15:539-46). Lectins can be classified according to their specific mechanism of carbohydrate interaction, their origin, or other criteria. As to their origin, high-mannose binding lectins can be from plants (Con-A, MHL, UDA, jacalin, GNA, SCL, NPA etc.), animals (DC-SIGN, MBL etc.), or from bluegreen algae (cyanovirin-N, scytovirin etc.) (Botos I, Wlodawer A. Proteins that bind high-mannose sugars of the HIV envelope. Prog Biophys Mol Biol: in press*).* Generally, all lectins bind saccharides via H-bonds, polar contacts and vander-Waals interactions. However, just like animal/human lectins, plant lectins bind carbohydrates with different specificities and binding preferences (Drickamer K. Multiplicity of lectin-carbohydrate interactions. Nature Struct Biol 1995;2:437-9).

In a preferred embodiment of the invention, plant lectins were chosen to be incorporated into the inventive targeted liposomal delivery system. Some plant lectins act mitogenic when given systemically/extracellularly to humans or animals. In these cases, the lectins induce the proliferation of discrete cells by engaging exocellular receptors, thus activating their signal transduction cascades. Some lectins exhibiting multiple sugar binding sites, when given systemically/extracellularly, agglutinate certain cell populations (e.g., erythrocytes) which, again, is mediated via engagement of the lectin with exocellular receptors. In this case, the lectins cross-link (agglutinate) several cells. Certain lectins display both features (Wimer BM, Mann PL. Mitogen Information Summarizes. Cancer Biother Radiophamaceut 2002;17).

Neither of these effects is acceptable when considering a therapeutic application of lectins. However, when encapsulated within a liposomal delivery system, lectins (or truncated versions thereof) are not exposed to the exterior milieu and are only released intracellularly. Consequently, the unfavorable systemic side-effects of some lectins - despite their most valuable therapeutic potentials - can be prevented by employing targeted delivery systems. In addition, the delivery system envisioned in context of the invention is intended for topical (e.g., subcutaneous) or organ-directed (e.g., *via* infusion into the hepatic artery) application for selectively targeting specific cell populations within a given location or organ. As a result, concentrations of lectin for achieving the desired effect(s) are far below those needed upon systemic administration. Moreover, therapeutically, such a lectin will be delivered for the purpose of agglutinating an infectious agent within the targeted cell. The first advantage of this strategy is to irreversibly mask the pathogens' surface molecules so as to functionally incapacitate the agent's pro-infective attachment sites. The second advantage, due to multiple lectin binding sites, is the creation of large complexes bound for phagolysosomal enzymatic degradation of both agent and lectin. Targeted cells could be destroyed in the course of this process. For example, apoptosis is known to be sometimes induced in response to receptor engagement (e.g., Coutinho-Silva R, Persechini PM, Da Cunha Bisaggio R, Perfettini J-L, Torres De Sa Neto AC, Kanellopoulos JM, Motta-Ly I, Dautry-Varsat A, Ojcius DM. P2Z/P2X7 receptor-dependent apoptosis of dendritic cells. Am J Physiol 276 [Cell Physiol 45] 1999:C1139-47; and Woltman AM, van der Kooij SW, Coffer PJ, Offringa R, Daha MR, van Kooten C. Rapamycin specifically interferes with GM-CSF signaling in human dendritic cells, leading to apoptosis via increased p27KIPI expression. Blood 2003;101:1439-45). However, even in such cases, total systemic concentrations of residual lectin upon leakage will be orders of magnitude lower than the activity optima of free lectin for effecting cell mitogenesis and/or agglutination.

### Specific Description of Selected Lectins

*Concanavalin A (Con-A):* Con-A, derived from the jack bean *Canavalia ensiformis,* is a well-characterized member of a larger family of plant lectins (reviewed in Sharon N. Lectin-carbohydrate complexes of plants and animals: an atomic view. Trends Biochem Sci 1993;18:221-6; Parkin S, Rupp B, Hope HK. Atomic Resolution Structure of Concanavalin A at 120 K. Acta Cryst 1996;D52, 1161-8; Blakeley MP, Kalb (Gilboa) AJ, Helliwell JR, Myles DAA. The 15-K neutron structures of saccharide-free concanavalin A. Proc Natl Acad Sci USA 2004;101:16405-10; and Harrop SJ, Helliwell JR, Wan TCM, Kalb (Gilboa) AJ, Tong L, Yariv J. Structure solution of a cubic crystal of concanavalin A complexed with methyl α-D-glucopyranoside Acta Cryst 1996;D52:143-55).

Con-A binds mannose and is most specific for α-Man-(1-3)-[α-Man-(1-6)]-Man cores of *N*-linked oligosaccharides (Mandal DK, Kishore N, Brewer CF. Thermodynamics of lectin-carbohydrate interactions. Titration microcalorimetry measurements of the binding of N-linked carbohydrates and ovalbumin to concanavalin A. Biochemistry 1994;33:1149-56). Con-A has been demonstrated to potently agglutinate HIV (e.g., Hayakawa T, Kawamura M, Okamoto M, Baba M, Niikawa T, Takehara S, Serizawa T, Akashi M. Concanavalin A-immobilized polystyrene nanospheres capture HIV-1 virions and gp120: potential approach towards prevention of viral transmission. J Med Virol 1998;56:327-31). Moreover, above pH 6.5, Con-A exists as a tetramer (Yariv J, Kalb AJ, Levitzki A. The interaction of concanavalin A with methyl alpha-D-glucopyranoside. Biochim Biophys Acta 1968;165:303-5), and each 25-kDa monomer is furnished with a sugar-binding site (Kalb AJ, Levitzki A. Metal-binding sites of concanavalin A and their role in the binding of α-methyl D-glucopyranoside. Biochem J 1968;109:669-72). The lectin, therefore, is able to cross-link (agglutinate) HIV. An additional advantage as to the application described herein is that the topology of the 237-residue Con-A is mainly unchanged in all native and liganded crystal structures, which implies that sugar binding does not involve significant conformational changes (Reeke Jr GN, Becker JW, Edelman GM. The covalent and three-dimensional structure of concanavalin A. IV. Atomic coordinates, hydrogen bonding, and quaternary structure. J Biol Chem 19.75;250:1525-47).

Two additional metal-binding sites per Con-A monomer capture a transition-metal ion and a Ca²⁺ ion (Kalb AJ, Levitzki A. Metal-binding sites of concanavalin A and their role in the binding of alpha-methyl D-glucopyranoside. Biochem J 1968; 109:669-72). Importantly, at low pH values, Con-A releases these metals and loses its sugar-binding capacity. However, addition of the missing metals restores sugar binding (Yariv J, Kalb AJ, Levitzki A. The interaction of concanavalin A with methyl alpha-D-glucopyranoside. Biochim Biophys Acta 1968;165:303-5). It was shown that the presence of Ca²⁺ is critical, because it interacts with an H₂O molecule, which is thought to stabilize Con-A's Ala₂₀₇-Asp₂₀₈ *cis* peptide bond (Naismith JH, Emmerich C, Habash J, Harrop SJ, Helliwell JR, Hunter WN, Raftery J, Kalb AJ, Yariv J. Refined structure of concanavalin a complexed with methyl α-D-Mannopyranoside at 2.0 Å resolution and comparison with the saccharide-free structure. Acta Crystallogr (D) 1994;50:847-58). In contrast, in the absence of the Ca²⁺, this peptide bond undergoes *cis-trans* isomerization, which is followed by an expansion of the sugar-binding loop Leu₉₉-Tyr₁₀₀ and results in the loss of the loop's ability to capture saccharide molecules (Bouckaert J, Loris R, Poortmans F, Wyns L. Crystallographic structure of metal -free concanavalin A at 2.5 Å resolution. Proteins 1995;23:510-24; and Reeke Jr GN, Becker JW, Edelman GM. Changes in the three-dimensional structure of concanavalin A upon demetallization. Proc Natl Acad Sci U S A 1978;75:2286-90).

In a preferred embodiment of the invention, Con-A is contained or encapsulated within the targeted liposomal delivery system to allow for agglutinating intraendosomally stored HIV. The low intraendosomal pH conditions require that the targeted liposomal delivery system co-encapsulates Ca²⁺ and transition-metal ions with the lectin for ensuring that it retains its sugar-binding capacity.

*Myrianthus holstii Lectin (MHL):* The roots of the African plant *Myrianthus holstii* Pal., Urticaceae, contain a 9284 Da lectin. The *Myrianthus holstti* lectin (MHL or Myrianthin; other names: Omufe; Mafwisa; Mswisya; Mswiza) (National Cancer Institute [NCI], Center for Cancer Research, USA. *Myrianthus holstii lectin.* http://home.ncifcif.gov/mtdp/compounds/714343.html) exhibits potent anti-HIV activity. MHL contains 16 disulfide-linked cysteine residues. Sequence analysis successfully assigned 79 amino acid residues out of 88, suggesting the presence of multiple isoforms differing in their primary structures at positions 52, 66, and 69 (Charan RD, Munro MH, O'Keefe BR, Sowder RCII, McKee TC, Currens MJ, Pannell LK, Boyd MR. Isolation and characterization of Myrianthus holstii lectin, a potent HIV-1 inhibitory proteins from the plant Myrianthus holstii. J Nat Prod 2000;63:1170-4; and reviewed in Botos I, Wlodawer A. Proteins that bind high-mannose sugars of the HIV envelope. Prog Biophys Mol Biol: in press*).* CEM-SS cells were protected by MHL from the cytopathic effects of the laboratory strain HIV-1RF. The effective concentration for 50% of cell protection (EC₅₀ value) was 1.4 µg/ml (150 nM). MHL did not prove to be toxic to target cells even at the highest tested concentration of 250 mg/ml (i.e., two orders of magnitude above the EC₅₀ value). The disulfide bonds apparently are structurally important for functional integrity, as the anti-HIV activity was lost upon their reductive cleavage (Balzarin J, Neyts J, Schols D, Hosoya M, Van Damme E, Peumans W, De Clercq E. The mannose-specific plant lectins from Cymbidium hybrid and Epipactis helleborine and the (N-acetyglucosamine)n-specific-specific plant lectin from Urtica dioica are potent and selective inhibitors of human immunodeficiency virus and cytomegalovirus replication in vitro. Antiviral Res 1992;18:191-207).

MHL reversibly inhibits HIV infection of host cells and, thus, needs to be present continuously for full activity (Charan RD, Munro MH, O'Keefe BR, Sowder RCII, McKee TC, Currens MJ, Pannell LK, Boyd MR. Isolation and characterization. of Myrianthus holstii lectin, a potent HIV-1 inhibitory protein from the planet Myrianthus holstii. J Nat Prod 2000;63:1170-4). As MHL can bind gp120 simultaneously with soluble CD4, MHL and CD4 appear to bind gp120 allosterically. From a series of sugars tested, only GlcNAc interferes with MHL-gp120 binding. Once bound to GlcNAc, MHL is unable to bind gp120, implying that MHL interacts with gp120 carbohydrate moieties. The lectin does not agglutinate human erythrocytes (Charan RD, Munro MH, O'Keefe BR, Sowder RCII, McKee TC, Currens MJ, Pannell LK, Boyd MR. Isolation and characterization of Myrianthus holstii lectin, a potent HIV-1 inhibitory protein from the plant Myrianthus holstii. J Nat Prod 2000;63:1170-4). Mitogenicity has not been tested for.

In an embodiment of the invention, MHL is contained or encapsulated within the targeted liposomal delivery system. However, MHL is not as extensively characterized as Con-A and does not exhibit Con-A's high specificity for α-Man-(1-3)-[α-Man-(1-6)]-Man cores of *N*-linked oligosaccharides. In addition, MHL inhibits HIV in a reversible manner. Finally, MHL exists as a monomer. Synthesis of di- or multimeric variants of MHL that can irreversibly agglutinate can also be prepared and employed. MHL can also be employed in targeted delivery systems directed to reservoirs formed by other infectious agents.

### Further Conditions

*Probability for Successful Pathogen. Agglutination:* It is imperative for endosomally delivered lectins to be able to effectively diffuse to their pathogen target for successful agglutination. As to the physicochemical conditions on whether this can be achieved, Kuthan's mathematical model for the location of single targets in subcellular compartments provides a basis. Proceeding from the simple unbiased (i.e., drift-free) Brownian movement of macromolecules (such as lectins) randomly searching for single or few target sites (such as viruses), he developed a WALK-AND-CAPTURE MODEL for random walks of individual macromolecules in spherical compartments (such as endosomes) (Kuthan H. A Mathematical model of single target site location by Brownian movement in subcellular compartments. J Theor Biol 2003;221:79-87); This model was based on the simple WALK-AND-CAPTURE MODEL first developed by Adam and Delbrück, and then complemented by others (Adam G, Delbrück M. Reduction of Dimensionality in Biological Diffusion Processes. In: Structural Chemistry and Molecular Biology. Rich A, Davidson N, eds. New York: Freeman & Co; 1968:198-215; Berg HC, Purcell EM. Physics of chemoreception. Biophys J 1977;20:193-219; and Szabo A, Schulten K, Schulten Z. First passage time approach to diffusion controlled reactions. J Chem Phys 1980;72:4350-7). Specifically, the closed-form analytic solution of the EXACT AND APPROXIMATE FIRST-PASSAGE-TIME (FPT) PROBABILITY DENSITY FUNCTION was applied. As determined on the example of a small intranuclear Cajal body (ø 0.1-1.0 µm, which is perfectly in the range of an endosome [Dundr M, Misteli T. Functional architecture in the cell nucleus. Biochem J 2001;356:297-310]), a single diffusing protein molecule, with an effective diffusion constant of D = 0.5 µm² s⁻¹ (deliberately set low) would encounter a medium-sized target protein (ø 5 nm) within 1 second with a near-certainty probability of *p* = 0.98 (Kuthan H. A Mathematical model of single target site location by Brownian movement in subcellular compartments. J Theor Biol 2003;221:79-87). As these considerations apply to endosomally delivered lectins as well, most rapid and complete agglutination of all target pathogens is expected as to this application of the invention described herein.

*Conditions for the Degradation of Intracellular Pathogen Reservoirs:* Representative for other CTL receptors, the general molecular and physicochemical conditions leading to reservoir formation - and thus encountered by a therapeutic plant lectin - are provided on the well-investigated example of DC-SIGN. Generally, the highly dynamic endosomal/lysosomal system of mammalian cells, physically, comprises early, recycling, and late endosomes, lysosomes, and the late Golgi apparatus. This system enables membrane transport for sorting and segregation of receptors and ligands from (i) the plasma membrane and (ii) the late Golgi. Molecules transported from the cell's surface to endosomes are internalized by receptor-mediated endocytosis *via* clathrin-coated pits (Teasdale RD, Loci D, Houghton F, Karlsson L, Gleeson PA. A large family of endosome-localized proteins related to sorting nexin 1. Biochem J 2001;358:7-16).

Whereas DC-SIGN-bound ligands generally are internalized for processing in degradation compartments, HIV is protected without being degraded (Kwon DS, Gregorio G, Bitton N, Hendrickson WA, Littman DR. DC-SIGN-mediated internalization of HIV is required for trans-enhancement of T cell infection. Immunity 2002;16:135-144). It is presently unclear how intact HIV virions escape this normal mechanism. However, it is known that, in mature mDCs, DC-SIGN is targeted to early endosomal compartments in which HIV is protected against degradation (Engering A, et al. The dendritic cell-specific adhesion receptor DC-SIGN internalizes antigen for presentation to T cells. J Immunol 2002;168:2118-2126), suggesting that maturation of mDC, as is induced by HIV, may lead to its altered internalization. In order to transmit the virus to T cells, virions internalized by DC-SIGN recycle back to the cell surface to contact entry receptors (CD4, CCR5, CXCR4) on the target cell (Geijtenbeek TBH, et al. DC-SIGN, a dendritic cell-specific HIV-1-binding protein that enhances trans-infection of T cells. Cell 2000;100:587-597). The other C-type lectins BDCA-2 and DEC-205 also deliver antigens to late endosomes or lysosomes. In addition, the mannose receptor (CD206) delivers antigen (or HIV) to early endosomes and directly recycles back to the surface (Cambi A, Figdor CG. Dual function of C-type lectin-like receptors in the immune system. Curr Opin Cell Biol 2003;15:539-46).

Specifically, both upon binding a ligand and in the course of routine uptake and membrane recycling, DC-SIGN is internalized by means of its two putative di-leucine- (LL) and tyrosine-based (YSQL) internalization motifs (Rapoport I, Chen YC, Cupers P, Shoelson SE, Kirchhausen T. Dileucine-based sorting signals bind to the β chain of AP-1 at a site distinct and regulated differently from the tyrosine-based motif- binding site. EMBO J 1998;17:2148-55; and Trowbridge IS. Endocytosis and signals for internalization. Curr Opin Cell Biol 1991;3:634-41). DC-SIGN is internalized into low-pH (6.0-6.8) early endosomal and lysosomal compartments (Engering A, Geijtenbeek TB, van Vliet SJ, Wijers M, van Liempt E, Demaurex N, Lanzavecchia A, Fransen J, Figdor CG, Piguet V, van Kooyk Y. The dendritic cell-specific adhesion receptor DC-SIGN internalizes antigen for presentation to T cells. J Immunol 2002; 168:2118-26). Indeed, such low pH conditions appear to be a prerequisite for the successful DC-SIGN-facilitated T-cell infection *in trans* from the intracellular reservoir (Kwon DS, Gregorio G, Bitton N, Hendrickson WA, Littman DR. (2002). DC-SIGN mediated internalization of HIV is required for trans-enhancement of T cell infection. Immunity 16:135-44). Internalization of HIV by DC-SIGN into a mDC's intracellular low-pH compartment thus appears critical for the chronic infectious role of the reservoir. Finally, it is conceivable that DC-SIGN remains attached to HIV while stored in this reservoir as, for example, is also the case for transferrin and its receptor while traversing the endosomal/lysosomal system before recycling back to the surface (Ciechanover A, Schwartz AL, Lodish HF. Sorting and recycling of cell surface receptors and endocytosed ligands: the asialoglycoprotein and transferrin receptors. J Cell Biochem 23;1982:107-30).

It was shown that neo-glycoproteins and monosaccharides interacting with mannose/fucose receptors stimulate lysosomal enzyme secretion in a dose- and time-dependent fashion. While combinations of sugars (most effectively L-fucose or mannose) and proteins stimulate lysosomal enzyme secretion more potently than the sugars alone, the same effect is achieved under both conditions. The fact that the secretion of lysosomal enzymes is restricted to several hours after incubation indicates that the receptor itself [Anntn: such as a CTL or CTLD receptor] participates in the process. Importantly, the fact that blocking of protein synthesis by cycloheximide does not inhibit enzyme secretion demonstrated that *de-novo* enzyme synthesis is not required (Ohsumi Y, Lee YC. Marrraose-receptor ligands stimulate secretion of lysosomal enzymes from rabbit alveolar macrophages. J Biol Chem 1987;262:7955-62). This implies that the katabolic enzymes are released from pre-formed lysosomal pools. As to the invention it is further important to note that internalization of the stimulating agent(s) [Anntn: the targeted compound delivery system] is essential, as temporary binding of such an agent to the cell surface alone fails to elicit lysosomal enzyme secretion (Ohsumi Y, Lee YC. Mannose-receptor ligands stimulate secretion of lysosomal enzymes from rabbit alveolar macrophages. J Biol Chem 1987;262:7955-62). This is enabled by the inventive targeting system. Most intriguingly, lysosomal enzyme secretion is induced to a similar extent both in the presence of an undegradable ligand or a degradable ligand (Ohsumi Y, Lee YC. Mannose-receptor ligands stimulate secretion of lysosomal enzymes from rabbit alveolar macrophages. J Biol Chem 1987;262:7955-62). As to the invention presented herein, pathogen reservoir-forming infectious agents, potentially attached receptors such as DC-SIGN, and the agglutinating lectins can thus be enzymatically degraded in phagolysosomes formed upon the fusion of targeted endosomal reservoir sites with lysosomes containing pathogen-digesting sets of enzymes.

### Intracellular Reservoirs of Pathogens other than HIV

As mentioned repeatedly, various pathogenic viruses, bacteria, and parasites besides HIV-1 form intracellular chronic infectious reservoirs. When limited to human pathogens, these include notorious species such as HIV-2, hepatitis C virus (HCV), cytomegalovirus (CMV), Epstein-Barr virus (EBV), Ebola, *Mycobacterium tuberculosis* and other *Mycobacterium* species, and *Leishmania* amastigotes (Cambi A, Figdor CG. Dual function of C-type lectin-like receptors in the immune system. Curr Opin Cell Biol 2003;15:539-46; Kaufmann SHE, Schaible UE. A Dangerous liaison between two major killers: Mycobacterium tuberculosis and HIV target dendritic cells through DC-SIGN. J Exp Med 2003;197;1-5; Baribaud F, Pöhlmann S, Doms RW. The role of DESIGN and DC-SIGNR in HIV and SIV attachment, infection, and transmission. Virology 2001;286:1-6; Pöhlmann S, Baribaud F, Lee B, Leslie GJ, Sanchez MD, Hiebenthal-Millow K, Munch J, Kirchhoff F, Doms RW. DC-SIGN interactions with human immunodeficiency virus type 1 and 2 and simian immunodeficiency virus. J Virol 2001;75:4664-72; Alvarez CP, Lasala F, Carrillo J, Muniz O, Corbi AL, Delgado R. C-type lectins DC-SIGN and L-SIGN mediate cellular- entry by Ebola virus in cis and in trans. J Virol 2002;76:6841-4; and Colmenares M, Puig-Kroger A, Muniz Pello O, Corbi AL, Rivas L. Dendritic-cell specific ICAM-3 grabbing nonintegrin (DC-SIGN, CD209), a C-type surface lectin in human dendritic cells, is a receptor for Leishmania amastigotes. J Biol Chem 2002;16:16).

Epidemiologically, two of these species are the causative agents of two of the major infectious diseases on a global scale. One of which, *M. tuberculosis* can occur in patients in problem-ridden co-infection with HIV (Schluger NW, Burzynski J. Tuberculosis and HIV infection: epidemiology, immunology, and treatment. HIV Clin Trials 2001;2:356-65; and Kaufmann SHE, Schaible UE. A Dangerous liaison between. two major killers: Mycobacterium tuberculosis and HIV target dendritic cells through DC-SIGN. J Exp Med 2003;197;1-5) or alone and is the most wide-spread bacterial infection (Frieden TR, Sterling TR, Munsiff SS, Watt CJ, Dye C. Tuberculosis. Lancet 2003;362:887-99; Corbett EL, Watt CJ, Walker N et al. The growing burden of tuberculosis: global trends and interactions with the HIV epidemic. Arch Intern Med 2003;163:1009-21; and van Lettow M, Fawzi WW, Semba RD. Triple trouble: the role of malnutrition in tuberculosis and human immunodeficiency virus co-infection. Nutr Rev 2003;61:81-90). The other, HCV, is considered the most wide-spread viral infection worldwide (Ray Kim W. Global epidemiology and burden of hepatitis C. Microbes Infect 2002;4:1219-25; Cheung RC, Hanson AK, Maganti K, Keeffe EB, Matsui SM. Viral hepatitis and other infectious diseases in a homeless population. J Clin Gastroenterol 2002;34:476-80; and Borgia G, Reynaud L, Gentile I, Piazza M. HIV and hepatitis C virus: facts and controversies. Infection 2003;31:232-40). These diseases, therefore, are urgent primary targets of the inventive therapeutic system.

To this end, it is known that the formation of intracellular reservoirs by *M. tuberculosis* and other myobacteria, again, is mediated *via* C-type lectin receptors (Maeda N, Nigou J, Herrmann JL et al. The cell surface receptor DESIGN discriminates between Mycobacterium species through selective recognition of the mannose caps on lipoarabinomannan. J Biol Chem 2003;278:5513-6; Geijtenbeek TB, Van Vliet SJ et al. Mycobacteria target DC-SIGN to suppress dendritic cell function. Exp Med. 2003;197:7-17; and Tailleux L, Schwartz O, Herrmann JL et al. DC-SIGN is the major Mycobacterium tuberculosis receptor on human dendritic cells. J Exp Med. 2003;197:121-7). The same applies to HCV (Pöhlmann S, Zhang J, Baribaud F et al. Hepatitis C virus glycoproteins interact with DC-SIGN and DC-SIGNR. J Virol 2003;77:4070-80; Lozach PY, Lortat-Jacob H, de Lacroix de Lavalette A et. al. DC-SIGN and L-SIGN are high affinity binding receptors for hepatitis C virus glycoprotein E2. J Biol Chem 2003;278:20358-66; and Gardner JP, Durso RJ, Arrigale RR et al. L-SIGN (CD209L) is a liver-specific capture receptor for hepatitis C virus. Proc Natl Acad Sci USA 2003;100:4498-503). Thus, the uptake mechanisms for both infectious agents provide the essential prerequisite for targeting their cellular reservoirs with the inventive system described herein.

As to the therapeutic compound to be delivered, a range of plant lectins, including Con-A, MHL, and others also bind to/agglutinate, and thus have the capacity to inactivate/eradicate these pathogens (as reviewed in Wimer BM, Mann PL. Mitogen Information Summaries. Cancer Biother Radiopharmaceut 2002;17-64; and Botos I, Wlodawer A. Proteins that bind high-mannose sugars of the HIV envelope. Prog Biophys Mol Biol: in press*).* The invention disclosed herein employs a fucose or polyfucose dependent cell specific targeting system that delivers a plant lectin into a pre-defined cell population(s), or subset(s) thereof, of a human or animal host, wherein the cell population(s) are reservoirs for pathogens/infectious agents.

In addition, for successively enabling complete degradation of the infectious agent within a cell's targeted compartment(s) (e.g., an endosome), the targeting system may, together with a given lectin(s), co-encapsulate a degradative enzyme(s) (e.g., mannosidase and/or *N*-acetylglucosaminidase), and/or a co-factor(s) essential for the lectin(s) and/or the enzyme(s) functional integrity (e.g., metal ions or co-enzymes), so as to (i) render the agent non-infectious by removing its surface carbohydrates, and to (ii) make its interior accessible to, e.g., the targeted cell's own (phago-)lysosomal enzymes for complete degradation of the pathogen's integral infectious, genomic, and structural components.

### Chronic Non-infectious Diseases: Cancers of the Colon and Rectum

Aside from infectious diseases, CTL receptor targeting may also enable to efficiently address therapeutically certain chronic non-infectious diseases. One prominent example is cancers of the colon and rectum. As to the CRD lectins of the REG family expressed in hepatogastroenterological organs and tissues, their currently 17 cloned and sequenced family members may offer new options for novel treatments. This is due to the fact that the members of the REG family, as regional CTL receptors, are involved in carcinogenesis, diabetes, inflammation and injury (reviewed in Zhang YW, Ding LS, Lai MD. Reg gene family and human diseases. World J Gastroenterol. 2003 Dec; 9(12):2635-41). As a result, it has been suggested that the role of some of these receptors in carcinogenesis makes them promising candidate molecules as (i) new prognostic indicators of tumor survival; (ii) early biomarkers of carcinogenesis; and (iii) molecular matrices for the design of novel chemotherapeutics (reviewed in Zhang YW, Ding LS, Lai MD. Reg gene family and human diseases. World J Gastroenterol 2003;9:2635-41).

The invention can complement and extend these earlier suggestions. Specifically, addressing members of the REG family in a hepatogastroenterological context by topical application of the inventive targeting system may afford delivering therapeutic drugs/compounds in a highly site-specific manner. In principal, such an approach may enable interference with either of the ailments mentioned above. However, the potentially most challenging application refers to cancers of the colon and rectum. As of 2003, in all western industrialized countries, such as the United States and Germany, such cancers have been the second most common cause of deaths from cancer in both sexes (Bruckner HW. Adenocarcinoma of the Colon and Rectum. In: Frei E, Holland JF (eds.) Cancer- Medicine. 5th ed. Chapter 103. BC Decker; Hamilton, London 2000: pp 1472-520; Jacobi V, Thalhammer A, Straub R, Vogl TJ. Importance of coloncontrast enema. Radiologe 2003;43:113-21). It presently appears unlikely that this situation will change considerably unless more promising treatment options become available (World Health Organization Mediacentre. Global cancer rates could increase by 50% to 15 million by 2020. Accessed on 8 August 2003. Available from: http://www.who.int/mediacentre/releases/2003/pr27/en/).

The inventive systems may, *via* targeting REG family members preferentially expressed in the different cancers of the colon and rectum, address these malignancies much more selectively than currently feasible. Moreover, selectivity may be further increased by applying this targeted compound delivery system *via* established organ-directed methods, such as, for example, its direct infusion into the hepatic artery (Kemeny MM, Alava G, Oliver JM. The effects on liver metastases of circadian patterned continuous hepatic artery infusion of FUDR. HPB Surg 1994;7:219-24). In addition, the inventive targeting system may specifically deliver already FDA-approved chemotherapeutic drugs, such as 5-fluorouracil, folinic acid, oxaliplatin and FUDR to cancer cells located in the liver and/or the intestines (Levi F, Misset J-L, Brienza S, Adam R, Metzger G, Itzakhi M, Caussanel JP, Kunstlinger F, Lecouturier S, Descorps-Declère A, Jasmin C, Bismuth H, Reinberg A. A chronopharmacologic phase II clinical trial with 5-fluorouracil, folinic acid, and oxaliplatin using an ambulatory multichannel programmable pump: high antitumor effectiveness against metastatic colorectal cancer. Cancer (Phila.) 1992; 69: 893-900*;* Kemeny MM, Alava G, Oliver JM. The effects on liver metastases of circadian patterned continuous hepatic artery infusion of FUDR. HPB Surg 1994;7:219-24). Finally, chronopharmacologic treatment regimens for such applications may be employed; even when employing non-targeted chemotherapeutics, clinical studies have revealed a clear advantage of circadian treatment protocols *(*Hrushesky WJ. Cancer chronotherapy: a drug delivery challenge. Prog Clin Biol Res 1990;341A:1-10*;* Levi F, Misset J-L, Brienza S, Adam R, Metzger G, Itzakhi M, Caussanel JP, Kunstlinger F, Lecouturier S, Descorps-Declère A, Jasmin C, Bismuth H, Reinberg A. A chronopharmacologic phase II clinical trial with 5-fluorouracil, folinic acid, and oxaliplatin using an ambulatory multichannel programmable pump: high antitumor effectiveness against metastatic colorectal cancer. Cancer (Phila.) 1992;69:893-900; Kemeny MM, Alava G, Oliver JM. The effects on liver metastases of circadian patterned continuous hepatic artery infusion of FUDR. HPB Surg 1994;7:219-24; Adler S, Lang S, Langenmayer I, Eibl-Eibesfeldt B, Rump W, Emmerich B, Hallek M. Chronotherapy with 5-fluorouracil and folinic acid in advanced colorectal carcinoma. Results of a chronophannacologic phase I trial. Cancer 1994;73:2905-12; and Mormont MC, Levi F. Cancer chronotherapy: principles, applications, and perspectives. Cancer 2003;97:155-69). As a result, by employing the targeted delivery system together with already approved drugs and established approaches for reducing pharmacologic side-effects, progress in the treatment of cancers of the colon and rectum may be achieved.

### Chronic Non-infectious Diseases: Non-alcoholic Steatohepatitis

Another example for a chronic non-infectious disease to which the inventive technology is applicable, is the non-alcoholic fatty liver disease (NAFLD). NAFLD is the most common liver disease in the developed countries, which may entail cirrhosis, acute-on-chronic liver failure and liver cancer. An advanced sub-entity of NAFLD is the so-called non-alcoholic steatohepatitis (NASH) (Angulo P. Nonalcoholic fatty liver disease. N Engl J Med 2002; 346:1221-31). Numerous studies have explored various treatment strategies for NASH, yet without any convincing benefit. Indeed, since the first description of NASH by Ludwig *et al.* (Ludwig J, Viggiano TR, McGill DB et al. Nonalcoholic steatohepatitis: Mayo Clinic experiences with a hitherto unnamed disease. Mayo Clin Proc 1980;55:434-38), no effective therapies have become available. Therefore, advanced insight into the actual pathomechanism(s) might now, for the first time, help to design truly efficacious therapeutic options. Specifically, a growing body of evidence suggests that pathologically increased hepatocyte apoptosis may be the most critical pathogenic mechanism contributing to inflammation and fibrogenesis of the liver and, thus, underlying the progression to steatohepatitis (Canbay A, Higuchi H, Bronk SF et al. Fas enhances fibrogenesis in the bile duct ligated mouse: a link between apoptosis and fibrosis. Gastroenterology 2002;123:1323-30; Jaeschke H. Inflammation in response to hepatocellular apoptosis. Hepatology 2002;35:964-6; Canbay A, Guicciardi ME, Higuchi H et al. Cathepsin B inactivation attenuates hepatic injury and fibrosis during cholestasis. J Clin Invest 2003;112:152-9). Thus, since both inflammation and fibrosis actually are prominent features of NASH, one may reasonably hypothesize that derailed intrahepatic apoptosis plays a key role in the progression of NAFLD to NASH (Feldstein AE, Canbay A, Angulo P et al. Hepatocyte apoptosis and fas expression are prominent features of human nonalcoholic steatohepatitis. Gastroenterology 2003;125:437-43).

Specifically, one may make a convincing case for a potentially high therapeutic benefit of inhibiting hepatocyte apoptosis in these patients. Such a strategy obviously might prevent liver inflammation, fibrosis, and their sequelae. To this end, inhibitors of caspases (i.e., key enzymes of the two principal apoptotic cascades) are currently being developed for clinical use. In the bile duct-ligated mouse model of cholestasis, the pan-caspase inhibitor, IDN-6556, has already proven beneficial as an antifibrotic agent (Canbay A, Feldstein A, Baskin-Bey E et al. The caspase inhibitor IDN-6556 attenuates hepatic injury and fibrosis in the bile duct ligated mouse. J Pharmacol Exp Ther 2004;308:1191-6). Likewise, treatment of HCV-positive patients with this inhibitor significantly reduces pathologic values of liver parameters (e.g., ALT) and, therefore, liver injury (Valentino KL, Gutierrez M, Sanchez R et al. First clinical trial of a novel caspase inhibitor: anti-apoptotic caspase inhibitor, IDN-6556, improves liver enzymes. Int J Clin Pharmacol Ther 2003;41:441-9). However, a recent approach has recently been presented by Eichhorst *et al.* who have applied suramin, a drug already approved by the FDA, to inhibit apoptosis in a mouse model of liver damage (Guicciardi ME, Gores GJ. Cheating death in the liver. Nat Med 2004;10:587-588; Eichhorst ST, Krueger A, Muerkoster S et al. Suramin inhibits death receptor-induced apoptosis in vitro and fulminant apoptotic liver damage in mice. Nat Med 2004;10:602-9). This finding may pave the way for first rationally-based therapies for patients suffering from the chronic liver disease NAFLD/NASH. The designing of novel liver-cell apoptosis-directed treatment options have recently been suggested (Canbay C, Gieseler RK, Gores GJ, Gerken G. The relationship between apoptosis and non-alcoholic fatty liver disease: an evolutionary cornerstone turned pathogenic. Z Gastroenterol 2005;43:211-7). As a caveat, the therapeutic long-term, and systemic, employment of anti-apoptotic drugs may potentially promote hyperproliferative disorders or even the development of certain types of cancers and/or lymphomas. However, the method of choice may be to deliver beneficial drugs, such as suramin, with a cell-specific targeted delivery system.

As to the invention described herein, members of the hepatogastroenterologically restricted family of REG proteins (reviewed in Zhang YW, Ding LS, Lai MD. Reg gene family and human diseases. World J Gastroenterol 2003 Dec; 9:2635-41), once more, comprise potential intrahepatic targeting structures. However, C-type lectins that may enable to address certain liver-cell subsets more specifically are (i) asialoglycoprotein receptors expressed by hepatocytes (which can be targeted with *Gal-4-Chol-labeled liposomes);* and (ii) mannose and fucose receptors expressed by non-parenchymal liver cells (which may both be targetable with *Fuc-4-Chol-labeled liposomes)* (Kawakami S, Wong J, Sato A, Hattori Y, Yamashita F, Hashida M. Biodistribution characteristics of mannosylated, fucosylated, and galactosylated liposomes in mice. Biochim Biophys Acta 2000;1524:258-65). In addition, *Fuc-4-Chol-labeled liposomes* may also address liver sinus endothelial cells by targeting liver/lymph node-specific ICAM-3-grabbing nonintegrin (L-SIGN), a DC-SIGN-related CTL receptor (Bashirova AA, Geijtenbeek TBH, van Duijnhoven GCV, van Vliet SJ, Eilering JBG, Martin MP, Wu L, Martin TD, Viebig N, Knolle PA, KewalRamani VN, van Kooyk Y, Carrington M. A dendritic cell-specific intercellular adhesion molecule 3-grabbing nonintegrin (DC-SIGN)-related protein is highly expressed on human liver sinusoidal endothelial cells and promotes HIV-1 infection. J Exp Med 2001;193:671-8).

In fact, in the absence of any efficient treatment for NAFLD/NASH as the most prevalent liver disease in the industrialized countries, such options may enable the first truly efficacious intervention strategies. Moreover, it can be expected that approaches based on the same rationale may also take effect in treating other diseases that likewise reveal increased, and pathogenetically relevant, rates of apoptosis (Canbay C, Gieseler RK, Gores GJ, Gerken G. The relationship between apoptosis and non-alcoholic fatty liver disease: an evolutionary cornerstone turned pathogenic. Z Gastroenterol 2005;43:211-7). Specifically, the therapeutic approach suggested harnesses two potent biological allies. First, for cell-specific delivery, targeting exocellular liver cell-specific CRD lectin domains; second, for interfering with derailed apoptosis, therapeutically targeting (for example, via the drug suramin) intracellular caspases of the apoptotic cascade. Both, the CRD lectin system and the system of apoptosis, are deeply engraved within the slate of our biological heritage. In other words, building upon these cornerstones of cellular evolution on earth lets such an approach appear to be bound for success. Employing an apoptosis/caspase-inhibiting drug such as suramin (Eichhorst ST, Krueger A, Muerkoster S et al. Suramin inhibits death receptor-induced apoptosis, in vitro and fulminant apoptotic liver damage in mice. Nat Med 2004;10:602-9) *via* hepatotopic and chronopharmacologic employment of the inventive targeting system (Kemeny MM, Alava G, Oliver JM. The effects on liver metastases of circadian patterned continuous hepatic artery infusion of FUDR. HPB Surg 1994;7:219-24) may allow for a first efficacious treatment of NAFLD/NASH, while greatly minimizing any potentially detrimental effects of the drug.

Some embodiments of the present invention are directed to a method of preferentially delivering an active agent to a reservoir cell(s), including an immune cell, of a mammalian subject, including a human. The targeted reservoir cells include myeloid progenitor cells in the bone marrow, monocytes, myeloid dendritic cells, macrophages, follicular dendritic cells, plasmacytoid dendritic cells, T lymphocytes, and natural killer cells. In the following, the term "dendritic cell" includes a myeloid, a plasmacytoid, or a follicular dendritic cell. The term "T lymphocyte" includes, but is not limited to, a T-helper cell, a T-regulatory cell, or a T-memory cell.

The term "preferentially" means that the targeting system, namely, the lipid-active agent/compound complex, or the targeted liposome having an outer surface that compresses a targeted ligand, is delivered to the cell, and the active agent/compound (e.g., the plant lectin or drug) is taken up by the cell more effectively than, in contrast to the invention, by delivery and uptake of the agent using a comparable lipid-active agent complex, or liposome, having an outer surface that does not comprise a targeting ligand, such as, mono- or poly-fucose moieties.

The method involves injecting into the mammalian subject, or applying, topically or otherwise, a lipid-active agenet complex, such as a targeted liposome, in accordance with the invention that comprises the active agent, or combination of active agents, the immune cell being infected with, or susceptible to infection with, an infectious agent (or pathogen), such as, but not limited to, human immunodeficiency virus (HIV). In some embodiments, the immune cell is infected with, or susceptible to infection with, an infectious agent, such as a virus, a bacterium, a fungus, or a protozoan. Examples of infectious agents are HIV-1, HIV-2, HCV, HSV, EBV, HPV, influenza virus, Ebola virus, or *Mycobacterium tuberculosis.* Some embodiments are particularly directed to intracellular targeting for intraendosomal HIV inactivation.

The present invention is also particularly directed to inventive targeting systems, such as lipid-active agent/compound complexes, including targeted liposomes, wherein their surfaces are labeled with a targeting ligand, such as a mono- or polyfucose, which are attached to the complexes and liposomes by known techniques such as have been presented herein, i.e., by preparing fucosylated (Fuc) liposomes by means of cholesterol linkage and membrane anchoring with cholesten-5-yloxy-*N*-(4-((1-imino-2-beta-D-thiofucosyle thyl)amino)alkyl)formamide (or, briefly, Fuc-C4-Chol), and the subsequent formulation of liposomes as a composition of distearoylphosphatidylcholine (DSPC), cholesterol (Chol), and Fuc-C4-Chol at a molar ratio of 60:35:5 (e.g., Kawakami S, Wong J, Sato A, Hattori Y, Yamashita F, Hashida M. Biodistribution characteristics of mannosylated, fucosylated, and galactosylated liposomes in mice. Biochim Biophys Acta. 2000 Dec 15;1524(2-3):258-65). Fucose, polyfucose and polyfucose derivatives are examples of targeting ligands or CRD receptor-specific anchors. In other embodiments of the invention, the targeting ligand or CRD receptor-specific anchor specifically binds a CTL or CTLD receptor. Such targeting ligands may also include, galactose, polygalactose and polygalactorse derivatives.

As to the invention described herein, the syntheses of the inventive targeting ligands or CRD receptor-specific anchors, including CTL receptor-specific carbohydrate anchors, for liposomal membrane modification are based on the protocols of Kawakami et al. In their study, they synthesized liposome membrane anchors of cholesterol derivatized with galactose (Gal-C4-Chol), mannose (Man-C4-Chol), and fucose (Fuc-C4-Chol),i.e., cholesten-5-yloxy-N-(4-((1-imino-2-1-d-thiogalactosylethyl)amino)alkyl)formamide; cholesten-5-yloxy-N-(4-1-imino-2-1-d-thiomannosylethyl)amino)alkyl)formamide; and cholesten-5-yloxy-N-(4-((1-imino-2-1-d-thiofucosylethyl)amino)alkyl)formamide, respectively (Kawakami S, Wong J, Sato A, Hattori Y, Yamashita F, Hashida M. Biodistribution characteristics of mannosylated, fucosylated, and galactosylated liposomes in mice. Biochim Biophys Acta 2000; 1524:258-65), as methodologically first developed in an earlier work of this group (Kawakami S, Yamashita F, Nishikawa M, Takakura Y, Hashida M. Asialoglcoprotein receptor-mediated gene transfer using novel galactosylated liposomes. Biochem Biophys Res Commun 1998;252:78-83).

Their protocols have been modified according to other known methodological steps (Nishikawa M, Kawakami S, Yamashita F, Hashida M. Glycosylated cationic liposomes for carbohydrate receptor-mediated gene transfer. Meth Enzymol 2003;373:384-399; Wolfrom ML, Thompson A. Acetylation. Methods in Carbohydrate Chemistry. Vol. II; pp. 211-5 (1963); Chipowsky Y, Lee YC. Synthesis of 1-thioaldosides having an amino group at the aglycon terminal. Carbohyd Res 1973;31:339-346; and Lee YC, Stowell CP, Krantz ML. 2-Imino-2-methoxyethylen 1-thioglycosides: new reagents for attaching sugars to proteins. Biochemistry 1976;15:3956-3963). Finally, further novel chemical modifications as well as quality control measures have been included. As to the mannose (Man-C4-Chol), fucose (Fuc-C4-Chol), and galactose (Gal-C4-Chol) targeting anchors obtained, these variants served different purposes. Due to its proven efficacy, Man-C4-Chol was employed as a positive control. Due to a binding efficacy similar to that of Man-C4-Chol, Fuc-4-Chol was specified as the targeting anchor for the delivery system. Finally, Gal-C4-Chol was used as a negative control when targeting mannose/fucose-specific targeting structures. However Gal-4-Chol is further envisioned as a specific targeting anchor of liposomes directed towards non-parenchymal liver cells (Kawakami S, Wong J, Sato A, Hattori Y, Yamashita F, Hashida M. Biodistribution characteristics of mannosylated, fucosylated, and galactosylated liposomes in mice. Biochim Biophys Acta 2000;1524:258-65).

A "complex" is a mixture or adduct resulting from chemical binding or bonding between and/or among its constituents or components, including the lipid, active agent, targeting ligand and/or other optional components of the inventive lipid-active agent complex or targeting system. Chemical binding or bonding can have the nature of a covalent bond, ionic bond, hydrogen bond, van der Waal's bond, hydrophobic bond, or any combination of these bonding types linking the constituents of the complex at any of their parts or moieties, of which a constituent can have one or a multiplicity of moieties of various sorts. Not every constituent of a complex need be bound to every other constituent, but each constituent has at least one chemical bond with at least one other constituent of the complex. In accordance with the present invention, examples of lipid-active agent complexes include liposomes (lipid vesicles), or lipid-active agent sheet-disk complexes. Lipid-conjugated active agents can also be a part of the lipid-active agent complex in accordance with the invention. In embodiments of the invention, the active agent is encapsulated within the lipid-active agent complex and the lipid-active agent complex has a targeting ligand on its outer surface. Active agents may be encapsulated with the lipid formulation, including encapsulation within a liposome.

Useful techniques for making lipid-active agent complexes, such as liposomes, are known in the art (e.g., Sullivan SM, Gieseler RKH, Lenzner S, Ruppert J, Gabrysiak TG, Peters JH, Cox G, Richer, L, Martin, WJ, and Scolaro, MJ. Inhibition of human immunodeficiency virus-1 propagation by liposome-encapsulated sense DNA to the 5' TAT splice acceptor site. Antisense Res Dev 1992;2:187-97; Laverman P, Boerman OC, Oyen WJG, Corstens FHM, Storm G, In vivo applications of PEG liposomes: unexpected observations. Crit Rev Ther Drug Carrier Syst 2001;18:551-66); Oussoren C, Storm G, Liposomes to target the lymphatics by subcutaneous administration, Adv Drug Deliv Rev 50(1-2):143-56 [2001]; Bestman-Smith J, Gourde P, Desormeaux A, Tremblay MJ, Bergeron MG, Sterically stabilized liposomes bearing anti-HLA-DR antibodies for targeting the primary cellular reservoirs of HIV-1, Biochim Biophys Acta 1468(1-2):161-74 [2000]; Bestman-Smith J, Desormeaux A, Tremblay MJ, Bergeron MG, Targeting cell-fi-ee HIV and virally-infected cells with anti-HLA-DR immunoliposomes containing amphotericin B, AIDS 14(16):2457-65 [2000]; Mayer LD, Hope MJ, Cullis PR. Vesicles of variable sizes produced by a rapid extrusion procedure, Biochim Biophys Acta 858: 161-168 [1986]; Kinman, L. et al., Lipid-drug associations enhanced HIV protease inhibitor indinovir localization in lymphoid tissues and viral load reduction: a proof of concept study in HIV-infected macaques, J AIDS [2003; in press]; Harvie P, Desormeaux A, Gagne N, Tremblay M, Poulin L, Beauchamp D, Bergeron MG, Lymphoid tissues targeting of liposome-encapsulated 2',3'-dideoxyinosine, AIDS 1995 Jul; 9(7):701-7 [1995]; U.S. Patent No. 5,773,027; U.S. Patent No. 5,223,263; WO 96/10399 A1).

Some useful methods of liposome preparation include extrusion, homogenization, remote loading, and reverse phase evaporation. In extrusion, a lipid film composed of phospholipids by themselves, or in combination with cholesterol, is formed by evaporating the organic solvent (such as chloroform) from the lipid solution. Hydrophobic active agents are added to the lipid solution prior to solvent evaporation. For entrapment of water soluble active agents, the dry lipid film is hydrated with and isotonic aqueous solution containing the active agent by agitation (ultrasound, vortex, motorized stirrer, etc.). The lipid suspension is frozen and thawed three or four times. The suspension is then passed through a series of polycarbonate filters containing pores of a defined diameter, such as 0.8 *µ*m, 0.4 *µ*m, 0.2 *µ*m, or 0.1 *µ*m. For water soluble active agents, unencapsulated active agents are removed by gel permeation column chromatography, dialysis or diafiltration. The liposomes can be sterile-filtered (e.g., through a 0.22 *µ*m filter).

A cryoprotectant, such as lactose, glucose, sucrose can be added to the sterile liposomes as long as isotonicity is maintained. The liposomes can then be frozen and lyophilized and stored indefinitely as a lyophilized cake (e.g., Mayer LD, Hope MJ, Cullis PR. Vesicles of variable sizes produced by a rapid extrusion procedure, Biochim Biophys Acta 858: 161-168 [1986]; Tsvetkova NM et al. Effect of sugars on headgroup mobility in freeze-dried dipalmitoylphosphatidylcholine bilayers: solid-state 31P NMR and FTIR studies, Biophys J 75: 2947-2955 [1998]; Crowe JH, Oliver AE, Hoekstra FA, Crowe LM. Stabilization of dry membranes by mixtures of hydroxyethyl starch and glucose: the role of vitrification, Cryobiology 35: 20-30 [1997]; Sun WQ, Leopold AC, Crowe LM, Crowe JH. Stability of dry liposomes in sugar glasses, Biophys J 70: 1769-1776 [1996]).

Homogenization is suited for large scale manufacture. The lipid suspension is prepared as described above. Freeze-and-thaw steps on a large scale may be a problem. The diameter of the liposomes is reduced by shooting the lipid suspension as a stream either at an oncoming stream of the same lipid suspension (microfluidization) or against a steel plate (gualinization). This later technology has been used by the dairy industry for homogenization of milk. Untrapped water soluble active agents are removed by diafiltration. Hydrophobic active agents are completely entrapped and there usually is no free active agent to be removed. (e.g., Paavola A, Kilpelainen I, Yliruusi J, Rosenberg P, Controlled release injectable liposomal gel of ibuprofen for epidural analgesia, Int J Pharm 199: 85-93 [2000]; Zheng S, Zheng Y, Beissinger RL, Fresco R, Liposome-encapsulated hemoglobin processing methods, Biomater Artif Cells Immobilization Biotechnol 20: 355-364 [1992]).

Another method of active agent entrapment is remote loading. The active agent to be entrapped must carry a charge. The degree of protonation or deprotonation is controlled by the pK of the ionizable group. A conjugate acid or base is trapped inside the liposomes. The ionizable active agent is added to the outside of the liposomes. The pH is dropped such that the active agent serves as a neutralizing salt of the ionizable substance trapped inside the liposomes. The counterion to the entrapped ionizable molecule can diffuse out of the liposomes due to the change in pH. This creates a gradient with sufficient energy to cause the active agent to diffuse into the liposomes. An example is the loading of doxorubicin into preformed liposomes.

In reverse phase evaporation, a lipid film is solubilized in diethylether to a final concentration of typically about 30 mM. Typically, one part water with entrapped active agent is added to 3 parts ether lipid solution. Energy in the form of sonication is applied forcing the suspension into a homogeneous emulsion. After a stable emulsion has been formed (does not separate out after standing for 1 to 3 hours), the ether is removed by evaporation, typically yielding liposomes with about a 200 nm diameter and a high trapping efficiency.

Ethanol/Calcium liposomes for DNA Entrapment, typically yielding liposomes 50 nm in diameter, are prepared by any of the above methods (extrusion, homogenization, or sonication). The liposomes are mixed with plasmid DNA plus 8 mM calcium chloride. Ethanol is typically added to the suspension to yield a concentration of about 40%. The ethanol is removed by dialysis and the resultant liposomes are generally less than 200 nm in diameter with about 75% of the DNA entrapped in the liposomes.

For cellular targeting, in accordance with the present invention, liposomes can be prepared by any of the above methods. The liposomes can contain a lipid to which proteins can be crosslinked. Examples of these lipids are: N-glutaryl-phosphatidylethnaolamine, N-succinyl-phospatidyethanolamine, Maleimido- phenyl-utyryl-phosphatidylethanolamine, succinimidyl-acetylthioacetate-phosphatidylethanolamine, SPDP-phosphatidlyethnaolamine. The glutaryl and succinimidyl phosphosphatidylethanolamine can be linked to a nucleophile, such as an amine, using cyclocarbodiimide. The maleimido, acetylthioacetate and SPDP phosphatidylethanolamines can be reacted with thiols on the proteins, peptides or small molecular weight ligands (<1000 gm/mole). The protein can be derivatized to the liposomes after formation. Underivatized protein can be removed by gel permeation chromatography. Peptides and low molecular weight ligands can be derivatized to the lipids and added to the organic lipid solution prior to formation of the lipid film.

In accordance with the present invention, examples of useful lipids include any vesicle-forming lipid, such as, but not limited to, phospholipids, such as phosphatidylcholine (hereinafter referred to as "PC"), both naturally occurring and synthetically prepared, phosphatidic acid (hereinafter referred to as "PA"), lysophosphatidylcholine, phosphatidylserine (hereinafter referred to as "PS"), phosphatidylethanolamine (hereinafter referred to as "PE"), sphingolipids, phosphatidyglycerol (hereinafter referred to as "PG"), spingomyelin, cardiolipin, glycolipids, gangliosides, cerebrosides and the like used either singularly or intermixed such as in soybean phospholipids (e.g., Asolectin, Associated Concentrates). The PC, PG, PA and PE can be derived from purified egg yolk and its hydrogenated derivatives.

Optionally, other lipids such as steroids, cholesterol, aliphatic amines such as long-chained aliphatic amines and carboxylic acids, long chained sulfates and phosphates, diacetyl phosphate, butylated hydroxytoluene, tocopherols, retinols, and isoprenoid compounds can be intermixed with the phospholipid components to confer certain desired and known properties on the formed vesicles. In addition, synthetic phospholipids containing either altered aliphatic portions such as hydroxyl groups, branched carbon chains, cycloderivatives, aromatic derivatives, ethers, amides, polyunsaturated derivatives, halogenated derivatives or altered hydrophilic portions containing carbohydrate, glycol, phosphate, phosphonate, quarternary amine, sulfate, sulfonate, carboxy, amine, sulfhydryl, or imidazole groups and combinations of such groups can be either substituted or intermixed with the above-mentioned phospholipids and used in accordance with the invention. Some of these components are known to increase liposomal membrane fluidity, thus entailing more efficacious uptake, others are known to have a direct effect on, e.g., tumor cells by affecting their differentiation potential. It will be appreciated from the above that the chemical composition of the lipid component prepared by the method of the invention can be varied greatly without appreciable diminution of percentage active agent capture, although the size of a vesicle can be affected by the lipid composition.

Saturated synthetic PC and PG, such as dipalmitoyl can also be used. Other amphipathic lipids that can be used, advantageously with PC, are gangliosides, globosides, fatty acids, stearylamine, long chain alcohols, and the like. PEGylated lipids, monoglycerides, diglycerides, triglycerides can also be included. Acylated and diacylated phospholipids are also useful.

By way of further example, in some embodiments, useful phospholipids include egg phosphatidylcholine ("EPC"), dilauryloylphosphatidylcholine ("DLPC"), dimyristoylphospha-tidylcholine ("DOPC"), dipalmitoylphosphatidylcholine ("DPPC"), distearoylphosphatidylcholine ("DSPC"), 1-myristoyl-2-palmitoylphosphatidylcholine ("MPPC"), 1-palmitoyl-2-myristoyl phosphatidylcholine ("PMPC"), 1-palmitoyl-2-stearoyl phosphatidylcholine ("PSPC"), 1-stearoyl-2-palmitoyl phosphatidylcholine ("SPPC"), dioleoylphosphatidylycholine ("DOPC"), dilauryloyl-phosphatidylglycerol ("DLPG"), dimyristoylphosphatidylglycerol ("DMPG"), dipalmitoylphos-phatidylglycerol ("DPPG"), distearoylphosphatidylglycerol ("DSPG"), distearoyl sphingomyelin ("DSSP"), distearoylphophatidylethanolamine (DSPE), dioleoylphosphatidylglycerol ("DOPG"), dimyristoyl phosphatidic acid ("DMPA"), dipalmitoyl phosphatidic acid ("DPPA"), dimyristoyl phosphatidylethanolamine ("DMPE"), dipalmitoyl phosphatidylethanolamine ("DPPE"), dimyr-istoyl phosphatidylserine ("DMPS"), dipalmitoyl phosphatidylserine ("DPPS"), brain phosphatidyl-serine ("BPS"), brain sphingomyelin ("BSP"), and dipalmitoyl sphingomyelin ("DPSP").

In one embodiment, phosphatidylcholine and cholesterol are employed. However, any suitable molar ratio of a non-steroidal lipid:steroidal lipid (e.g., cholesterol) mixture can optionally be employed to promote the stability of a particular lipid-active agent complex during storage and/or delivery to a mammalian subject.

Mixing the active agent and lipids can be by any useful known technique, for example, by sonication, vortexing, extrusion, microfluidization, homogenization, use of a detergent (later removed, e.g., by dialysis). The active agent and lipid are mixed at a lipid-to-active agent molar ratio of about 3:1 to about 100:1 or higher (especially useful for relatively more toxic active agents), and more preferably about 3:1 to about 10:1, and most preferably about 5:1 to about 7:1.

For some active agents, the use of an organic solvent can facilitate the production of the lipid-active agent complex, such as a liposome. The organic solvent is removed, after the mixing of the active agent and lipids, by any suitable known means of removal, such as evaporating by vacuum, or by the application of heat, for example by using a hair dryer or oven, or hot ethanol injection (e.g., Deamer, United States Patent No. 4,515,736), as long as the lipid and active agent components are stable at the temperature used. Dialysis and/or chromatography, including affinity chromatography can also be employed to remove the organic solvent. Hydrating the active agent is performed with water or any biocompatible aqueous buffer, e.g., phosphate-buffered saline, HEPES, or TRIS, that maintains a physiologically balanced osmolarity. Rehydration of liposomes can be accomplished, simultaneously with removing the organic solvent, or alternatively, can be delayed until a more convenient time for using the liposomes (see, e.g., Papahadjopoulos et al., United States Patent No. 4,235,871). The shelf life of hydratable (i.e., "dry") liposomes is typically about 8 months to about one year, which can be increased by lyophilization.

In one embodiment, the lipid-active agent complex is a unilamellar liposome. Unilamellar liposomes provide the highest exposure of active agent to the exterior of the liposome, where it may interact with the surfaces of target cells and/or infectious agents within the target cell. However, multilamellar liposomes can also be used in accordance with the present invention. The use of PEGylated liposomes is also encompassed within the present invention.

The lipid-active agent complex, such as a liposome, is preferably, but not necessarily, about 30 to about 250 nanometers in diameter.

In accordance with the present invention, the lipid-active agent complexes can be preserved for later use by any known preservative method, such as lyophilization (e.g., Crowe et al., United States Patent No. 4,857,319). Typically, lyophilization or other useful cryopreservation teclmiques involve the inclusion of a cryopreservative agent, such as a disaccharide (e.g., trehalose, maltose, lactose or sucrose).

The lipid-active agent complex, e.g., a liposome containing the active agent, is administered to a subject by any suitable means, for example by injection. Injection can be intrarterial, intravenous, intrathecal, intraocular, subcutaneous, intramuscular, intraperitoneal, or by direct (e.g., stereotactic) injection into a particular lymphoid tissue, or into a tumor or other lesion. Subcutaneous or intramuscular injection are preferred for introducing the lipid-active agent complex into lymphatic vessels.

In accordance with the present invention, "lymphoid tissue" is (i) a lymph node, such as an inguinal, mesenteric, ileocecal, or axillary lymph node; (ii) the spleen; (iii) the thymus, (iv) mucosal-associated lymphoid tissue such as found in the lung, the lamina propria of the of the intestinal wall, the structures termed Peyer's patches associated with the small intestine, or (v) Waldeyer's neck ring also encompassing the lingual, palatine and pharyngeal tonsils as anatomically dicrete structures.

Injection is by any method that drains directly, or preferentially, into the lymphatic system or bone marrow, as opposed to the blood stream. Therefore, most preferred are subcutaneous, intracutaneous, and bone marrow-directed injection routes, typically employing a syringe needle gauge larger than the lipid-active agent complex. Referring to the localization of cells providing chronic infectious reservoirs, suitable intraplacental or intrauteral application, as well as intraperitoneal injection routes are also useful. Finally, organ-specific application routes, such as, but not limited to, application via the hepatic artery are useful. Typically, injection of the injectate volume (generally about 1-5 cm³) is into the subject's arm, leg, or belly, but any convenient site can be chosen for subcutaneous injection. As the active agent, e.g., when administered subcutaneously, in accordance with some embodiments of the present invention, enters the lymphatic system prior to entering systemic blood circulation, benefits include (i) its distribution throughout the lymphoid system and localization in lymph nodes; (ii) to avoid or minimize (serum) protein-mediated destabilization of lipid-active agent complexes; and (iii) the delivery of the agent at concentrations that cannot be achieved with a soluble form of the active agent administered by any other (e.g., the intravenous) route of administration.

Typically, in treating HIV/AIDS, the frequency of injection is most preferably once per week, but more or less frequent injections (e.g., monthly) can be appropriate, too.

For purposes of the present invention, the "active agent" is an agent, a lectin, a drug, or an immunomodulatory compound (i) active against an infectious agent of interest, (ii) directed against a neoplastic formation of interest, or (iii) interfering with a non-infectious chronic degenerative disease of interest.

In a preferred embodiment, the active agent is a plant lectin. Plant lectins are a class of substances highly interesting for intraendosomal inhibition of HIV or other infectious agents establishing chronic intracellular/intraendosomal reservoirs. Although the present invention does not depend on any particular mechanism for its therapeutic effectiveness, the ability of plant lectins to strongly bind mannose or fucose (such as, for example, prominently displayed by the HIV envelope glycoprotein gp 120) is thought to therapeutically interfere with the virus-cell fusion process. Examples of useful plant lectins include: the mannose-specific plant lectins from *Canavalia ensiformis, Myrianthus holstii, Galanthus nivalis, Hippeastrum hybrid, Narcissus pseudonarcissus, Epipactis helleborine,* and *Listera ovata,* and the *N-*acetylglucosamine-specific lectin from *Urtica dioica* which inhibit HIV-1 and HIV-2 infection at an IC₅₀ of about 0.04 to about 0.08 µg/ml. Importantly, as this low concentration is required upon non-targeted delivery, still much lower concentrations may be achieved by cell-targeted liposomal delivery. An irreversible agglutination network is formed among intraendosomally stored HIV virions or other pathogen particles in reservoir cells, thus inactivating the infectious agent's infectious capacity. Plant lectins strongly interfere with herpes simplex viruses (HSV-1, HSV-2), cytomegalocirus (CMV), influenza virus, and other viruses. Specifically, hepatitis virus C (HCV) and vesicular stomatitis virus (VSV) are inactivated by plant lectins derived from *Canavalia ensiformis* and *Arceuthobium spp.* (mistletoe). This may carry the same concept, even without major modification(s), directly to its application in other viral diseases.

Spatially, tetramers of plant lectins are typically ~0.5 Å (5 nm) in diameter (which, depending on the pH, exist either as dimers or tetramers). Thus, many such molecules should be entrapped in, and delivered from, a liposome of a diameter of approximately 150-200 nm to successively agglutinate endosomally stored viral reservoirs.

In other embodiments of the invention, the active agent comprised by the mono- or poly-fucose targeted liposome is a drug, which can be an anti-viral drug or virostatic agent (such as an interferon), a nucleoside analog, or a non-nucleoside anti-viral drug. Examples include anti-HIV drugs (e.g., a HIV reverse protease inhibitor), such as indinavir (aka Crixivan®, Merck & Co., Inc., Rahway, NJ; saquinavir (N-tert-butyl-decahydro-2-[2(R)-hydroxy-4-phenyl-3(S)-[[N-(2-quinolylcarbonyl)-L-asparaginyl]-amino]butyl]-(4aS,8aS)-isoquinoline-3(S)-carboxamide; MW 670.86 Da (aka Fortovase^{®}, Roche Laboratories, Inc., Nutley, NJ); or nelfinavir (i.e., nelfinavir mesylate, aka Viracept^{®}; [3S-[2(2S*, 3S*), 3a,4ab,8ab]]-N-(1,1-dimethylethyl)decahydro-2-[2-hydroxy-3-[(3-hydroxy-2-methylbenzoyl)amino]-4-(phenylthio)butyl]-3-isoquinolinecarboxamide mono-methanesulfonate (salt), MW = 663.90 Da [567.79 as the free base]; Agouron Pharmaceuticals, Inc., La Jolla, CA). Nelfinavir mesylate is a white to off-white amorphous powder, slightly soluble in water at pH <4 and freely soluble in methanol, ethanol, isopropanol and propylene glycol. Other examples of antiviral drug include reverse transcriptase inhibitors, such as tenofovir disoproxil fumarate (9-[(R)-2-[[bis[[(isopropoxycarbonyl) oxy]methoxy] phosphinyl]methoxy] propyl]adenine fumarate (1:1); MW = 635.52 Da; aka Viread®, Gilead Sciences, Foster City, CA, USA). The anti-HIV drug can also be HIV-specific siRNA, anti-sense or sense DNA molecules.

In other embodiments, the active agent is an anticancer drug, an antibacterial drug, an antifungal drug, or a compound interfering with a parasitic protozoan.

In yet further embodiments, the active agent is an immunomodulatory agent (i.e., an immunoactivator, an immunogen, an immunosuppressant, or an antiinflammatory agent), such as cyclosporin, steroids and steroid derivatives. Other examples of useful drugs, in accordance with the invention, include therapeutic cytotoxic agents (e.g., cisplatin, carboplatin, methotrexate, 5-fluorouracil, and amphotericin), naked DNA expression vectors, therapeutic proteins, therapeutic oligonucleotides or nucleotide analogs, interferons, cytokines, or cytokine agonists or antagonists. Also useful as a drug is a cytotoxic alkylating agent, such as, but not limited to, busulfan (1,4-butanediol dimethanesulphonate; Myleran, Glaxo Wellcome), chlorambucil; cyclophosphamide, melphalan, or ethyl ethanesulfonic acid. Such drugs or agents are particularly useful in treating conditions involving pathological proliferation of immune cells, for example, lymphoid cancers or autoimmune diseases.

Combinations of active agents, whether plant lectins and/or drugs are also encompassed within the invention. In embodiments of the invention, active agents may be encapsulated within the lipid-active agent complex.

### EXAMPLES

### Example 1. Materials and Methods

Preparation and characterization of liposomes. Dioleoylphosphatidylcholine (DOPC) was purchased from Lipoid (Ludwigshafen, Germany) and cholesterol (Chol) was from Caelo (Caesar and Lorentz, Hilden, Germany). Sugar-cholesterol derivatives were synthesized as described below. Lipid-saccharide derivative mixtures (i.e. DOPC:Chol:Gal-C₄-Chol, DOPC:Chol:Man-C₄-Chol or DOPC:Chol:Gal-C₄-Chol, each at 60:35:5 molar ratios; or DOPC:Chol as a negative control, at a 60:40 molar ratio) were dissolved in dichloromethane/methanol (1:2 v/v). The solvent was completely removed under reduced pressure at 35°C, followed by evaporation at high vacuum.

For fluorescence analyses, a solution of 50 mM calcein (Sigma, St. Louis, USA) and 1 mM EDTA (Merck, Darmstadt, Germany) was prepared and the pH was adjusted to 7.5 with NaOH. The dried lipid film was hydrated with 1 ml of the calcein solution by gentle mixing to yield a dispersion of approximately 30 mM total lipid. The dispersion was freeze-thawed 6 times and then extruded 11 times through a polycarbonate membrane with 200 nm pores and subsequently 21 times through a membrane 80 nm pores (both membranes purchased from Whatman, Maidstone, Kent, UK), using the LiposoFast device (Avestin, Ottawa, Canada). Non-encapsulated calcein was completely removed by gel chromatography utilizing a Sepharose CL-4B column

(Pharmacia Biotech, Uppsala, Sweden) equilibrated with isotonic HEPES buffer (130 mM NaCl, 10 mM HEPES, 1 mM EDTA, pH 7.4).

The final phospholipid concentration was determined by a long-established highly reliable phosphorus assay (Barttlett GR. Phosphorus assay in column chromatography. J Biol Chem 1959, 234: 466-8). Liposome size was measured by photon correlation spectroscopy (Zeta-Master S, Malvern Instruments, Malvern, UK). Samples were diluted with HEPES buffer freshly filtered particle-free (0.22 µm Minisart, Göttingen, Germany) to yield a counting rate of 100-150 kcounts/s. All samples were placed into the sample holder 5 min before onset of measurement, so as to equilibrate the sample to 25°C.

Synthesis of Cholesterol-Glycosyl Derivatives. The synthesis of three differently glycosylated cholesterol derivatives principally followed the synthetic pathway published by Hashida and co-workers *[*Kawakami S, Yamashita F, Nishikawa M, Takakura Y, Hashida M Asialoglcoprotein receptor-mediated gene transfer using novel galactosylated liposomes. Biochem Biophys Res Commun 1998;252:78-83*;* Nishikawa M, Kawakami S, Yamashita F, Hashida M. Glycosylated cationic liposomes for carbohydrate receptor-mediated gene transfer. Meth Enzymol 2003;373:384-399*],* with some modifications. Protocols for obtaining cholesterol derivatized either with (i) *fucose,* i.e., cholesten-5-yloxyl-*N*-(4-((1-imino-c-β-D-thiofucosylethyl)amino)butyl) formamide; (ii) mannose, i.e., cholesten-5-yloxyl-*N*-(4-((1-imino-c-β-D-thiomannosylethyl)amino)butyl)formamide; or (iii) galactose, i.e., cholesten-5-yloxyl-*N*-(4-((1-imino-c-β-D-thiogalactoctosylethyl)amino)butyl)formamide - as detailed below for the fucosyl derivative - closely resembled one another. Throughout synthesis, the structures of all isolated intermediates were confirmed by nuclear magnetic resonance (NMR) spectroscopy.

Step 1: Acetylation of fucose / Synthesis of 1,2,3,4-tetra-O-acetyl-fucopyranoside: According to [Nishikawa M, Kawakami S, Yamashita F, Hashida M. Glycosylated cationic liposomes for carbohydrate receptor-mediated gene transfer. Meth Enzymol 2003;373:384-399; and Wolfrom ML, Thompson A. Acetylation. Methods in Carbohydrate Chemistry. Vol. II; pp. 211-5 (1963)], 152 mmol of fucose was added stepwise under continuous agitation to a mixture of 175 ml acetic anhydride and 1.25 ml perchloric acid pre-cooled on ice. The solution was then stirred at RT for a further 3 h under the exclusion of air humidity. This reaction mixture was poured onto 200 ml of iced water, before adding 300 ml of ethyl acetate. After phase separation in a funnel, the collected organic layer was washed x3 with 100 ml of cold water and dried over anhydrous magnesium sulphate. Filtration and evaporation of the solvent resulted in 1,2,3,4-tetra-O-acetyl-fucopyranoside as an oil.

Step 2: Synthesis of 1-bromo-2,3,4-tri-O-acetyl-fucopyranoside: 47.6 mmol of 1,2,3,4-tetra-*O*-acetyl-fucopyranoside were added slowly (step wise) to 75 ml of a hydrogen bromide/acetic acid solution (33%) while stirring in an ice bath; this mixture was further stirred overnight at 4°C. (In contrast, the 1-bromo-2,3,4-tri-*O-*acetly-galactopyranoside was purified adding 400 ml, each, of ice water and ethyl acetate). After phase separation, the organic layer was collected and the aqueous layer was washed ×3 with 100 ml of ethyl acetate. Combined ethyl acetate fractions were subsequently neutralized by adding 200 ml of a saturated sodium bicarbonate solution. The neutralized organic phase was then washed with 200 ml of a 1% sodium chloride solution, resulting layers were separated, and the organic layer was dried with magnesium sulphate, filtered, and evaporated to give 2,3,4-tri-*O*-acetyl-fucopyranosylbromide.

Step 3: Synthesis of 2-S-(2,3,4-tri-*O*-acetyl-thiofucopyranosyl)-2-thiopseudourea hydrobromide: According to protocols developed by Chipowsky and Lee [Chipowsky Y, Lee YC. Synthesis of 1-thioaldosides having an amino group at the aglycon terminal. Carbohyd Res 1973;31:339-346*],* 45.17 mmol of dried 1-bromo-2,3,4-tri-*O*-acetyl-fucopyranoside was dissolved in 20 ml of dry acetone. The solution was transferred to a 250-ml round-bottomed flask, and 50 mmol of thiourea were added under an argon atmosphere. The reaction mixture was refluxed for 15 min and then allowed to cool off in an ice bath. Thus precipitated 2-S-(2,3,4-tri-*O*-acetyl-thiofucopyranosyl)-2-thiopseudourea hydrobromide was collected by filtration.

Step 4: Synthesis of Cyanomethyl -2,3,4-tri-*O*-acetyl-thiofucopyranoside: Cyanomethyl-2,3,4-tri-*O*-acetyl-thiofucopyranoside was synthesized according *[*Nishikawa M, Kawakami S, Yamashita F, Hashida M. Glycosylated cationic liposomes for carbohydrate receptor-mediated gene transfer. Meth Enzymol 2003;373:384-399*; and* Lee YC, Stowell CP, Krantz ML. 2-Imino-2-methoxyethylen 1-thioglycosides: new reagents for attaching sugars to proteins. Biochemistry 1976;15:3956-3963*]:* under continuous agitation, 12.3 mmol of 2-S-(2,3,4-tri-O-acetyl-thiofucopyranosyl)-2-thiopseudourea hydrobromide were dissolved in 3.1 ml of chloroacetonitrile plus 24 ml of 1:1 (v/v) water/acetone. Upon obtaining a nearly clear solution, 14.3 mmol potassium carbonate and 24.9 mmol sodium bisulfate were added, and the reaction mixture was stirred for 30 min at RT. For purification, this mixture was evaporated, and the residue was dissolved in ethyl acetate. The product, cyanomethyl-2,3,4-tri-*O*-aceiyl-thiofucopyranoside, was subsequently purified by a new procedure developed by Schwarz and Streicher. Briefly, after employing flash chromatography (at 1:1 eluent:ethyl acetate/toluol), fractions containing the product were combined, evaporated, and kept at 4°C overnight to allow for crystallization.

Step 5: Deacetylation / Synthesis of 2-imino-2-methoxyethyl-1-thiofucopyranoside (IME-thiofucoside): Deacetylation was performed according to *[*Kawakami S, Yamashita F, Nishikawa M, Takakura Y, Hashida M. Asialoglcoprotein receptor-mediated gene transfer using novel galactosylated liposomes. Biochem Biophys Res Commun 1998;252:78-83; *and* Lee YC, Stowell CP, Krantz ML. 2-Imino-2-methoxyethylen 1-thioglycosides: new reagents for attaching sugars to proteins. Biochemistry 1976;15:3956-3963], i.e. 5 mmol of cyanomethyl-2,3,4-tri-*O*-acetyl-thiofucopyranoside were dissolved in 18 ml of methanol and 2 ml of 0.1 M sodium methoxide and kept at RT overnight. After methanol evaporation, the resulting IME-thiofucoside-containing syrup was used for coupling this intermediate to *N*-(4-aminobutyl)-(cholesten-5-yloxyl)formamide (see Step 6, Part 2).

Step 6: Synthesis of Cholesten-5-yloxyl-*N*-(4-((1-imino-c-β-D-thiofucosylethyl)amino)butyl) formamide: This step was performed according to *[*Nishikawa M, Kawakami S, Yamashita F, Hashida M Glycosylated cationic liposomes for carbohydrate receptor-mediated gene transfer. Meth Enzymol 2003;373:384-399]. *Part 1: N*-(4-aminobutyl)-(cholesten-5-yloxyl)formamide was prepared by combining 5.22 mmol *N*-Boc-1,4-butanediamine and 4.75 mmol cholesteryl chloroformate in 95 ml of chloroform and stirring for 24 h at RT. A solution of 9.5 ml trifluoroacetic acid in 24 ml chloroform and was added dropwise, and the reaction mixture was kept at 4°C for 4 h while stirring. Evaporation of the chloroform, and removal of the trifluoroacetic acid by co-evaporation with 100 ml of toluene, gave *N*-(4-aminobutyl)-(cholesten-5-yloxyl)formamide, which was crystallized by adding 15 ml of *n*-pentane. *Part* 2: Next, 2.5 mmol of the *fucose* derivative IME-thiofucoside were dissolved in 20 ml of dry pyridine and added 153 ml of triethylamine. The final reaction mixture was obtained by subsequently adding 1 mmol of the *cholesterol* derivative *N*-(4-aminobutyl)-(cholesten-5-yloxyl)formamide and kept at RT for 24 h. Following evaporation of the solvent and co-evaporation with toluene, the material was suspended in 30 ml of distilled water, dialyzed against water in a dialysis tube with a 12-kDa cut-off (2 days, 4°C), lyophilized, and finally washed with diethylether. The resulting substance, cholesten-5-yloxlyl-*N*-(4-((1-imino-c-β-D-thiofucosylethyl)amino)butyl) formamide, is further referred to as Fuc-C4-Chol. Accordingly, the other sugar derivatives synthesized are referred to as Man-C4-Chol and Gal-C4-Chol.

Lectin Encapsulation into Targeted Liposomes. To encapsulate horseradish peroxidase (HRPO)-conjugated concanavalin-A (Con-A), i.e., Con-A×HRPO (Sigma, St. Louis, MI, USA), we employed a modified protocol according to Gerber et. al. [Gerber CE, Bruchelt G, Falk UB, Kimpfler A, Hauschild O, Kuci S, Bachi T, Niethammer D, Schubert R. Reconstitution of bactericidal activity in chronic granulomatous disease cells by glucose-oxidase-containing liposomes. Blood 2001;98:3097-3105]. Briefly, a lipid mixture of (DOPC:Chol:Fuc-C4-Chol), at a 70:25:5 molar ratio, was dissolved in dichlormethane/methanol (1:2 v/v). The organic solvent was removed, and the lipid was dried in a vacuum. For encapsulating Con-A×HRPO into liposomes, the lipid was dispersed as a film in 1 ml PBS (pH 6.3) containing 0.5 mg ConA×HRPO. This dispersion was freeze-thawed ×5 and then extruded ×11 through a 0.4-mn polycarbonate membrane and, subsequently, ×21 through a 0.2-nm membrane using the LiposoFast device (Avestin, Ottawa, Canada). After this procedure, it cannot be excluded that Con-A×HRPO molecules are embedded within the liposomes' membranes or adhere to their outer surface. In such a case, lectin might interact with a target cells' surface sugars, which is unfavorable. Moreover, the size of Con-A×HRPO conjugates (at molecular weights of 102 kDa for Con-A and 44 kDa for HRPO) exceeds that of the fucosyl residues (MW 102 Da) that are supposed to act as the actual targeting mediators by a factor of almost x1500. Therefore, a few exoliposomal Con-A×HRPO molecules could completely invalidate the targeting mechanism. For these reasons, the lectin-loaded system was trypsinized for 5 min after completing the encapsulation procedure. Finally, all liposome preparations (Tab. 1) were purified by gel chromatography on Sepharose 4B-CL columns (Pharmacia Biotech, Uppsala, Sweden) and kept at 4°C in the dark until used.

Cellular binding/uptake studies. Mature cells were harvested on day 7 of culture by pelleting non-adherent veiled cells from the supernatants and detaching weakly adherent cells with 1% EDTA in PBS for 30 min at 4°C; strongly adherent cells were obtained by gently applying a cell scraper (TPP). All fractions were pooled, washed with PBS and kept in medium 80/20 plus 1% FBS on ice until used. For testing, cells were plated in fresh culture medium with 1 % FBS at a density of 2 x 10⁵ cells/well. To obtain the time-dependency of the targeting to dendritic cells, the 2 x 10⁵ MoDCs per microwell onset (96-well microtiter plates at 200 µl/well) in the same medium were incubated with liposomes at 50 µM lipid at 37 °C for 1, 3, or 24 hours, or other times and temperatures, as described herein below. After incubation, the cells were washed three times with phosphate-buffered saline (PBS, pH 7.2; without bivalent cations) and analyzed by flow cytometry.

Flow cytometry. Flow cytometry can be employed (i) to determine the phenotype of myeloid dendritic cells (further referred to as mDCs or DCs) at different times throughout DC differentiation with or without the mDCs being infected with select M- or T-tropic strains of HIV-1, and/or treated with the fucose-targeted delivery system or control liposomes; and (ii) to determine co-delivery of calcein/drug(s) to non-infected or infected mDC. Labeled mDCs were analyzed on a Coulter Epics XL-MCL (Beckman Coulter, Fullerton, CA) flow cytometer according to the manufacturer's instructions, immediately after (i) indirect staining with primary mAbs and secondary polyclonal IgG conjugated with fluorescein-5-isothiocyanate (FITC) (*e*Bioscience) (Gieseler R et al., In-vitro differentiation of mature dendritic cells from human blood monocytes, Dev Immunol 6:25-39 [1998]); (ii) incubation with the respective calcein-containing liposomal preparation; or (iii) incubation with negative controls for specific antibody stains or liposomal targeting studies. When flow cytometry was performed; only gated cells were evaluated for antigen expression, as well as for liposomal targeting and uptake studies. Briefly, cells were gated *via* forward and side scatter dot plotting to exclude debris. Histograms were established for gated cells, as commonly suitable for FITC and calcein, i.e. at excitation and emission wavelengths of λ_{EX} = 488 nm or λ_{EM} = 525 nm, respectively. Data were downloaded, and the corresponding histograms for test samples and controls were overlaid and analyzed with WinMDI 2.8 software (J. Trotter; facs.scripps.edu). Targeting efficacy was determined directly after incubating mDCs (or, when employed, macrophages generated for 7 days in Medium 80/20 supplemented with 10% autologous donor serum or 10% FBS, with similar results) with the respective targeted liposome construct, or with liposomal negative controls, or after employing the irrelevant isotype control IgG antibody MOPC-21/P3. Results of negative controls employing liposomes whose surface was deliberately kept devoid of carbohydrate labeling were identical to those obtained with irrelevant control IgG. An influence *via* nonspecific uptake of liposomes by mDCs could thus be excluded. For flow cytometric analyses, immature mDCs were harvested on day 5 or day 7. Mature non-adherent and adherent DCs were harvested on day 7 or day 8. Macrophages were also harvested on day 7 or day 8.

Peripheral blood leukocytes (PBL). Mononuclear leukocytes (MNLs) were prepared as described before (Gieseler, R. et al., In-vitro differentiation of mature dendritic cells from human blood monocytes, Dev. Immunol. 6:25-39 [1998]). Briefly, MNLs were enriched from whole blood diluted 1:1 with phosphate-buffered saline (PBS) without Ca²⁺/Mg²⁺ (Cambrex, Walkersville, MD, USA) by density gradient centrifugation over Lymphoprep (ρ = 1.077 g/cm³; Nyegaard, Oslo, Norway). Buffy coats were harvested and pooled, and residual platelets were removed by 3-4 washes with PBS. These procedures involved several 10-min centrifugation steps at 260 g and 4°C.

Magnetic-activated cell separation (MACS) of monocytes. Monocytes were isolated *via* negative magnetic-activated cell separation (MACS; Miltenyi, Bergisch-Gladbach, Germany) by removing CD3⁺, CD7⁺, CD19⁺, CD45RA⁺, CD56⁺ and mIgE⁺ cells with mAb-coated magnetic microbeads. Negative monocyte separation, which had been chosen to avoid an undesirable activation of freshly isolated monocytes, was performed according to the manufacturer's instructions. Briefly, the procedure involved 2 washes with PBS supplemented with 0.5% bovine serum albumin (BSA; cell-culture grade, < 0.1 ng/mg endotoxin; ICN, Irvine, CA, USA) and 2 mM EDTA (Sigma, St. Louis, MO, USA), and the washed cells were passed through an LS magnetic microcolumn placed in a defined magnetic field (Miltenyi), thus enriching the monocytes to 98.6-99.9% purity (range of n = 3), as determined by flow cytometry for CD14.

Differentiation of myeloid dendritic cells. Mature and immature mDCs were generated from peripheral blood monocytes. Briefly, monocytes were isolated by successive density gradient centrifugation of PBS-diluted whole blood over Lymphoprep (ρ = 1.077 g/cm³) (Nyegaard, Oslo, Norway) and, successively, by negative magnetic cell separation (MACS), in accordance with the manufacturers' instructions (Miltenyi). Monocytes were then seeded at 1 x 10⁵/200 µl in 96-well microtiter plates (TPP, Trasadingen, Switzerland). According to a generally accepted protocol earlier established in our hands, we employed granulocyte/macrophage colony-stimulating factor (GM-CSF) and interleukin 4 (IL-4) on day 0 as basic DC differentiation factors, thus leading to an immature, antigen-capturing mDC stage (Peters JH, Xu H, Ruppert J, Ostermeier D, Friedrichs D & Gieseler RK. Signals required for differentiating dendritic cells from human monocytes in vitro. Adv Exp Med Biol; 329:275-80 [1993]; Ruppert J, Schütt C, Ostermeier D & Peters JH. Down-regulation and release of CD14 on human monocytes by IL-4 depends on the presence of serum or GM-CSF. Adv Exp Med Biol; 329:281-6 [1993]). Successively, if desired, mature mDCs were obtained by further adding tumor-necrosis factor (TNF)-α on day 5 or 6, thus leading to mDCs able to initiate both T-helper (Th)1- and Th2-dependent immunity (Caux C, Dezutter-Dambuyant C, Schmitt D & Banchereau J. GM-CSF and TNF-α cooperate in the generation of dendritic Langerhans cells. Nature; 360:258-61 [1992]; Sallusto F & Lanzavecchia A. Efficient presentation of soluble antigen by cultured human dendritic cells is maintained by granulocyte/macrophage colony-stimulating factor plus interleukin 4 and downregulated by tumor necrosis factor alpha. J Exp Med; 179:1109-18 [1994]; Banchereau J & Steinman RM. Dendritic cells and the control of immunity. Nature; 392:245-52 [1998]).

DC harvesting and liposome incubation. Harvested mDCs and liposome preparations were incubated at differing relative concentrations (depending on the experimental context) for 3 hours at room temperature. Immature non-adherent and adherent mDCs were harvested on day 7 or 8. Mature non-adherent and adherent mDCs were harvested on day 7 or 8. To this end, the differentiation medium was collected, centrifuged, and the pelleted DC fraction of non-adherent veiled cells was harvested. Second, adherent DCs were detached from the wells by incubating them with PBS/EDTA for 30 min at 4°C, and by successively employing a cell scraper (also referred to as a "rubber policeman"). Detached adherent mDCs were pooled with the non-adherent fraction, and the combination of both was adjusted to the cell numbers required and thereafter incubated with liposomes or irrelevant control antibody.

As described above, mDCs were analyzed flow-cytrometically for expression of CD1a, CD4, CD14, CD40, CD45RA, CD45R0, CD68, CD69, CD83, CD184, CD195, CD206 (mannose receptor), CD207, CD208, and/or CD209 (DC-SIGN), with mouse anti-human IgG1κ (clone MOPC-21/P3) as a negative control (available form Serotec, Oxford, UK). Depending on whether only one or two mAbs were employed (aka direct staining *vs*. indirect staining), antigens were either stained directly with FITC-conjugated marker-specific monoclonal antibodies (mAbs), or were stained indirectly with unlabeled first mAbs plus secondary polyclonal IgG x FITC (available from *e*Bioscience).

The MOPC-21/P3 immunoglobulin was employed as the IgG1κ isotype control. Results obtained with this antibody served three purposes, i.e. (i) to verify that the cells differentiated *in vitro* exhibited genuine DC phenotypes; (ii) to define their phenotypic and interindividual differences; and (iii) to compare the expression of a given marker with the histogram pattern displayed after incubation with liposomes targeted by the same antibody.

For cell targeting, aliquots of mDC suspensions of at least 5 x 10⁴ DCs (or, when employed, macrophages) were incubated with liposomes, at 0.1, 1.0, or 10.0 µl per onset, for 2 or 3 hours at 37°C under continuous agitation in an incubator, and then examined by flow cytometry. Reliable and reproducible results were obtained by 2-h co-incubation; excellent targeting and compound-delivery results were obtained upon 3-h co-incubation).

HIV strains. HIV-1 strains were obtained from the NIH Repository (Rockville Pike, Bethesda, MD), i.e., *M-tropic* (aka CXCR5- or R5-tropic) HIV-1 Ada-M and *T-tropic* (aka CCR4- or X4-tropic) HIV-1 Lai. HIV strains were propagated as given in **Tab. 2** and tested for their tissue-culture 50% infective dosage (TCID₅₀) according to protocols known to the art. Referring to the TCID₅₀ results, viral supernatants were appropriately pre-diluted for their subsequent employment, aliquoted, and frozen at - 70°C until employed for infection at dosages specified in the legend to **Fig. 11****.**

ELISA for HIV p24 core antigen. Supernatants can be tested for presence of p24 according to the manufacturer's instructions by a commercially available ELISA (Abbott Laboratories, Chicago, IL, USA).

Quantitative polymerase chain reaction (qPCR) for HIV. The degree of integration of HIV proviral DNA into dendritic-cell host DNA can be determined by using nested primer pairs (nested semi-qPCR) for HIV proviral sequences, such as the following:

| | |
|---|---|
| Outer Primers: | 5'-agt-ggg-ggg-aca-tca-agc-agc-cat-gca-aat-3' //(SEQ ID NO:1) |
| | 5'-tca-tct-ggc-ctg-gtg-caa-3' //(SEQ ID NO:2) |
| Inner Primers: ID NO:3) | 5'-cag-ctt-aga-gac-cat-caa-tga-gga-agc-5g-3' (5-FAM) //(SEQ |
| (this is a LUX-primer, labeled with 5-carboxyfluorescein, i.e., "5" = 5-FAM) | |
| | 5'-ggt-gca-ata-ggc-cct-gca-t-3' //(SEQ ID NO:4). |

Isolation of DNA can be accomplished according to manufacturer's instructions ("Easy-DNA-Kit", in protocol #3 "Small Amounts of Cells, Tissues, or Plant Leaves", Invitrogen). The PCR reaction mixture typically includes the following: Buffer (5 µl of 10X PCR Rxn Buffer, Invitrogen); MgCl₂ (3 µl of 50 mM MgCl₂, Invitrogen); dNTP (1 µl of mixture of dATP, dCTP, dGTP, dTTP: 10 µM, each); Outer Primer (SEQ ID NO:1; 1 µl of 10 pmol/µl); Outer Primer (SEQ ID NO:2; 1 µl of 10 pmol/µl); Taq (0.2 µl of 5 Units/µl, Platinum Taq DNA Polymerase, Invitrogen); double distilled water (37 µl); DNA sample (2 µl). One standard thermal cycling profile was the following: 5 min at 95°C; (20 s at 95°C; 30 s at 55°C; 30 s at 72°C) x 25; 2 min at 72°C; hold at 4°C. PCR is generally repeated using two microliters of amplified DNA transferred from the first reaction in fresh PCR reaction mixture, except using the inner primers (SEQ ID NO:3 and SEQ ID NO:4) instead of the outer primers, and employing a different thermal cycling profile: 5 min at 95°C; (20 s at 95°C; 30 s at 55°C; 30 s at 72°C) x 35; 2 min at 72°C (melting curve 95°C down to 55°C in steps of 0.5 °C).

In a given sample, DNA quantification can be achieved by comparison with a serial dilution of a DNA sample of known quantity of HIV proviral DNA. To allow quantifying HIV proviral DNA from samples with different contents of total cellular DNA (e.g., from dendritic cells), a Multiplex-PCR can be performed. Briefly, a second nested PCR can be performed in the same reaction, with a LUX primer labeled with 6-carboxy-4',5'-dichloro-2',7'-dimethoxyfluorescein succinimidyl ester, for a human chromosome sequence (genome equivalent). This permits quantification of the total DNA content per sample. Numbers of proviral copies per human genome equivalent can be calculated from such data.

### Example 2. Phenotyping of myeloid dendritic cells

Peripheral blood mononuclear cells (PBMNCs) were evaluated according to their size (forward scatter) and granularity (side scatter) and thus were gated as naive T and B cells; activated T-cells and B-cells; and monocytes, including a small proportion of blood dendritic cells (data not shown). Cultured monocyte-derived myeloid dendritic cells (mDCs or DCs) were tested for expression of markers delineating their developmental stage (maturity), as well as for mDC subtype markers. The DCs expressed markers typical for skin and mucosal DC phenotypes that are considered to play a key role in HIV infection. When being infected *via* the mucosal route, mucosal mDCs are the first immune cell type to be directly infected by HIV and (i) occasionally integrate its genetic information as proviral DNA and/or (ii) fixate HIV on their surface by DC-SIGN and/or (iii) take up HIV by any of various mechanisms to retain the virus in intracytoplasmic compartments (e.g., endosomes). Such cells then migrate to regional and local lymph nodes where passing on HIV to other cell types, most prominently T-helper cells (aka "CD4 cells") as well as other reservoir cells, including the next generation of lymph node-settling mDCs in the course of continuous mDC turnover. In light of these facts, the mDCs generated in our *in-vitro* system provide an ideal model for evaluating the presumptive *in-vivo* targeting efficacy to such cells.

MDCs matured by 7-day culture with GM-CSF, IL-4 and subsequent TNF-α were tested by flow cytometry for expression of markers known to be expressed by mDCs or subpopulations thereof. Apart from DC-SIGN (CD-209), we chose markers delineating mature DCs *in vitro* and *in vivo* (CD40, CD45R0, CD83), as well as dendritic Langerhans cells of the epidermis (CD1a) and the intestinal (CD4) and nasal mucosa (CD14). Phenotyping thus served (i) for verifying mDCs generated *in vitro* as immature or mature; (ii) for proving strong expression of DC-SIGN (CD209) and the mannose receptor (CD206) as pre-conceived targets for immunoliposomal compound delivery to mDCs; and (iii) for determining whether the generated MoDCs expressed CD1a as a hallmark of epidermal and mucosal Langerhans cells *in vivo.*

Relative mean fluorescence intensities (ΔMFI) of test conditions vs. negative controls (n = 3) characterized the phenotypic profile of mature MoDCs as CD1a⁺⁺⁺, CD4^{±}, CD14^{± to +++}, CD40^{++ to +++}, CD45R0^{+ to +++}, CD83⁺ and CD209⁺⁺⁺ [with: (-), test antibody congruent with negative control; (±), ΔMFI peak ≤×5 above negative control; (+), ΔMFI peak ≤×10 above negative control; and (+++), ΔMFI peak × ≥ 250 negative control]. Of all markers tested, expression of CD14 varied most considerably among the donors. In contrast, DC-SIGN (CD209) and the Langerhans-cell marker CD1a consistently revealed high expression in all donors examined.

### Example 3. Active targeting of mDCs with carbohydrate surface-labeled liposomes: fluorescence-microscopic uptake studies, flow-cytometric uptake and binding studies, and HIV inhibition studies

In initial control studies, cellular binding of Fuc-4C-Chol- as well as Man-4C-Chol-targeted liposomes was completely inhibited by adding 100 mM of free soluble L-fucose or D-mannose (positive control) to the cells before their incubation with liposomes. As expected, addition of D-galactose (negative control) was ineffective (all monosaccharides were purchased from Sigma, St. Louis, MI, USA). These controls (results not shown) demonstrated that the fucose- and mannose-labeled liposomes specifically bound to the envisioned exocellular targeting structures, i.e. C-type lectin receptors.

A member of the CTL family, termed dendritic cell-specific intercellular adhesion molecule (ICAM)-3-grabbing nonintegrin (DC-SIGN; CD209), was targeted on immature and mature monocyte-derived dendritic cells at 37°C. Highly efficient dendritic cell-specific binding, uptake, and intracellular delivery of encapsulated calcein were achieved when targeting DC-SIGN with immunoliposomes carrying DC-SIGN-specific antibodies. While intracellular delivery was largely confined to endosomes, calcein was also demonstrable in the cytoplasm, but not in the nucleus. Variants of immunoliposomes directed against other likely target molecules on the cells' surfaces (CD1a, CD4, CD14, CD45R0, CD83, and dual combinations thereof) also investigated revealed a superior net targeting efficiency for DC-SIGN-specific immunoliposomes. A membrane redundancy of bound DC-SIGN-specific liposomes of approximately 2 hours before uptake was also demonstrated (Gieseler RK, Marquitan G, Halm MJ, Perdon LA, Driessen WH, Sullivan SM, Scolaro MJ. DC-SIGN-specific liposomal targeting and selective intracellular compound delivery to human myeloid dendritic cells: implications for HIV disease. Scand J Immunol 2004;59:415-24).

Figure 1 shows the basic morphological appearance of human myeloid dendritic cells (mDCs) during differentiation in vitro. Phase contrast photomicrographs were taken on days 3 (a), 5 (b), 7 (c, d). (a) On day 3, upon induction of differentiation by GM-CSF and IL-4, oval-shaped mDCs start to grow out membrane veils and thin membrane projections (arrows) (original magnification x400). (b) On day 5, most immature mDCs have assumed a stretched morphology and express membrane projections (i.e., dendrites), although oval-shaped cells are still present. Even at this immature stage, some mDCs associate to form small homotypic clusters (arrows) (original magnification x180). (c) However, upon induction of mDC maturation by TNF-a on day 5, fully matured DCs generally associate in the form of large homotypic clusters on day 7. Strong clustering by de novo-expressed adhesion molecules indicates that mDCs have reached their full functional maturity. Note the abundant filiform projections pointing out of the cluster (arrows). When located in a lymphoid organ, such dendrites establish intimate contact with T cells for antigen-specific stimulation in heterotypic mDC-T-cell clusters (original magnification x180).

Although this series of events represents the differentiation course of mDCs in most healthy donors, monocytes obtained from a minor portion of donors respond differently to the same microenvironmental conditions. Specifically, probably due to genetic pre-disposition, cells from some donors express fewer dendrites and/or form smaller, but more numerous, clusters. An example is depicted in (d) at x50 magnification of a complete microtiter well. Also, in very rare cases, mDC differentiation completely fails and macrophages develop instead; this may be due to overriding priming signals acting on the monocytes in the respective donor, for example, in case of an ongoing infection. Importantly, our targeting studies on human myeloid dendritic cells were carried out on mDCs following the regular differentiation path observed in approximately 80-90% of the cases in the presence of GM-CSF/IL-4 and sequential TNF-a.

Figure 2 shows serial optical sections through immature myeloid dendritic cells (mDCs) targeted with fucose-labeled liposomes delivering the tracer dye calcein. Immature mDCs generated for 5 days with GM-CSF and IL-4 were detached from the substratum by EDTA and incubated for 3 h under continuous agitation at 37°C with Fuc-C4-Chol-targeted liposomes delivering the green fluorescent tracer dye calcein. Cells were counterstained with blue nuclear DAPI stain and fixated. (a-1) Fluorescence-microscopic overlays of serial sections (~1-mm steps) depict uptake of the system by two mDCs representing the lowest and highest uptake rates observed. In the mDC on the left, calcein was mainly confined to endosomes (e.g., c; arrow), with faint occasional cytoplasmic staining (e.g., overlaid to the nucleus in frame g; arrow). In contrast, the mDC on the right revealed bright staining of both endosomes (punctuate fluorescence, e.g., c, f) and cytoplasm (e.g., b). When comparing larger numbers of cells, all mDCs from all donors tested (n = 3) had internalized the fucose-targeted system. As apparent from the blue stain, liposome payload was never delivered into the nucleus. Man-C4-Chol-targeted positive controls were taken up less efficiently, and Gal-C4-Chol-targeted negative controls were not bound and/or internalized (not shown). Original magnification x400.

Figure 3 shows binding and uptake of mannose-labeled liposomes by immature mDCs after 5 days of culture. The extent of binding or uptake is depicted in two donors (A, upper row; B, lower row) by comparison of phase contrast (left column) and fluorescence (right column) photomicrographs. In both cases, and in contrast to fucose-labeled liposomes, only less than 50% of the cells revealed the tracer dye, calcein, within liposomes bound to their surface or internalized after 3-hour incubation. While some of tracer-positive mDCs showed intracellular uptake (B1, B2: upper and median circles), others still only revealed surface binding without uptake (A1, A2 and B1, B2: lower circles) after prolonged incubation. In immature mDCs, targeting by Man-C4-Chol-labeled liposomes thus not only reached far fewer mDCs than observed when employing the Fuc-C4-Chol-targeted delivery system (compare also flow cytometry), but the mannose-targeted system was also much less efficiently taken up by receptor-mediated endocytosis. However, mannose targeting was equally efficient in macrophages, probably due to these cells' higher expression of the mannose receptor (CD206) C-type lectin (not shown). Incubation with Gal-C4-Chol-labeled negative-control liposomes never led to surface binding or intracellular uptake (not shown). Arrows in phase contrast micrographs A1 and B1 point at cells that had died off during culture, as apparent by their blebbing surface membranes. Although an occasional dead cell revealed non-specific calcein staining (A1, A2: upper circles), non-specific binding of fucose-, mannose- or galactose-labeled liposomes to dead cells was generally not observed at this point in time (compare boxed cells exactly positioned with equidistant bars in B1 and B2).

Figure 4 shows C-type lectin-specific targeting of clustered mature mDCs. Homotypic clusters of mature mDCs after 7-day culture in the presence of GM-CSF, IL-4, and TNF-a usually are overall round in shape and can comprise several hundreds of cells (cf. Fig. 1). Clusters partially disintegrate upon processing of cultured cells before incubation with the targeting system. However, due to the tight binding of mature mDCs via adhesion molecules (e.g., ICAM-1, ICAM-3, LFA-1), fragments of such clusters remain physically intact. The large fluorescence photomicrograph shows such a fragment comprising several tightly associated mDCs after 3-hour incubation with Fuc-C4-Chol-labeled liposomes. With a thin blue outline marking the contour of this fragment, each individual cell is enumerated on its lower right-hand side in a clockwise manner spiraling inwards. All 17 mDCs counted display at least faint cytoplasmic staining by liposome-delivered calcein. In most of the cases, stained endosomes stand out by their bright, sometimes outshining, punctuate fluorescence. The tracer dye never stained the cells' nuclei (when visible), as indicated by arrows. Mature mDCs generally revealed a lower uptake after targeting than seen in immature mDCs (see Fig. 2 and flow-cytometric results). The fucose-targeted system thus reached all mature mDCs despite their tight physical association. The same can thus be expected for homotypically clustered mDCs, as well as for mDCs within heterotypic mDC-T-cell clusters in lymphoid organs and tissues in vivo. Original magnification x400. As depicted in the small insert, single mDCs from 7-day cultures more often showed intense uptake of fucose-targeted liposomes and calcein delivery. The typical irregular-shaped nucleus of the mDC outlined in red is completely spared from calcein delivery. Targeted liposomes likely bound to surface (blue outline) C-type lectin receptors are indicated by arrows. Original magnification x1000.

Importantly, a consistent portion of the immature mDCs depicted in Fig. 2 as well as the mature mDCs shown in Fig. 4 expressed the Langerhans-cell marker CD1a (see flow-cytometry). Such cells correspond to mucosal and epidermal mDC subsets first infected upon sexual transmission of HIV. Conversely, another portion of mDCs did not express CD1a (see flow cytometry), thus corresponding to other systemic and lymphoid mDC subsets. Finally, mature mDCs (Fig. 4) expressed the immunoglobulin superfamily marker CD83 consistently expressed by mature DCs located in the lymphoid organs. All these types of mDCs were successfully targeted for intracellular endosomal and cytoplasmic delivery of an encapsulated compound. Thus, these results strongly indicate that all peripheral and lymphoid mDC subsets can be targeted efficiently with a Fuc-4C-Chol-labeled system for intracellular delivery of a therapeutic compound.

Figure 5 shows binding and uptake of fucose-labeled liposomes by human macrophages after 7 days of culture. Before incubation, macrophages were detached from the substratum by EDTA/trypsin treatment and then kept under continuous agitation to prevent their firm re-attachment, so as to enable their subsequent transfer to slides. (a-h) Serial optical sections through a representative macrophage revealed, already after 2 hours of incubation with Fuc-C4-Chol-targeted liposomes, abundant endosome-confmed intracellular staining by the tracer dye, calcein, delivered by the targeting system. In contrast to myeloid dendritic cells, cytoplasmic staining (i.e., liposomal delivery) was much less apparent in macrophages. Man-C4-Chol-labeled liposomes had a comparable targeting efficiency (not shown). Incubation with the Gal-C4-Chol-labeled negative control only led to minor uptake by an occasional macrophage (not shown). In the case depicted in here, the cells were generated in the presence of 10% autologous donor serum. Original magnification x1000.

Figure 6 is a color fluorescence photomicrograph of a representative macrophage from a different donor 2 hours after targeting with fucose-labeled liposomes. In this case, macrophages were differentiated for 7 days in the presence of 10% xenogenic fetal bovine serum (FBS). Under such conditions, binding and uptake results were identical to those obtained with macrophages generated with autologous serum, including the results upon targeting with the positive (Man-C4-Chol) and negative (Gal-C4-Chol) control systems. FBS-dependent differentiation can thus be employed in vitro for macrophage targeting studies. Median section. Original magnification x1000.

Importantly, these results showed that the fucose-targeted liposomal delivery system was also efficiently internalized by macrophages representing a system of cells that, as in HIV disease, forms the major infectious cellular reservoir of the gastrointestinal tract and, perhaps, of the brain.

Figure 7 shows serial optical sections through a monocyte targeted with Fuc-4C-Chol-labeled liposomes delivering the tracer dye calcein. Freshly isolated peripheral-blood monocytes were incubated for 3 h under continuous agitation at 37°C with Fuc-C4-Chol-targeted liposomes delivering the green fluorescent tracer dye calcein. Cells were counterstained with the blue nuclear DAPI stain and fixated. (a-f) Fluorescence-microscopic green/blue overlays of serial sections (~1.5-mm steps) depict uptake of the system by a representative monocyte (note the typical nuclear shape). In monocytes, the intracellular distribution of calcein as the targeted system's payload was identical to that seen in mDCs. The fluorescent compound was concentrated in the cells' endosomes (as most apparent in frames c, d, and e; punctuate fluorescence), as well as, more diffusely, in the monocytes' cytoplasm (i.e., all of the serial micrographs), but never within their nuclei. Moreover, as found for mDCs, too, all monocytes from all donors tested (n = 3) had internalized the fucose-targeted system. Again, Man-C4-Chol-targeted positive controls were taken up less efficiently, and Gal-C4-Chol-targeted negative controls were not bound and/or internalized at all (not shown). Original magnification x400.

Importantly, these results show that, besides reaching myeloid dendritic cells and macrophages, fucose-mediated targeted delivery of a therapeutic compound can be achieved for monocytes, too. This conclusion may have a profound impact when considering that monocytes, as has been explained above, potentially are the earliest myeloid lineage-derived cell type to be recruited as an infectious reservoir for HIV and other infectious agents. In fact, the case that monocytes were so efficiently targeted by a C-type lectin-specific system highlights the importance of this pathway for the uptake of infectious agents and the subsequent formation of chronically infectious intracellular reservoirs in a plethora of physiological and pathological monocytic descendants.

Figure 8 shows that the fucose-targeted compound delivery system is highly specific and has an extremely high targeting efficacy. When employing both immature and mature myeloid dendritic cells as important reservoir populations for HIV and other infectious agents, fucose targeting was most efficient in immature mDCs. Binding or uptake of calcein-delivering carbohydrate-labeled liposomes is depicted as filled histograms overlaid with empty histograms of background staining with non-sugar-labeled liposomes that bind to cells nonspecifically. The binding efficacy of Gal-C4-Chol-labeled negative control liposomes never differed from nonspecific control liposomes, thus verifying the correct choice of galactose labeling as a negative control. In contrast, mannose and fucose-labeled liposomes showed different degrees of specific cellular surface targeting and/or uptake. When compared with the Man-C4-Chol positive control, the Fuc-C4-Chol-targeted system revealed far superior binding efficacy in immature mDCs. In both donors, on a logarithmic scale (abscissa), the targeting efficacy of fucose-labeled liposomes exceeded that of the positive control by one order of magnitude. Specific targeting of mature mDCs was donor-dependent, in that some individuals, such as donor A, produce mature mDCs that express only low levels of C-type lectins. Yet, most donors, e.g. donor B, reveal at least median membrane densities of such molecules (see also Fig. 10), so that their net sum expression allows for efficient targeting with a Fuc-4-Chol-labeled liposomal delivery system. Nevertheless, even low binding to mature mDCs in such individuals can be significantly increased by higher concentrations of this system (see Fig. 9).

Figure 9 shows that increased concentrations of fucose-labeled liposomes targets both immature and mature mDCs highly efficiently. Employing the same positive and negative controls (see legend to Fig. 8), immature and mature mDCs were incubated with different concentrations of the Fuc-C4-Chol-targeted system. This experiment was carried out with two donors (C and D) in which a low concentration of the targeting system (lower row) efficiently reached immature, but not mature mDCs. However, when increasing the system's concentration by factors of x10 or x100, respectively (medium and upper rows), immature DCs were targeted highly efficiently. The medium concentration was applied in the experiments depicted in Fig. 2 and Fig. 4 Arrows in the medium row (donor C) indicate approximated positions of the two cells shown in Fig 2 that represent the cellular spectrum of binding-and-uptake efficacy of the Fuc-4C-Chol targeting system under this condition. Taken together, both cells expressing high and low surface membrane densities of C-type lectin receptors can be addressed successfully with our targeted compound delivery system.

Figure 10 depicts phenotyping of immature and mature myeloid dendritic cells. Marker-positive cells are depicted as filled histograms and overlaid with empty histograms indicating background staining with negative irrelevant control antibody. Gray areas left of the negative-control cutoff reflect the portion of cells not expressing a given marker; gray areas right of the cutoff express the marker (as exemplarily shown in the graph showing CD1a expression in immature mDCs from donor A). Abscissas indicate logarithmic fluorescence intensities of cell labeling with FITC-conjugated secondary antibodies after adding primary monoclonal antibodies recognizing the respective marker. DC-SIGN and the mannose receptor as typical representatives of C-type lectins expressed by mDCs are both expressed more pronounced in immature than in mature mDCs. Individual variances are apparent. In vivo, immature DCs reside in the peripheral nonlymphoid organs and tissues. Here, strong expression of such surface molecules ensures the cells' capability to bind and ingest many pathogens. Once migrated to the lymphoid organs and tissues, matured mDCs downregulate C-type lectin expression, but usually retain medium membrane densities of these targeting markers. Notably, as far as currently known, mDCs generally express at least four different surface C-type lectins (DC-SIGN, DEC-205, MR and DLEC), so that the net sum expression of such molecules always allows for efficient targeting with a fucose-labeled liposomal delivery system. Similarly, macrophages can be targeted in all their developmental stages, as they reveal consistently high expression of the mannose receptor (not shown). These cells can also be induced to express other C-type lectins such as DC-SIGN. Expression of CD83 indicates the mature status of mDCs. In vivo, expression of CD1a is indicative of Langerhans-cell mDC subsets (thus also expressing Langerin as a fifth C-type lectin) located in the mucosae and epidermis. Note that both immature and mature mDCs, at all times, comprised a spectrum of CDla-negative to strongly CD1a-positive cells, thus covering a corresponding spectrum of non-Langerhans to Langerhans cell-like mDCs. Fuc-C4-Chol-targeted liposomes successfully delivered calcein intracellularly to all these subtypes (see Figs. 2, 4), thus indicating their high potential as a system for delivering therapeutic compound(s) to endosomal and intracytoplasmatic sites.

Figure 11 shows the morphological changes in mDCs after 8-day culture of HIV-infected mDCs upon or without targeted treatment. I. Culture appearance and homotypic mDC clustering. Cells were differentiated in the presence of GM-CF/IL-4 (day 0) and sequential TNF-a (day 5). On days 2, 4, or 6, the mDCs were infected with the M-tropic HIV-1 strain, Ada-M, or the T-tropic HIV-strain, Lai, respectively. Tissue culture infective doses for 50% of the cells were I. HIV-1 Ada-M: 67 x TCID50 (i.e., 1 ml virus stock solution + 199 ml culture medium); and II. HIV-1 LAI: 6.7 x TCID50 (i.e., 0.1 ml virus stock solution + 199.9 ml culture medium. Results were obtained by scanning all areas of four separate culture wells for each situation. Homotypic mDC clustering as a criterion indicating the functional integrity of these cells was evaluated on day 8; results are given as semi-quantitative and absolute (rounded) values. One day after infection with the respective strain, mDCs were treated with concanavalin-A (Con-A)-delivering Fuc-4C-Chol-targeted liposomes. This time delay allowed the cells to form intracellular HIV reservoirs. As apparent, in both types of HIV-1 infection, and under all conditions tested, the clustering behavior was normalized. As homotypic and heterotypic mDC clustering is upregulated by the HIV upon infection (Sol-Foulon N, Moris A, Nobile C, Boccaccio C, Engering A, Abastado JP, Heard JM, van Kooyk Y, Schwartz O. HIV-1 Nef-induced upregulation of DC-SIGN in dendritic cells promotes lymphocyte clustering and viral spread. Immunity 2002;16:145-55), these results indirectly demonstrate the successful elimination of HIV (see also Fig. 12).

Figure 12 shows the morphological changes in mDCs after 8-day culture of HIV-infected mDCs upon or without targeted treatment. II. Types of mDCs and viability. All conditions for generating mDCs, infection with HIV-1, targeted treatment are as given in the legend to Fig. 11. Results were obtained by scanning all areas of four separate culture wells for each situation. The increased death rate of mDCs upon infection with HIV-1 was normalized upon treatment with Con-A-delivering fucose-targeted liposomes. Note that the washing procedure after liposomal treatment for 3 hours removed the dead cells accumulated after infection of the mDCs. Cultures, thus, sometimes comprise significantly reduced cell numbers when compared to uninfected cultures at the same given point in time, which, via lower concentrations of autocrine self-conditioning signals, may take effect on the relative ratio of mDC morphologies. Nevertheless, the relative shift between veiled-cell and dendritiform mDC types upon HIV infection was largely normalized after treatment. These results again indirectly demonstrate the successful elimination of HIV.

**Table 1. The Fuc-4C-Chol Targeting System and Control Preparations.**

| | | Liposomal Surface Labeling | | | | Intraliposomal Payload | | |
|---|---|---|---|---|---|---|---|---|
| | Conditions | Fuc | Man | Gal | None | Con-A | Calcein | None |
| **A** | **Fucose-dependent Cell Targeting** | | | | | | | |
| | Complete-Targeting System | + | - | - | - | + | - | - |
| | Tracing for uptake and localization | + | - | - | - | - | + | - |
| | Lectin Negative Control | + | - | - | - | - | - | + |
| | | | | | | | | |
| **B** | **Mannose-dependent Cell Targeting** | | | | | | | |
| | Surface-Targeting Positive Control | - | + | - | - | + | - | - |
| | Tracing for uptake and localization | - | + | - | - | - | + | - |
| | Internal Lectin Negative Control | - | + | - | - | - | - | + |
| | | | | | | | | |
| **C** | **Galactos-dependent Cell Targeting** | | | | | | | |
| | Surface-Targeting Negative Control | - | - | + | - | + | - | - |
| | Tracing for uptake and localization | - | - | + | - | - | + | - |
| | Internal Lectin Negative Control | - | - | + | - | - | - | + |
| | | | | | | | | |
| **D** | **No Surface Sugar Present** | | | | | | | |
| | Control for non-specific uptake | - | - | - | + | + | - | - |
| | Tracing for uptake and localization | - | - | - | + | - | + | - |

**Table 2. Propagation of M- (RS-) and T-(X4-) HIV-1 strains.** HIV-1 Lai and HIV-1 Ada-M were obtained from the NIH Repository. Shown are the conditions for propagating highly infectious stocks of these strains. Such stocks were either generated in unseparated peripheral blood mononuclear cells (PBMNLs) or MACS-enriched monocytes (MO) for 14 days, both of which cultured in 75 cm² ("T75") flasks. In some cases, pooled cells of four donors were employed, thus creating mixed-leukocyte culture conditions for stimulating the generation, and thus increasing the supernatant concentrations, of cytokines for activating the cells and potentially integrated provirus. Propagation of T-tropic HIV-1 Lai involved the addition of phytohaemagglutinin, type M (PHA). The propagation of M-tropic HIV Ada-M was achieved in the presence of polybrene (PB). Subsequent PBMNL cultures were infected with the harvested virus-containing supernatants, and after one week of culture, the tissue culture 50% infective doses (TCID₅₀) of both strains were determined according to methods known to the art. HIV-1 Ada-M and HIV-1 Lai were kept frozen at-70°C until used.

| EXP # | HIV Strain | Donor | PBMNL | MO | PHA | PB |
|---|---|---|---|---|---|---|
| I.1 | **HIV-1 Lai** | 1 | + | - | + | - |
| I.2 | | 2 | + | - | + | - |
| I.3 | | 3 | + | - | + | - |
| I.4 | | 4 | + | - | + | - |
| I.5 | | 1+2+3+4¹ | + | - | + | - |
| II.1 | **HIV-1 Ada-M** | 1 | - | + | - | + |
| II.2 | | 2 | - | + | - | + |
| II.3 | | 3 | - | + | - | + |
| II.4 | | 4 | - | + | - | + |
| II.5 | | 1+2+3+4¹ | - | + | - | + |

| | | | | | | |
|---|---|---|---|---|---|---|
| ¹ At identical ratios, i.e., by employing 25% of cells of each healthy donor. | | | | | | |

The data presented herein, including the Figures and Tables discussed above which are incorporated herein by reference, indicate that a fucose- (Fuc-4C-Chol)-targeted liposomal delivery system can specifically and highly efficaciously address different HIV reservoir populations, including monocytes, myeloid dendritic cells, and macrophages, for delivering compounds of any or all types known at present, or to be known in the future, that allow interference with HIV or other infectious agents. In accordance with the present invention, these reservoir populations, as well as further reservoir and non-reservoir populations described herein can be targeted with this inventive targeting system or with a functionally related system, such as one featuring, for example, poly-fucose membrane labels as mediators of presumptively even more efficacious cell targeting.

Importantly, the high targeting efficacy was achieved in the presence of serum-borne mannan- or mannose-binding lectin (MBL) which very likely - as a liver-derived substance (Downing, I et al., Immature dendritic cells possess a sugar-sensitive receptor for human mannan-binding lectin, Immunology 2003;109:360-4) - constitutes a component of the small amount of fetal bovine serum employed during culture and incubation. It has also been shown that MBL is autologously secreted by immature human MoDCs (Downing I et al., Immature dendritic cells possess a sugar-sensitive receptor for human mannan-binding lectin, Immunology 2003;109:360-4). Furthermore, MBL, *via* its own C-type lectin domain, can prevent HIV-1 from binding to DC-SIGN (Spear GT et al., Inhibition of DC-SIGN-mediated trans infection of T cells by mannose-binding lectin, Immunology 2003;110:80-5). Therefore, soluble MBL (and other unidentified molecules potentially displaying such characteristics) did not prevent the inventive CTL/CTLD-specific liposomes from interacting with the membrane-bound C-type lectin.

By employing a liposomally entrapped tracer, calcein, a superior targeting efficacy for C-type lectins was demonstrated fluorescence-microscopically and flow-cytometrically. Fluorescence microscopy further revealed time-dependent surface binding and intracellular uptake of C-type lectin-specific liposomes by both immature and mature mDCs. These results clearly reveal efficient binding, internalization and intracellular compound delivery. The data show that fucose-labeled immunoliposomes deliver their contents to immature and mature mDCs, to monocytes, and to macrophages, and that, in addition to their more faint cytoplasmatic distribution, their contents strongly accumulate in the intracellular endosomal/lysosomal system. These observations, together with the fact that HIV and the liposomes administered are comparable in size, enable the inventive delivery system to reach exactly the same compartments where highly infectious HIV is stored and rescued from any systemic attack until being released to infect further cells. Suitable delivered therapeutic agents, in accordance with the present invention, will thus reach an important sanctuary that is not as yet addressed by any therapeutic strategy. Another important benefit is that, due to the fact that these liposomes are retained on the surface of mDCs for prolonged times, T cell subsets interacting with DCs within lymphoid organs and tissues in the course of antigen-specific stimulation, can also be reached for inducing a therapeutic effect(s) in these non-reservoir cells.

Thus, if administered by either or all of bone marrow-directed, intracutaneous, subcutaneous, intraperitoneal, intraplacental or intrauteral, or other envisioned application routes, the inventive methods and products offer the benefit of targeting, *via* C-type lectin receptors or receptors displaying C-type lectin-like domains, those reservoir cells that apparently play a key role in the intraindividual, as well as the interindividual (e.g. mother-to-child HIV transfer, aka vertical transmission) transmission of HIV and other chronically infectious agents, as well as non-reservoir cells actively replicating HIV or another infectious agent (**Fig. 13**). Similarly, the same results may allow for the targeting of regionally restricted C-type lectins (e.g., gastrointestinally or hepatically restricted) implicated in chronic non-infectious diseases, so as to deliver a therapeutically active agent(s) or enable/improve novel approaches for vaccination. The overall inherent potential of the technology is depicted in Fig. 14.

## Claims

1. Use of a lipid-active agent complex comprising the active agent and further comprising at least one targeting ligand on the outer surface of the lipid-active agent complex that binds a group/family of markers on the surface of a reservoir cell for the preparation of a pharmaceutical composition for the prevention or treatment of an infection caused by an infectious agent, or for the prevention or treatment of a chronic degenerative or malignant non-infectious disease, wherein said targeting ligand is fucose, polyfucose or a derivative of polyfucose, wherein the reservoir cell is a dendritic cell, a pre-monocytic myeloid lineage-associated precursor cell, a monocyte, a macrophage or a T cell, and wherein the pharmaceutical composition is for administering by a transvascular route, a subcutaneous route, an intradermal route, a bone-marrow-directed route, an intraplacental route, an intrauteral route, intrahepatic route, an intraperitoneal route or a parenteral route.

2. The use of claim 1, wherein the infectious agent is a virus, bacterium, fungus or protozoan.

3. The use of Claim 2, wherein the virus is selected from the group consisting of HIV-I, HIV-2, HCV, CMV, HSV, EBV, HPV, influenza virus, and Ebola virus.

4. The use of Claim 2, wherein the bacterium is selected from the group consisting of Mycobacterium tuberculosis and Mycobacterium spec.

5. The use of Claim 2, wherein the protozoan is selected from the group consisting of Leishmania amastigotes and the discrete maturation stages of the Plasmodium life cycle.

6. The use of any one of claims 1 to 5, wherein the lipid-active agent complex is a liposome- active agent complex.

7. The use of any one of claims 1 to 6, wherein the active agent is a plant lectin, an anti-viral drug, an anti-HIV drug, an anticancer drug, a cytotoxic agent, an apoptosis inhibitor, an antifungal drug, an antibacterial drug, or an immunomodulatory agent.

8. The use of Claim 7, wherein the active agent is indinavir, saquinavir, nelfinavir, or tenofovir disoproxil fumarate.

9. The use of any one of claims 1 to 8, wherein the lipid-active agent complex further comprises one or more secondary active agents.

10. The use of any one of claims 1 to 8, wherein the lipid-active agent complex further comprises one or more accessory factors, wherein the accessory factors is bivalent cations, co-enzymes, enzyme activators, or pH-modifying agents.

11. The use of any one of claims 1 to 6, wherein the active agent is a small interfering RNA (siRNA).

12. The use of any of claims 1 to 6, wherein the active agent is a sense or an anti-sense RNA.

13. The use of any one of claims 1 to 6, wherein the active agent is an expression vector suitable for dendritic cell-mediated vaccination, such as tumor vaccination.

14. The use of any one of claims 1 to 6, wherein the active agent is a preprocessed protein or peptide suitable for dendritic cell-mediated vaccination, such as tumor vaccination.

15. The use of claim 7, wherein the immunomodulatory agent is an immunosuppressant or immunoactivating agent.

16. The use of claim 6, wherein the active agent is encapsulated in the liposome of the liposome-active agent complex.

17. The use of claim 1, wherein the administering by the intrahepatic route is by infusion into the hepatic artery.

18. The use of any one of claims 1 to 17, wherein the dendritic cell is a myeloid dendritic cell, a follicular dendritic cell, or a plasmacytoid dendritic cell.

19. The use of any one of claims 1 to 17, wherein the T cell is a CD4+ T-helper cell, a CD4+ T- memory cell, a CD8+ T-memory cell, or a CD4+ regulatory T cell.

20. The use of claim 7, wherein the plant lectin is Con-A or MHL.

21. The use of any one of claims 1 to 20, wherein the polyfucose derivative is a fucosyl-cholesterol derivative.

22. The use of claim 20, wherein the lipid-plant lectin complex further comprises Ca²⁺ and transition-metal ions.

23. The use of any one of claims 20 to 22, wherein the MHL is a dimeric or multimeric variant of MHL.

24. The use of any one of claims 7 to 23, wherein the lipid-plant lectin complex comprises a lipid to plant lectin ratio between 5:1 to 7:1.

25. The use of any one of claims 7 to 24, wherein the lipid-plant lectin complex is between 30-250 nm in diameter.

26. The use of claim 1 wherein the lipid-active agent complex is a liposome-plant lectin complex, wherein the outer surface of the liposome comprises a fucose derivative and wherein the infectious agent is HIV.

27. The use of claim 26, wherein the fucose is Fuc-4C-Chol.

28. The use of claim 26 or 27, wherein the plant lectin is Con-A.

29. The use of any one of claims 26 to 28, wherein the pharmaceutical composition is for administering by a subcutaneous route.

30. The use of claim 27, wherein the plant lectin is Con-A and wherein the outer surface of the liposome comprises a Fuc-4C-Chol.

31. A lipid-active agent complex suitable for a targeted delivery of an active agent to a reservoir cell for use in a method of prevention or treatment of an infection caused by an infectious agent, or prevention or treatment of a chronic degenerative or malignant non-infectious disease, wherein said lipid-active agent complex comprises the active agent, and further comprises a targeting ligand on the outer surface of the lipid-active agent complex, wherein said targeting ligand is fucose, polyfucose, or a polyfucose derivative, wherein the reservoir cell is a dendritic cell, a pre-monocytic myeloid lineage-associated precursor cell, a monocyte, a macrophage, or a T cell, and wherein the lipid-active agent complex is for administering by a transvascular route, a subcutaneous route, an intradermal route, a bone-marrow-directed route, an intraplacental route, an intrauteral route, intrahepatic route, an intraperitoneal route or a parenteral route..

32. The lipid-active agent complex for the use of claim 31, wherein the lipid-active agent complex is a liposome-active agent complex.

33. The lipid-active agent complex for the use of claim 32, wherein the active agent is a plant lectin.

34. The lipid-active agent complex for the use of claim 33, wherein the plant lectin is Con-A or MHL.

35. The lipid-active agent complex for the use of any one of claims 32 to 34, wherein the liposome-active agent complex further comprises Ca and transition-metal ions.

36. The lipid-active agent complex for the use of any one of claims 31 to 35, wherein the liposome-active agent complex further comprises one or more accessory factors, wherein in the accessory factors is bivalent cations, co-enzymes, enzyme activators, or pH-modifying agents.

37. The lipid-active agent complex for the use of any one of claims 31 to 36, wherein the liposome-active agent complex comprises a lipid to active agent ratio between 5:1 to 7: 1, 3 : 1 to 10 : 1,or 3 : 1 to 100 : 1.

38. The lipid-active agent complex for the use of any one of claims 31 to 37, wherein the liposome-active agent complex is between 30-250 nm in diameter.

## Patentansprüche

1. Verwendung eines Lipid-Wirkstoff-Komplexes, umfassend den Wirkstoff und weiter umfassend mindestens einen zielgerichteten Liganden auf der Außenfläche des Lipid-Wirkstoff-Komplexes, der eine Gruppe/Familie von Markern auf der Oberfläche einer Reservoirzelle bindet, zur Herstellung einer pharmazeutischen Zusammensetzung zur Verhinderung oder Behandlung einer Infektion, die durch einen Infektionserreger verursacht wird, oder zur Verhinderung oder Behandlung einer chronischen degenerativen oder malignen, nicht infektiösen Erkrankung, wobei der zielgerichtete Ligand Fucose, Polyfucose oder ein Derivat aus Polyfucose ist, wobei die Reservoirzelle eine dendritische Zelle, eine prämonozytische myeloidlinienassozüerte Vorläuferzelle, ein Monozyt, ein Makrophage oder eine T-Zelle ist und wobei die pharmazeutische Zusammensetzung zur Verabreichung über einen transvaskulären Weg, einen subkutanen Weg, einen intradermalen Weg, einen auf das Knochenmark gerichteten Weg, einen intraplazentalen Weg, einen intrauterinen Weg, intrahepatischen Weg, einen intraperitonealen Weg oder einen parenteralen Weg bestimmt ist.

2. Verwendung nach Anspruch 1, wobei der Infektionserreger ein Virus, Bakterium, Pilz oder Protozoon ist.

3. Verwendung nach Anspruch 2, wobei das Virus aus der Gruppe bestehend aus HIV-1, HIV-2, HCV, CMV, HSV, EBV, HPV, Influenza-Virus und Ebolavirus ausgewählt ist.

4. Verwendung nach Anspruch 2, wobei das Bakterium aus der Gruppe bestehend aus Mycobakterium tuberculosis und Mycobakterium spec. ausgewählt ist.

5. Verwendung nach Anspruch 2, wobei das Protozoon aus der Gruppe bestehend aus Leishmania amastigotes und den diskreten Reifestadien des Plasmodium-Lebenszyklus ausgewählt ist.

6. Verwendung nach einem der Ansprüche 1 bis 5, wobei der Lipid-Wirkstoff-Komplex ein Liposom-Wirkstoff-Komplex ist.

7. Verwendung nach einem der Ansprüche 1 bis 6, wobei der Wirkstoff ein Pflanzenlektin, ein antivirales Medikament, ein anti-HIV-Medikament, ein Antikrebsmedikament, ein zytotoxisches Mittel, ein Apoptosehemmer, ein antifungales Medikament, ein antibakterielles Medikament oder ein immunmodulatorisches Mittel ist.

8. Verwendung nach Anspruch 7, wobei der Wirkstoff Indinavir, Saquinavir, Nelfinavir oder Tenofovirdisoproxilfumarat ist.

9. Verwendung nach einem der Ansprüche 1 bis 8, wobei der Lipid-Wirkstoff-Komplex weiter einen oder mehrere sekundäre Wirkstoffe umfasst.

10. Verwendung nach einem der Ansprüche 1 bis 8, wobei der Lipid-Wirkstoff-Komplex weiter einen oder mehrere zusätzliche Faktoren umfasst, wobei die zusätzlichen Faktoren bivalente Kationen, Coenzyme, Enzymaktivatoren oder pH-modifizierende Mittel sind.

11. Verwendung nach einem der Ansprüche 1 bis 6, wobei der Wirkstoff eine kleine interferierende RNA ist (siRNA).

12. Verwendung nach einem der Ansprüche 1 bis 6, wobei der Wirkstoff eine Sense- oder eine Antisense-RNA ist.

13. Verwendung nach einem der Ansprüche 1 bis 6, wobei der Wirkstoff ein Expressionsvektor ist, der für die dendritische zellvermittelte Impfung, wie beispielsweise eine Tumorimpfung, geeignet ist.

14. Verwendung nach einem der Ansprüche 1 bis 6, wobei der Wirkstoff ein aufbereitetes Protein oder Peptid ist, das für die dendritenzellvermittelte Impfung, wie beispielsweise eine Tumorimpfung, geeignet ist.

15. Verwendung nach Anspruch 7, wobei das immunmodulatorische Mittel ein immununterdrückendes oder immunaktivierendes Mittel ist.

16. Verwendung nach Anspruch 6, wobei der Wirkstoff im Liposom des Liposom-Wirkstoff-Komplexes verkapselt ist.

17. Verwendung nach Anspruch 1, wobei das Verabreichen über den intrahepatischen Weg durch Infusion in die Leberarterie erfolgt.

18. Verwendung nach einem der Ansprüche 1 bis 17, wobei die dendritische Zelle eine myeloide dendritische Zelle, eine follikuläre dendritische Zelle oder eine plasmazytoide dendritische Zelle ist.

19. Verwendung nach einem der Ansprüche 1 bis 17, wobei die T-Zelle eine CD4+ T-Helferzelle, eine CD4+ T-Speicherzelle, eine CD8+ T-Speicherzelle oder eine CD4+ regulatorische T-Zelle ist.

20. Verwendung nach Anspruch 7, wobei das Pflanzenlektin Con-A oder MHL ist.

21. Verwendung nach einem der Ansprüche 1 bis 20, wobei das Polyfucosederivat ein Fucosyl-Cholesterin-Derivat ist.

22. Verwendung nach Anspruch 20, wobei der Lipid-Pflanzenlektin-Komplex weiter Ca²⁺- und Übergangsmetallionen umfasst.

23. Verwendung nach einem der Ansprüche 20 bis 22, wobei das MHL eine dimere oder multimere Variante von MHL ist.

24. Verwendung nach einem der Ansprüche 7 bis 23, wobei der Lipid-Pflanzenlektin-Komplex ein Verhältnis von Lipid zu Pflanzenlektin von 5:1 bis 7:1 umfasst.

25. Verwendung nach einem der Ansprüche 7 bis 24, wobei der Lipid-Pflanzenlektin-Komplex von 30 bis 250 nm im Durchmesser ist.

26. Verwendung nach Anspruch 1, wobei der Lipid-Wirkstoff-Komplex ein Liposom-Pflanzenlektin-Komplex ist, wobei die Außenfläche des Liposoms ein Fucosederivat umfasst und wobei der Infektionserreger HIV ist.

27. Verwendung nach Anspruch 26, wobei die Fucose Fuc-4C-Chol ist.

28. Verwendung nach Anspruch 26 oder 27, wobei das Pflanzenlektin Con-A ist.

29. Verwendung nach einem der Ansprüche 26 bis 28, wobei die pharmazeutische Zusammensetzung zur Verabreichung über den subkutanen Weg bestimmt ist.

30. Verwendung nach Anspruch 27, wobei das Pflanzenlektin Con-A ist und wobei die Außenfläche des Liposoms Fuc-4C-Chol umfasst.

31. Lipid-Wirkstoff-Komplex, der für eine gezielte Abgabe eines Wirkstoffs an eine Reservoirzelle geeignet ist, zur Verwendung bei einem Verfahren zur Verhinderung oder Behandlung einer Infektion, die durch einen Infektionserreger hervorgerufen wird, oder Verhinderung oder Behandlung einer chronischen degenerativen oder malignen, nicht infektiösen Erkrankung, wobei der Lipid-Wirkstoff-Komplex den Wirkstoff umfasst und weiter einen zielgerichteten Liganden auf der Außenfläche des Lipid-Wirkstoff-Komplexes umfasst, wobei der zielgerichtete Ligand Fucose, Polyfucose oder ein Polyfucosederivat ist, wobei die Reservoirzelle eine dendritische Zelle, eine prämonozytische myeloidlinienassozüerte Vorläuferzelle, ein Monozyt, ein Makrophage oder eine T-Zelle ist und wobei der Lipid-Wirkstoff-Komplex zur Verabreichung über einen transvaskulären Weg, einen subkutanen Weg, einen intradermalen Weg, einen auf das Knochenmark gerichteten Weg, einen intraplazentalen Weg, einen intrauterinen Weg, intrahepatischen Weg, einen intraperitonealen Weg oder einen parenteralen Weg bestimmt ist.

32. Lipid-Wirkstoff-Komplex zur Verwendung nach Anspruch 31, wobei der Lipid-Wirkstoff-Komplex ein Liposom-Wirkstoff-Komplex ist.

33. Lipid-Wirkstoff-Komplex zur Verwendung nach Anspruch 32, wobei der Wirkstoff Pflanzenlektin ist.

34. Lipid-Wirkstoff-Komplex zur Verwendung nach Anspruch 33, wobei das Pflanzenlektin Con-A oder MHL ist.

35. Lipid-Wirkstoff-Komplex zur Verwendung nach einem der Ansprüche 32 bis 34, wobei der Liposom-Wirkstoff-Komplex weiter Ca- und Übergangsmetallionen umfasst.

36. Lipid-Wirkstoff-Komplex zur Verwendung nach einem der Ansprüche 31 bis 35, wobei der Liposom-Wirkstoff-Komplex weiter einen oder mehrere zusätzliche Faktoren umfasst, wobei in den zusätzlichen Faktoren bivalente Kationen, Coenzyme, Enzymaktivatoren oder pH-modifizierende Mittel vorliegen.

37. Lipid-Wirkstoff-Komplex zur Verwendung nach einem der Ansprüche 31 bis 36, wobei der Liposom-Wirkstoff-Komplex ein Verhältnis von Lipid zu Wirkstoff von 5:1 bis 7:1, 3:1 bis 10:1 oder 3:1 bis 100:1 umfasst.

38. Lipid-Wirkstoff-Komplex zur Verwendung nach einem der Ansprüche 31 bis 37, wobei der Liposom-Wirkstoff-Komplex von 30 bis 250 nm im Durchmesser ist.

## Revendications

1. Utilisation d'un complexe d'agent actif-lipide comprenant l'agent actif et comprenant en outre au moins un ligand de ciblage sur la surface extérieure du complexe d'agent actif-lipide liant un groupe/famille de marqueurs sur la surface d'une cellule réservoir pour la préparation d'une composition pharmaceutique pour la prévention ou le traitement d'une infection provoquée par un agent infectieux ou pour le prévention ou le traitement d'une maladie non-infectieuse maligne ou dégénérative chronique, dans laquelle ledit ligand de ciblage est un fucose, un polyfucose ou un dérivé d'un polyfucose, dans laquelle la cellule réservoir est une cellule dendritique, une cellule de précurseur associée à une lignée myéloïde pré-monocytaire, un monocyte, un macrophage ou une cellule T, et dans laquelle la composition pharmaceutique est destinée à être administrée par voie transvasculaire, sous-cutanée, intradermique, dirigée vers la moelle osseuse, intra-placentaire, intra-utérine, intra-hépatique, intra-péritonéale ou parentérale.

2. Utilisation selon la revendication 1, dans laquelle l'agent infectieux est un virus, une bactérie, un fongus ou un protozoaire.

3. Utilisation selon la revendication 2, dans laquelle le virus est sélectionné parmi le groupe constitué par le virus HIV-I, HIV -2, HCV, CMV, HSV, EBV, HPV, le virus de la grippe et le virus Ébola.

4. Utilisation selon la revendication 2, dans laquelle la bactérie est sélectionnée parmi le groupe constitué par la Mycobacterium tuberculosis et la Mycobacterium spec.

5. Utilisation selon la revendication 2, dans laquelle le protozoaire est sélectionné parmi le groupe constitué par des amastigotes Leishmania et les stades de maturation discrète du cycle de vie du Plasmodium.

6. Utilisation selon l'une quelconque des revendications 1 à 5, dans laquelle le complexe d'agent actif-lipide est un complexe d'agent actif-liposome.

7. Utilisation selon l'une quelconque des revendications 1 à 6, dans laquelle l'agent actif est une lectine de plante, un médicament antiviral, un médicament anti-HIV, un médicament anti-cancer, un agent cytotoxique, un inhibiteur d'apoptose, un médicament antifongique, un médicament antibactérien ou un agent immunomodulateur.

8. Utilisation selon la revendication 7, dans laquelle l'agent actif est un fumarate de disoproxile d'indinavir, de saquinavir, de nelfinavir ou de tenofovir.

9. Utilisation selon l'une quelconque des revendications 1 à 8, dans laquelle le complexe d'agent actif-lipide comprend en outre un ou plusieurs agents actifs secondaires.

10. Utilisation selon l'une quelconque des revendications 1 à 8, dans laquelle le complexe d'agent actif-lipide comprend en outre un ou plusieurs facteurs accessoires, les facteurs accessoires étant des cations bivalents, des coenzymes, des activateurs d'enzymes ou des agents modificateurs de pH.

11. Utilisation selon l'une quelconque des revendications 1 à 6, dans laquelle l'agent actif est un petit ARN interférant (siRNA).

12. Utilisation selon l'une quelconque des revendications 1 à 6, dans laquelle l'agent actif est un ARN sens ou anti-sens.

13. Utilisation selon l'une quelconque des revendications 1 à 6, dans laquelle l'agent actif est un vecteur d'expression adéquat à une vaccination médiatisée par des cellules dendritiques, telle qu'une vaccination tumorale.

14. Utilisation selon l'une quelconque des revendications 1 à 6, dans laquelle l'agent actif est une protéine ou un peptide prétraité adéquat à une vaccination médiatisée par des cellules dendritiques, telle qu'une vaccination tumorale.

15. Utilisation selon la revendication 7, dans laquelle l'agent immunomodulateur est un agent immunosuppresseur ou immunoactivateur.

16. Utilisation selon la revendication 6, dans laquelle l'agent actif est encapsulé dans le liposome du complexe d'agent actif-liposome.

17. Utilisation selon la revendication 1, dans laquelle l'administration par voie intra-hépatique est effectuée par perfusion dans l'artère hépatique.

18. Utilisation selon l'une quelconque des revendications 1 à 17, dans laquelle la cellule dendritique est une cellule dendritique myéloïde, une cellule dendritique folliculaire ou une cellule dendritique plasmocytoïde.

19. Utilisation selon l'une quelconque des revendications 1 à 17, dans laquelle la cellule T est une cellule auxiliaire T-CD4+, une cellule-mémoire T-CD4+, une cellule-mémoire T-CD8+ ou une cellule T régulatrice CD4+.

20. Utilisation selon la revendication 7, dans laquelle la lectine de plante est une Con-A ou une MHL.

21. Utilisation selon l'une quelconque des revendications 1 à 20, dans laquelle le dérivé de polyfucose est un dérivé de cholestérol-fucosyle.

22. Utilisation selon la revendication 20, dans laquelle le complexe de lectine de plante-lipide comprend en outre du Ca²⁺ et des ions métalliques de transition.

23. Utilisation selon l'une quelconque des revendications 20 à 22, dans laquelle le MHL est une variante dimère ou multimère du MHL.

24. Utilisation selon l'une quelconque des revendications 7 à 23, dans laquelle le complexe de lectine de plante-lipide comprend une proportion de lipide par rapport à la lectine de plante comprise entre 5:1 et 7:1.

25. Utilisation selon l'une quelconque des revendications 7 à 24, dans laquelle le complexe de lectine de plante-lipide a un diamètre compris entre 30 et 250 nm.

26. Utilisation selon la revendication 1, dans laquelle le complexe d'agent actif lipide est un complexe de lectine de plante-liposome, la surface extérieure du liposome comprenant un dérivé du fucose et l'agent infectieux étant un HIV.

27. Utilisation selon la revendication 26, dans laquelle le fucose est un Fuc-4C-Chol.

28. Utilisation selon la revendication 26 ou 27, dans laquelle la lectine de plante est une Con-A.

29. Utilisation selon l'une quelconque des revendications 26 à 28, dans laquelle la composition pharmaceutique est destinée à être administrée par voie sous-cutanée.

30. Utilisation selon la revendication 27, dans laquelle la lectine de plante est une Con-A et dans laquelle la surface extérieure du liposome comprend un Fuc-4C-Chol.

31. Complexe d'agent actif-lipide adéquat à une administration ciblée d'un agent actif dans une cellule réservoir destiné à être utilisé dans un procédé de prévention ou de traitement d'une infection provoquée par un agent infectieux, ou de prévention ou de traitement d'une maladie non-infectieuse maligne ou dégénérative chronique, dans lequel ledit complexe d'agent actif-lipide comprend l'agent actif et comprend en outre un ligand de ciblage sur la surface extérieure du complexe d'agent actif-lipide, dans lequel ledit ligand de ciblage est un fucose, un polyfucose ou un dérivé d'un polyfucose, dans lequel la cellule réservoir est une cellule dendritique, une cellule de précurseur associée à une lignée myéloïde pré-monocytaire, un monocyte, un macrophage ou une cellule T, et dans lequel le complexe d'agent actif-lipide est destiné à être administré par voie transvasculaire, sous-cutanée, intradermique, dirigée vers la moelle osseuse, intra-placentaire, intra-utérine, intra-hépatique, intra-péritonéale ou parentérale.

32. Complexe d'agent actif-lipide pour l'utilisation selon la revendication 31, dans lequel le complexe d'agent actif-lipide est un complexe d'agent actif-liposome.

33. Complexe d'agent actif-lipide pour l'utilisation selon la revendication 32, dans lequel l'agent actif est une lectine de plante.

34. Complexe d'agent actif-lipide pour l'utilisation selon la revendication 33, dans lequel la lectine de plante est une Con-A ou une MHL.

35. Complexe d'agent actif-lipide pour l'utilisation selon l'une quelconque des revendications 32 à 34, dans lequel le complexe d'agent actif-liposome comprend en outre du Ca et des ions métalliques de transition.

36. Complexe d'agent actif-lipide pour l'utilisation selon l'une quelconque des revendications 31 à 35, dans lequel le complexe d'agent actif-liposome comprend en outre un ou plusieurs facteurs accessoires, les facteurs accessoires étant des cations bivalents, des coenzymes, des activateurs d'enzymes ou des agents modificateurs de pH.

37. Complexe d'agent actif-lipide pour l'utilisation selon l'une quelconque des revendications 31 à 36, dans lequel le complexe d'agent actif-liposome comprend une proportion de lipide par rapport à l'agent actif comprise entre 5:1 et 7:1, entre 3:1 et 10:1 ou entre 3:1 et 100:1.

38. Complexe d'agent actif-lipide pour l'utilisation selon l'une quelconque des revendications 31 à 37, dans lequel le complexe d'agent actif-liposome a un diamètre compris entre 30 et 250 nm.


### SEQUENCE LISTING

<110> Let There Be Hope Medical Research Institute (Assignee)
   Gieseler, Robert K. (Inventor)
   Marquitan, Guido (Inventor)
   Scolaro, Michael J. (Inventor)
   Schwarz, Andreas (Inventor)
<120> CARBOHYDRATE-DERIVATIZED LIPOSOMES FOR TARGETING CELLULAR CARBOHYDRATE RECOGNITION DOMAINS OF CTL/CTLD LECTINS, AND INTRACELLULAR DELIVERY OF THERAPEUTICALLY ACTIVE COMPOUNDS
<130> 23046-81260
<140> Unassigned
   <141> 2005-03-21
<150> 60/554,790
   <151> 2004-03-19
<160> 4
<170> FastSEQ for Windows Version 4.0
<210> 1
   <211> 30
   <212> DNA
   <213> Artificial Sequence
<220>
   <223> Primer
<400> 1
   agtgggggga catcaagcag ccatgcaaat 30
<210> 2
   <211> 18
   <212> DNA
   <213> Artificial Sequence
<220>
   <223> Primer
<400> 2
   tcatctggcc tggtgcaa 18
<210> 3
   <211> 28
   <212> DNA
   <213> Artificial Sequence
<220>
   <223> Primer; Labeled with 5-carboxyfluorescein
<400> 3
   cagcttagag accatcaatg aggaagcg 28
<210> 4
   <211> 19
   <212> DNA
   <213> Artificial Sequence
<220>
   <223> Primer
<400> 4 19
   ggtgcaatag gccctgcat 19